# EUROPEAN PATENT APPLICATION

(11) **EP 2 309 264 A1**
(43) Date of publication of application: **13.04.2011**
(21) Application number: 09758417.1
(22) Date of filing: 05.06.2009
(51) Int. Cl.: G01N 33/543, C07K 16/10, G01N 33/53, G01N 33/569, C12N 15/09, C12P 21/08

(54) **DEVICE FOR DETECTION OF INFLUENZA VIRUS**

(30) Priority: 06.06.2008 JP 2008149300; 04.03.2009 JP 2009050066
(71) Applicant: National University Corporation University Of Toyama, Toyama-shi, Toyama 930-8555 (JP); SC World Inc., Toyama 930-0866 (JP); Kyowa Medex CO., LTD., Tokyo 104-6004 (JP)
(72) Inventor: MURAGUCHI, Atsushi, Toyama-shi Toyama 930-0194 (JP); KISHI, Hiroyuki, Toyama-shi Toyama 930-0194 (JP); OZAWA, Tatsuhiko, Toyama-shi Toyama 930-0194 (JP); IKEMOTO, Mamoru, Toyama-shi Toyama 930-0866 (JP); ONO, Hitoshi, Sunto-gun Shizuoka 411-0932 (JP); MORITA, Kazuki, Sunto-gun Shizuoka 411-0932 (JP); UZAWA, Koji, Sunto-gun Shizuoka 411-0932 (JP)
(74) Representative: Vossius & Partner
(86) International application number: PCT/JP2009/060328
(87) International publication number: WO 2009/148150

(57) **Abstract**

A device for detecting or quantifying influenza viruses in a sample, which comprises a detection region having a human anti-influenza virus nucleoprotein antibody immobilized onto a support, a sample supply region, and a sample-migrating region; and a kit for detecting or quantifying influenza viruses, which comprises a solid phase in which a human anti-influenza virus nucleoprotein antibody is fixed onto a carrier.

SEQUENCE LISTING

<110>  NATIONAL UNIVERSITY CORPORATION UNIVERSITY OF TOYAMA
       SC WORLD, INC.
       KYOWA MEDEX CO.,LTD

<120>  Device for detecting influenza virus

<130>  1000P12013WO0

<150>  JP 2008-149300
<151>  2008-06-06

<150>  JP 2009-050066
<151>  2009-03-04

<160>  40

<170>  PatentIn version 3.4

<210>  1
<211>  379
<212>  DNA
<213>  Artificial

<220>
<223>  Variable region of human antibody

<400>  1
caggtgcagc tggtgcagtc tggggctgtg gtgaagaagc ctggggactc agtgaaggtc      60

tcctgcaagg catctggata cacgttcacc acctactgga tgcactgggt gcgacaggcc     120

cctggacaag ggcccgaatg gatgggaata gtcaatccca caggtggcac cgttatgtac     180

gcaaagaagt tccagggtcg ggtcatcatg accagggaca cgtccacgac gacagtcttt     240

ttgcaactga acggcctgga atctgaggac accgccatat attactgtgc gagagatctg     300

gggcctgcaa gggtggattt tgactggttg tccacctttg tctcctgggg ccagggaacc     360

ctggtcaccg tctcctcag                                                  379


<210>  2
<211>  358
<212>  DNA
<213>  Artificial

<220>
<223>  Variable region of human antibody

<400>  2
caggtgcagc tggtgcagtc tggggctgag gtgaagaagc ctgggtcctc ggtgaaggta      60

tcctgcaagg ctaccggaga caccttcagc agctattcta tcagctgggt gcgacaggct     120

cctggacaag ggcttgagtg gatgggaagg gtcacccctt cctttggaat atcactctac     180

gcacagaaat tccagggcag agtcaccatt accgcggaca gatcctcgag cacagcctat     240

atggagctga acaacctgac atctgatgac accgccgtgt attactgtgc ggctgggggt 300

cttgtagtgg tggtggcgaa ccactggggc cagggaaccc tggtcaccgt ctcctcag 358

<210> 3
<211> 351
<212> DNA
<213> Artificial

<220>
<223> Variable region of human antibody

<400> 3
caggtgcagc tggtgcagtc tgggggaggc gtggtccagc ctgggaggtc cctgagactc 60

tcctgtgcag cgtctggatt caccttcagt agatatggca tgcactgggt ccgccaggct 120

ccaggcaagg ggctggagtg ggtggcagtg atattatatg agggaattaa taaagactat 180

gcagactccg tgaagggccg attcaccatc tccagagaca actccaagaa cacactgtat 240

ttgcaaatga atagtctgag agccgaggac acggctgtgt attactgtgc gagagatcgt 300

gagctgcgac ttgactactg gggccaggga accctggtca ccgtctcctc a 351

<210> 4
<211> 401
<212> DNA
<213> Artificial

<220>
<223> Variable region of human antibody

<400> 4
caggtgcagc tggtgcagtc tgggggaggc ttggtacagc ctggcaggtc cctgagactc 60

tcttgtatag cctctggatt cagttttgat gattatgcca tgcactgggt ccggcaaatt 120

ccagggaagg gcctggagtg ggtctcaggt attagttgga acagtggtgc cagaggctat 180

gcggactctg tgaagggccg atttaccatc tccagagaca acgccaaaaa ttccctgtat 240

ctacaaatga acagtctcag agttggggac acggccttct attactgtgc cagagacatg 300

ggggccgggc caaatgatta tgataccagt ggcaattacc cccttcacct ctatggcatg 360

gacgtctggg gccatgggac cacggtcacc gtctcctcag g 401

<210> 5
<211> 359
<212> DNA
<213> Artificial

<220>
<223> Variable region of human antibody

<400> 5
caggtgcagc tgcaggagtc gggcccagga ctggtgaagc cttcacagac cctgtccctc 60

acctgcagtg tctctggtgg ctccatcagc aatcctgatc actactggaa ctggatccga 120

cagcccccag ggaagggcct tgagtggatt gggtacatct attacactgg gcgcacctgg 180

tacagcccgt ccctcaagag tcgcgtttcc ttatcagtag acacgtccaa gagccagttc 240

tccttgaacg tggactctgt gattgccgca gacacggccg tatattactg cgccagaagt 300

cacgtgtggt tcggggagtt atactggggc cagggagccc tggtcaccgt ctcctcagg 359

<210> 6
<211> 355
<212> DNA
<213> Artificial

<220>
<223> Variable region of human antibody

<400> 6
caggtgcagc tggtgcagtc tgggggaggc ttggtcaagc ctggagggtc cctgagactc 60

tcctgtgcag gctctggatt cagcttcagt gactattata tgacgtggat ccgccaggct 120

ccagggaagg ggctggagtg ggtttcatcc atcaatagtg gcagcactgt cacaaactac 180

gcagactctg tgcagggccg attcaccatc tccagagaca agaactcagt gtatctgcaa 240

atgaacagcc tgagaaccga ggacacggcc gtctattact gtgcgagaga ttttggtatc 300

aaagcgggta cttcggacta ctggggccag ggaaccctgg tcaccgtctc ctccg 355

<210> 7
<211> 393
<212> DNA
<213> Artificial

<220>
<223> Variable region of human antibody

<400> 7
caggtgcagc tggtgcagtc tggggctgag gtgaagaggc ctggggcctc agtgaaggtc 60

tcctgcaagg cttctggata caacctcatc gacttctatg tgaactgggt gcgacaggtc 120

cctggacaag gacttgagtg gatggggtac atcaaccctg acagtggtgg cgcacactat 180

gaagggaaat ttcagggcag ggtcatcatg accagggaca cgtccatcaa cacagcctat 240

atggaattgc ccagcctgac atctgacgac acggccgtct atttctgtgc gagaggggct 300

agtagtggct tgttgaccta ctattacggt ttggacgtct ggggcgaagg gaccacggtt 360

atcgtctcct caggtgagtg tcccaagcag ctt 393

<210> 8
<211> 126
<212> PRT

<213>  Artificial

<220>
<223>  Variable region of human antibody

<400>  8

Gln Val Gln Leu Val Gln Ser Gly Ala Val Val Lys Lys Pro Gly Asp
1               5                   10                  15

Ser Val Lys Val Ser Cys Lys Ala Ser Gly Tyr Thr Phe Thr Thr Tyr
            20                  25                  30

Trp Met His Trp Val Arg Gln Ala Pro Gly Gln Gly Pro Glu Trp Met
        35                  40                  45

Gly Ile Val Asn Pro Thr Gly Gly Thr Val Met Tyr Ala Lys Lys Phe
    50                  55                  60

Gln Gly Arg Val Ile Met Thr Arg Asp Thr Ser Thr Thr Thr Val Phe
65                  70                  75                  80

Leu Gln Leu Asn Gly Leu Glu Ser Glu Asp Thr Ala Ile Tyr Tyr Cys
                85                  90                  95

Ala Arg Asp Leu Gly Pro Ala Arg Val Asp Phe Asp Trp Leu Ser Thr
            100                 105                 110

Phe Val Ser Trp Gly Gln Gly Thr Leu Val Thr Val Ser Ser
        115                 120                 125

<210>  9
<211>  119
<212>  PRT
<213>  Artificial

<220>
<223>  Variable region of human antibody

<400>  9

Gln Val Gln Leu Val Gln Ser Gly Ala Glu Val Lys Lys Pro Gly Ser
1               5                   10                  15

Ser Val Lys Val Ser Cys Lys Ala Thr Gly Asp Thr Phe Ser Ser Tyr
            20                  25                  30

Ser Ile Ser Trp Val Arg Gln Ala Pro Gly Gln Gly Leu Glu Trp Met
        35                  40                  45

Gly Arg Val Thr Pro Ser Phe Gly Ile Ser Leu Tyr Ala Gln Lys Phe
    50                  55                  60

Gln Gly Arg Val Thr Ile Thr Ala Asp Arg Ser Ser Ser Thr Ala Tyr
65                  70                  75                  80

Met Glu Leu Asn Asn Leu Thr Ser Asp Asp Thr Ala Val Tyr Tyr Cys
                85                  90                  95

Ala Ala Gly Gly Leu Val Val Val Val Ala Asn His Trp Gly Gln Gly
            100                 105                 110

Thr Leu Val Thr Val Ser Ser
        115

<210>  10
<211>  117
<212>  PRT
<213>  Artificial

<220>
<223>  Variable region of human antibody

<400>  10

Gln Val Gln Leu Val Gln Ser Gly Gly Gly Val Val Gln Pro Gly Arg
1               5                   10                  15

Ser Leu Arg Leu Ser Cys Ala Ala Ser Gly Phe Thr Phe Ser Arg Tyr
            20                  25                  30

Gly Met His Trp Val Arg Gln Ala Pro Gly Lys Gly Leu Glu Trp Val
        35                  40                  45

Ala Val Ile Leu Tyr Glu Gly Ile Asn Lys Asp Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Asp Arg Glu Leu Arg Leu Asp Tyr Trp Gly Gln Gly Thr Leu
            100                 105                 110

Val Thr Val Ser Ser
        115

<210>  11
<211>  133
<212>  PRT
<213>  Artificial

<220>
<223>  Variable region of human antibody

<400>  11

Gln Val Gln Leu Val Gln Ser Gly Gly Gly Leu Val Gln Pro Gly Arg
1               5                   10                  15

Ser Leu Arg Leu Ser Cys Ile Ala Ser Gly Phe Ser Phe Asp Asp Tyr
            20                  25                  30

Ala Met His Trp Val Arg Gln Ile Pro Gly Lys Gly Leu Glu Trp Val
        35                  40                  45

Ser Gly Ile Ser Trp Asn Ser Gly Ala Arg Gly Tyr Ala Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Val Gly Asp Thr Ala Phe Tyr Tyr Cys
                85                  90                  95

Ala Arg Asp Met Gly Ala Gly Pro Asn Asp Tyr Asp Thr Ser Gly Asn
            100                 105                 110

Tyr Pro Leu His Leu Tyr Gly Met Asp Val Trp Gly His Gly Thr Thr
        115                 120                 125

Val Thr Val Ser Ser
    130

<210>  12
<211>  119
<212>  PRT
<213>  Artificial

<220>
<223>  Variable region of human antibody

<400>  12

Gln Val Gln Leu Gln Glu Ser Gly Pro Gly Leu Val Lys Pro Ser Gln
1               5                   10                  15

Thr Leu Ser Leu Thr Cys Ser Val Ser Gly Gly Ser Ile Ser Asn Pro
            20                  25                  30

Asp His Tyr Trp Asn Trp Ile Arg Gln Pro Pro Gly Lys Gly Leu Glu
        35                  40                  45

Trp Ile Gly Tyr Ile Tyr Tyr Thr Gly Arg Thr Trp Tyr Ser Pro Ser
    50                  55                  60

Leu Lys Ser Arg Val Ser Leu Ser Val Asp Thr Ser Lys Ser Gln Phe
65                  70                  75                  80

Ser Leu Asn Val Asp Ser Val Ile Ala Ala Asp Thr Ala Val Tyr Tyr
                85                  90                  95

Cys Ala Arg Ser His Val Trp Phe Gly Glu Leu Tyr Trp Gly Gln Gly
            100                 105                 110

Ala Leu Val Thr Val Ser Ser
        115

<210>  13
<211>  118
<212>  PRT
<213>  Artificial

<220>
<223>  Variable region of human antibody

<400>  13

Gln Val Gln Leu Val Gln Ser Gly Gly Gly Leu Val Lys Pro Gly Gly
1               5                   10                  15

Ser Leu Arg Leu Ser Cys Ala Gly Ser Gly Phe Ser Phe Ser Asp Tyr
            20                  25                  30

Tyr Met Thr Trp Ile Arg Gln Ala Pro Gly Lys Gly Leu Glu Trp Val
        35                  40                  45

Ser Ser Ile Asn Ser Gly Ser Thr Val Thr Asn Tyr Ala Asp Ser Val
    50                  55                  60

Gln Gly Arg Phe Thr Ile Ser Arg Asp Lys Asn Ser Val Tyr Leu Gln
65                  70                  75                  80

Met Asn Ser Leu Arg Thr Glu Asp Thr Ala Val Tyr Tyr Cys Ala Arg
                85                  90                  95

Asp Phe Gly Ile Lys Ala Gly Thr Ser Asp Tyr Trp Gly Gln Gly Thr
            100                 105                 110

Leu Val Thr Val Ser Ser
        115

<210>  14
<211>  124
<212>  PRT
<213>  Artificial

<220>
<223>  Variable region of human antibody

<400>  14

Gln Val Gln Leu Val Gln Ser Gly Ala Glu Val Lys Arg Pro Gly Ala
1               5                   10                  15

Ser Val Lys Val Ser Cys Lys Ala Ser Gly Tyr Asn Leu Ile Asp Phe
            20                  25                  30

Tyr Val Asn Trp Val Arg Gln Val Pro Gly Gln Gly Leu Glu Trp Met
        35                  40                  45

Gly Tyr Ile Asn Pro Asp Ser Gly Gly Ala His Tyr Glu Gly Lys Phe
    50                  55                  60

Gln Gly Arg Val Ile Met Thr Arg Asp Thr Ser Ile Asn Thr Ala Tyr
65                  70                  75                  80

Met Glu Leu Pro Ser Leu Thr Ser Asp Asp Thr Ala Val Tyr Phe Cys
                85                  90                  95

Ala Arg Gly Ala Ser Ser Gly Leu Leu Thr Tyr Tyr Tyr Gly Leu Asp
            100                 105                 110

Val Trp Gly Glu Gly Thr Thr Val Ile Val Ser Ser
        115                 120

<210>  15
<211>  327
<212>  DNA
<213>  Artificial

<220>
<223>  Variable region of human antibody

<400>  15
gacatccaga tgacccagtc tccatcgtcc ctgtctgcat ctgtgggaga cagagtcatc      60

atcacttgcc gggcaagtca gggcattacc agacacttaa attggtatca gcagaaatca    120

ggcaaagccc ccaaactcct gatctacgat gcgtccagtt tgcaaagtgg ggtcccatcc    180

aggttcagtg gcagtggatc tggggcagat ttcactctca ccatcagcag tgtgcaacct    240

gaagattttg caacttacta ctgtcaacag agttatagta accctccgac tttcggcccc    300

gggaccaaag tggatatcaa acgtgag                                        327


<210>  16
<211>  328
<212>  DNA
<213>  Artificial

<220>
<223>  Variable region of human antibody

<400>  16
gaaattgtgt tgacacagtc tccagccacc ctgtctgcat ctgtgggaga cagagtcacc     60

atcacttgcc gggcgagtca ggacattacc agttccttag gctggtatca gcagagagca    120

ggggaagccc cgaagctcct gctctttgct gcatccagat tggaaagtgg ggtcccgtcc    180

aggttcagtg gcagtggatc tgggacagaa tatactctca ccatcagcaa cctgcagcct    240

gaagattttg caacttatta ctgtctacac tattttggtt cccgtccgtg gacgttcggc    300

caagggacca aggtggagat caaacgtg                                       328


<210>  17
<211>  333
<212>  DNA
<213>  Artificial

<220>
<223>  Variable region of human antibody

<400>  17
cagtctgtgc tgacgcagcc gccctcagtg tctggggccc cagggcagag ggtcaccttc     60

tcctgcactg ggagcagctc caacatcggg gcaggttatg atgtaaactg gtaccagcag    120

cttccaggaa cagcccccaa agtcctcatc tatggcaaca ccaatcggcc ctcaggggtc    180

cctgatcgat tctctgcctc caagtctggc acctcagcct ccctggccat cactgggctc    240

caggctgagg atgaggctga ttattactgc cagtcctatg acagtagcct gaggggttat    300

gtcttcggaa cagggaccaa ggtcagcgtc cta                                 333


<210>  18
<211>  325
<212>  DNA
<213>  Artificial

<220>
<223>  Variable region of human antibody

<400>  18
gaaattgtgt tgacacagtc tccagccacc ctgtctgtgt ctccagggga tagagccacc      60

ctctcctgca gggccagtca gagtgttagc gacaagttag cctggtacca gcagaaacct     120

ggccaggctc ccaggctcct catctatggt gcatacacca gggccacagg taccccagcc     180

aggttcagtg gcagtgggtc tgggacagag ttcactctca gcatcacagg tctgcaggct     240

gaagattttg caatttatta ctgtcaccaa tacaataact ggccgctcac tttcggcgga     300

gggaccaagg tggayatcaa acgtg                                           325


<210>  19
<211>  330
<212>  DNA
<213>  Artificial

<220>
<223>  Variable region of human antibody

<400>  19
cagtctgtgc tgacgcagcc gccctcagtg tctgcggccc caggacagaa ggtcaccatc      60

tcctgctctg gacgcagctc caacgttggg gataattatg tgtcctggta ccagcagttc     120

ccaggaacag cccccaaact ccttatctat gacaatacta agcgcccctc agggattcct     180

gaccgattct ctggctccaa gtctggcacg tcagccaccc tgggcatcac cggactccag     240

actggagacg aggccgctta ttactgcgca acatgggata gcgttctgag tgctggggtt     300

ttcggcggag ggaccaagct gaccgtccta                                      330


<210>  20
<211>  324
<212>  DNA
<213>  Artificial

<220>
<223>  Variable region of human antibody

<400>  20
gacatccaga tgacccagtc tccatcctcc ctgtctgcat ctgtaggaga cagagtcacc      60

atcacttgcc gggcaagtca gaccattagc aactttttaa attggtatca gcagaaacca     120

ggaaaagccc ctaaactcct gatctatgct gcatccagat tgcaaagttg ggtcccatca     180

aggttcactg gcagtggatc tgggacagat ttcgctctca ccattagcag tctgcaacct     240

gaagattttg caacttacta ctgtcagcag actttcagtc cccgaatcac tttcggccct     300

gggaccaagg tggaaatgaa acgt                                            324

<210>  21
<211>  323
<212>  DNA
<213>  Artificial

<220>
<223>  Variable region of human antibody

<400>  21
gacatccaga tgacccagtc tccatcctcc ctgtctgcat ctataggaga cagagtcacc      60

atcacttgcc gggcgagtcc gggcattaac agttatttag cctggtatca gcacaaacca     120

gggaaagctc ctaagctcct gatctatgct gcctccactt tgcagtccgg ggtcccatct     180

cggttcagtg gcagtggttc tgggacagac ttcactctca ccatcagcag cctgcagcct     240

gaagatgttg caacttatta ctgtcagaag tataatagtg ccccgtggac gttcggccca     300

gggaccaagg tggaaatcaa acg                                             323


<210>  22
<211>  108
<212>  PRT
<213>  Artificial

<220>
<223>  Variable region of human antibody

<400>  22

Asp Ile Gln Met Thr Gln Ser Pro Ser Ser Leu Ser Ala Ser Val Gly
1               5                   10                  15


Asp Arg Val Ile Ile Thr Cys Arg Ala Ser Gln Gly Ile Thr Arg His
            20                  25                  30


Leu Asn Trp Tyr Gln Gln Lys Ser Gly Lys Ala Pro Lys Leu Leu Ile
        35                  40                  45


Tyr Asp Ala Ser Ser Leu Gln Ser Gly Val Pro Ser Arg Phe Ser Gly
    50                  55                  60


Ser Gly Ser Gly Ala Asp Phe Thr Leu Thr Ile Ser Ser Val Gln Pro
65                  70                  75                  80


Glu Asp Phe Ala Thr Tyr Tyr Cys Gln Gln Ser Tyr Ser Asn Pro Pro
                85                  90                  95


Thr Phe Gly Pro Gly Thr Lys Val Asp Ile Lys Arg
            100                 105


<210>  23

<211>  109
<212>  PRT
<213>  Artificial

<220>
<223>  Variable region of human antibody

<400>  23

Glu Ile Val Leu Thr Gln Ser Pro Ala Thr Leu Ser Ala Ser Val Gly
1               5                   10                  15

Asp Arg Val Thr Ile Thr Cys Arg Ala Ser Gln Asp Ile Thr Ser Ser
            20                  25                  30

Leu Gly Trp Tyr Gln Gln Arg Ala Gly Glu Ala Pro Lys Leu Leu Leu
        35                  40                  45

Phe Ala Ala Ser Arg Leu Glu Ser Gly Val Pro Ser Arg Phe Ser Gly
    50                  55                  60

Ser Gly Ser Gly Thr Glu Tyr Thr Leu Thr Ile Ser Asn Leu Gln Pro
65                  70                  75                  80

Glu Asp Phe Ala Thr Tyr Tyr Cys Leu His Tyr Phe Gly Ser Arg Pro
                85                  90                  95

Trp Thr Phe Gly Gln Gly Thr Lys Val Glu Ile Lys Arg
            100                 105


<210>  24
<211>  111
<212>  PRT
<213>  Artificial

<220>
<223>  Variable region of human antibody

<400>  24

Gln Ser Val Leu Thr Gln Pro Pro Ser Val Ser Gly Ala Pro Gly Gln
1               5                   10                  15

Arg Val Thr Phe Ser Cys Thr Gly Ser Ser Ser Asn Ile Gly Ala Gly
            20                  25                  30

Tyr Asp Val Asn Trp Tyr Gln Gln Leu Pro Gly Thr Ala Pro Lys Val
        35                  40                  45

Leu Ile Tyr Gly Asn Thr Asn Arg Pro Ser Gly Val Pro Asp Arg Phe
    50                  55                  60

Ser Ala Ser Lys Ser Gly Thr Ser Ala Ser Leu Ala Ile Thr Gly Leu
65                  70                  75                  80

Gln Ala Glu Asp Glu Ala Asp Tyr Tyr Cys Gln Ser Tyr Asp Ser Ser
                85                  90                  95

Leu Arg Gly Tyr Val Phe Gly Thr Gly Thr Lys Val Ser Val Leu
            100                 105                 110

<210>  25
<211>  107
<212>  PRT
<213>  Artificial

<220>
<223>  Variable region of human antibody

<400>  25

Glu Ile Val Leu Thr Gln Ser Pro Ala Thr Leu Ser Val Ser Pro Gly
1               5                   10                  15

Asp Arg Ala Thr Leu Ser Cys Arg Ala Ser Gln Ser Val Ser Asp Lys
            20                  25                  30

Leu Ala Trp Tyr Gln Gln Lys Pro Gly Gln Ala Pro Arg Leu Leu Ile
        35                  40                  45

Tyr Gly Ala Tyr Thr Arg Ala Thr Gly Thr Pro Ala Arg Phe Ser Gly
    50                  55                  60

Ser Gly Ser Gly Thr Glu Phe Thr Leu Ser Ile Thr Gly Leu Gln Ala
65                  70                  75                  80

Glu Asp Phe Ala Ile Tyr Tyr Cys His Gln Tyr Asn Asn Trp Pro Leu
                85                  90                  95

Thr Phe Gly Gly Gly Thr Lys Val Asp Ile Lys
            100                 105

<210>  26
<211>  110
<212>  PRT
<213>  Artificial

<220>
<223>  Variable region of human antibody

<400>  26

Gln Ser Val Leu Thr Gln Pro Pro Ser Val Ser Ala Ala Pro Gly Gln
1               5                   10                  15

Lys Val Thr Ile Ser Cys Ser Gly Arg Ser Ser Asn Val Gly Asp Asn
            20                  25                  30

Tyr Val Ser Trp Tyr Gln Gln Phe Pro Gly Thr Ala Pro Lys Leu Leu
        35                  40                  45

Ile Tyr Asp Asn Thr Lys Arg Pro Ser Gly Ile Pro Asp Arg Phe Ser
    50                  55                  60

Gly Ser Lys Ser Gly Thr Ser Ala Thr Leu Gly Ile Thr Gly Leu Gln
65                  70                  75                  80

Thr Gly Asp Glu Ala Ala Tyr Tyr Cys Ala Thr Trp Asp Ser Val Leu
                85                  90                  95

Ser Ala Gly Val Phe Gly Gly Gly Thr Lys Leu Thr Val Leu
            100                 105                 110

<210>  27
<211>  108
<212>  PRT
<213>  Artificial

<220>
<223>  Variable region of human antibody

<400>  27

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Thr Ile Ser Asn Phe
            20                  25                  30

Leu Asn Trp Tyr Gln Gln Lys Pro Gly Lys Ala Pro Lys Leu Leu Ile
        35                  40                  45

Tyr Ala Ala Ser Arg Leu Gln Ser Trp Val Pro Ser Arg Phe Thr Gly
    50                  55                  60

Ser Gly Ser Gly Thr Asp Phe Ala Leu Thr Ile Ser Ser Leu Gln Pro
65                  70                  75                  80

Glu Asp Phe Ala Thr Tyr Tyr Cys Gln Gln Thr Phe Ser Pro Arg Ile
                85                  90                  95

Thr Phe Gly Pro Gly Thr Lys Val Glu Met Lys Arg
            100                 105

<210>  28
<211>  107
<212>  PRT
<213>  Artificial

<220>
<223>  Variable region of human antibody

<400>  28

Asp Ile Gln Met Thr Gln Ser Pro Ser Ser Leu Ser Ala Ser Ile Gly
1               5                   10                  15

Asp Arg Val Thr Ile Thr Cys Arg Ala Ser Pro Gly Ile Asn Ser Tyr
            20                  25                  30

Leu Ala Trp Tyr Gln His Lys Pro Gly Lys Ala Pro Lys Leu Leu Ile
        35                  40                  45

Tyr Ala Ala Ser Thr Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Val Ala Thr Tyr Tyr Cys Gln Lys Tyr Asn Ser Ala Pro Trp
                85                  90                  95

Thr Phe Gly Pro Gly Thr Lys Val Glu Ile Lys
            100                 105

<210>  29
<211>  65
<212>  DNA
<213>  Artificial

<220>
<223>  an artificially synthesized primer sequence

<220>
<221>  misc_feature
<222>  (65)..(65)
<223>  n is a, c, g, or t

<400>  29
acagcaggtc agtcaagcag tagcagcagt tcgataagcg gccgccatgg accccccccc      60

cccdn                                                                    65

<210>  30
<211>  22
<212>  DNA
<213>  Artificial

<220>
<223>  an artificially synthesized primer sequence

<400>  30
acagcaggtc agtcaagcag ta                                                 22

<210>  31
<211>  22
<212>  DNA
<213>  Artificial

<220>
<223>  an artificially synthesized primer sequence

<400>  31
agcagtagca gcagttcgat aa                                                 22

<210>  32
<211>  20
<212>  DNA
<213>  Artificial

<220>
<223>  an artificially synthesized primer sequence

<400>  32
aggtgtgcac gccgctggtc                                                    20

<210>  33
<211>  20
<212>  DNA
<213>  Artificial

<220>
<223>  an artificially synthesized primer sequence

<400>  33
cgcctgagtt ccacgacacc                                                    20

<210>  34
<211>  20
<212>  DNA
<213>  Artificial

<220>
<223>  an artificially synthesized primer sequence

<400>  34
tcggggaagt agtccttgac                                                    20

<210>  35
<211>  19
<212>  DNA
<213>  Artificial

<220>
<223>  an artificially synthesized primer sequence

<400>  35
acacyagtgt ggccttgtt                                                    19


<210>  36
<211>  19
<212>  DNA
<213>  Artificial

<220>
<223>  an artificially synthesized primer sequence

<400>  36
gcttgragct cctcagagg                                                    19


<210>  37
<211>  19
<212>  DNA
<213>  Artificial

<220>
<223>  an artificially synthesized primer sequence

<400>  37
gggygggaac agagtgacc                                                    19


<210>  38
<211>  20
<212>  DNA
<213>  Artificial

<220>
<223>  an artificially synthesized primer sequence

<400>  38
gttattcagc aggcacacaa                                                   20


<210>  39
<211>  20
<212>  DNA
<213>  Artificial

<220>
<223>  an artificially synthesized primer sequence

<400>  39
gaggcagttc cagatttcaa                                                   20

<210>  40
<211>  21
<212>  DNA
<213>  Artificial

<220>
<223>  an artificially synthesized primer sequence

<400>  40
gggaagatga agacagatgg t                                                21

SEQUENCE LISTING

<110>  NATIONAL UNIVERSITY CORPORATION UNIVERSITY OF TOYAMA
       SC WORLD, INC.
       KYOWA MEDEX CO.,LTD

<120>  Device for detecting influenza virus

<130>  1000P12013WO0

<150>  JP 2008-149300
<151>  2008-06-06

<150>  JP 2009-050066
<151>  2009-03-04

<160>  40

<170>  PatentIn version 3.4

<210>  1
<211>  379
<212>  DNA
<213>  Artificial

<220>
<223>  Variable region of human antibody

<400>  1
caggtgcagc tggtgcagtc tggggctgtg gtgaagaagc ctggggactc agtgaaggtc      60

tcctgcaagg catctggata cacgttcacc acctactgga tgcactgggt gcgacaggcc     120

cctggacaag ggcccgaatg gatgggaata gtcaatccca caggtggcac cgttatgtac     180

gcaaagaagt tccagggtcg ggtcatcatg accagggaca cgtccacgac gacagtcttt     240

ttgcaactga acggcctgga atctgaggac accgccatat attactgtgc gagagatctg     300

gggcctgcaa gggtggattt tgactggttg tccacctttg tctcctgggg ccagggaacc     360

ctggtcaccg tctcctcag                                                  379

<210>  2
<211>  358
<212>  DNA
<213>  Artificial

<220>
<223>  Variable region of human antibody

<400>  2
caggtgcagc tggtgcagtc tggggctgag gtgaagaagc ctgggtcctc ggtgaaggta      60

tcctgcaagg ctaccggaga caccttcagc agctattcta tcagctgggt gcgacaggct     120

cctggacaag ggcttgagtg gatgggaagg gtcacccctt cctttggaat atcactctac     180

gcacagaaat tccagggcag agtcaccatt accgcggaca gatcctcgag cacagcctat     240

atggagctga acaacctgac atctgatgac accgccgtgt attactgtgc ggctgggggt     300

cttgtagtgg tggtggcgaa ccactggggc cagggaaccc tggtcaccgt ctcctcag       358

<210>  3
<211>  351

<212>  DNA
<213>  Artificial

<220>
<223>  Variable region of human antibody

<400>  3
caggtgcagc tggtgcagtc tgggggaggc gtggtccagc ctgggaggtc cctgagactc      60

tcctgtgcag cgtctggatt caccttcagt agatatggca tgcactgggt ccgccaggct     120

ccaggcaagg ggctggagtg ggtggcagtg atattatatg agggaattaa taaagactat     180

gcagactccg tgaagggccg attcaccatc tccagagaca actccaagaa cacactgtat     240

ttgcaaatga atagtctgag agccgaggac acggctgtgt attactgtgc gagagatcgt     300

gagctgcgac ttgactactg gggccaggga accctggtca ccgtctcctc a              351

<210>  4
<211>  401
<212>  DNA
<213>  Artificial

<220>
<223>  Variable region of human antibody

<400>  4
caggtgcagc tggtgcagtc tgggggaggc ttggtacagc ctggcaggtc cctgagactc      60

tcttgtatag cctctggatt cagttttgat gattatgcca tgcactgggt ccggcaaatt     120

ccagggaagg gcctggagtg ggtctcaggt attagttgga acagtggtgc cagaggctat     180

gcggactctg tgaagggccg atttaccatc tccagagaca acgccaaaaa ttccctgtat     240

ctacaaatga acagtctcag agttggggac acggccttct attactgtgc cagagacatg     300

ggggccgggc caaatgatta tgataccagt ggcaattacc cccttcacct ctatggcatg     360

gacgtctggg gccatgggac cacggtcacc gtctcctcag g                         401

<210>  5
<211>  359
<212>  DNA
<213>  Artificial

<220>
<223>  Variable region of human antibody

<400>  5
caggtgcagc tgcaggagtc gggcccagga ctggtgaagc cttcacagac cctgtccctc      60

acctgcagtg tctctggtgg ctccatcagc aatcctgatc actactggaa ctggatccga     120

cagcccccag ggaagggcct tgagtggatt gggtacatct attacactgg gcgcacctgg     180

tacagcccgt ccctcaagag tcgcgtttcc ttatcagtag acacgtccaa gagccagttc     240

tccttgaacg tggactctgt gattgccgca gacacggccg tatattactg cgccagaagt     300

cacgtgtggt tcggggagtt atactggggc cagggagccc tggtcaccgt ctcctcagg      359

<210>  6
<211>  355

<212>  DNA
<213>  Artificial

<220>
<223>  Variable region of human antibody

<400>  6
caggtgcagc tggtgcagtc tgggggaggc ttggtcaagc ctggagggtc cctgagactc      60

tcctgtgcag gctctggatt cagcttcagt gactattata tgacgtggat ccgccaggct     120

ccagggaagg ggctggagtg ggtttcatcc atcaatagtg gcagcactgt cacaaactac     180

gcagactctg tgcagggccg attcaccatc tccagagaca agaactcagt gtatctgcaa     240

atgaacagcc tgagaaccga ggacacggcc gtctattact gtgcgagaga ttttggtatc     300

aaagcgggta cttcggacta ctggggccag ggaaccctgg tcaccgtctc ctccg          355


<210>  7
<211>  393
<212>  DNA
<213>  Artificial

<220>
<223>  Variable region of human antibody

<400>  7
caggtgcagc tggtgcagtc tggggctgag gtgaagaggc ctggggcctc agtgaaggtc      60

tcctgcaagg cttctggata caacctcatc gacttctatg tgaactgggt gcgacaggtc     120

cctggacaag gacttgagtg gatggggtac atcaaccctg acagtggtgg cgcacactat     180

gaagggaaat ttcagggcag ggtcatcatg accagggaca cgtccatcaa cacagcctat     240

atggaattgc ccagcctgac atctgacgac acggccgtct atttctgtgc gagaggggct     300

agtagtggct tgttgaccta ctattacggt ttggacgtct ggggcgaagg gaccacggtt     360

atcgtctcct caggtgagtg tcccaagcag ctt                                  393


<210>  8
<211>  126
<212>  PRT
<213>  Artificial

<220>
<223>  Variable region of human antibody

<400>  8

Gln Val Gln Leu Val Gln Ser Gly Ala Val Val Lys Lys Pro Gly Asp
1               5                   10                  15


Ser Val Lys Val Ser Cys Lys Ala Ser Gly Tyr Thr Phe Thr Thr Tyr
            20                  25                  30


Trp Met His Trp Val Arg Gln Ala Pro Gly Gln Gly Pro Glu Trp Met
        35                  40                  45


Gly Ile Val Asn Pro Thr Gly Gly Thr Val Met Tyr Ala Lys Lys Phe
    50                  55                  60

Gln Gly Arg Val Ile Met Thr Arg Asp Thr Ser Thr Thr Thr Val Phe
65                  70                  75                  80

Leu Gln Leu Asn Gly Leu Glu Ser Glu Asp Thr Ala Ile Tyr Tyr Cys
                85                  90                  95

Ala Arg Asp Leu Gly Pro Ala Arg Val Asp Phe Asp Trp Leu Ser Thr
            100                 105                 110

Phe Val Ser Trp Gly Gln Gly Thr Leu Val Thr Val Ser Ser
        115                 120                 125

<210>  9
<211>  119
<212>  PRT
<213>  Artificial

<220>
<223>  Variable region of human antibody

<400>  9

Gln Val Gln Leu Val Gln Ser Gly Ala Glu Val Lys Lys Pro Gly Ser
1               5                   10                  15

Ser Val Lys Val Ser Cys Lys Ala Thr Gly Asp Thr Phe Ser Ser Tyr
            20                  25                  30

Ser Ile Ser Trp Val Arg Gln Ala Pro Gly Gln Gly Leu Glu Trp Met
        35                  40                  45

Gly Arg Val Thr Pro Ser Phe Gly Ile Ser Leu Tyr Ala Gln Lys Phe
    50                  55                  60

Gln Gly Arg Val Thr Ile Thr Ala Asp Arg Ser Ser Ser Thr Ala Tyr
65                  70                  75                  80

Met Glu Leu Asn Asn Leu Thr Ser Asp Asp Thr Ala Val Tyr Tyr Cys
                85                  90                  95

Ala Ala Gly Gly Leu Val Val Val Val Ala Asn His Trp Gly Gln Gly
            100                 105                 110

Thr Leu Val Thr Val Ser Ser
        115

<210>  10
<211>  117
<212>  PRT
<213>  Artificial

<220>
<223>  Variable region of human antibody

<400>  10

Gln Val Gln Leu Val Gln Ser Gly Gly Gly Val Val Gln Pro Gly Arg
1               5                   10                  15

Ser Leu Arg Leu Ser Cys Ala Ala Ser Gly Phe Thr Phe Ser Arg Tyr
            20                  25                  30

Gly Met His Trp Val Arg Gln Ala Pro Gly Lys Gly Leu Glu Trp Val
        35                  40                  45

Ala Val Ile Leu Tyr Glu Gly Ile Asn Lys Asp Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Asp Arg Glu Leu Arg Leu Asp Tyr Trp Gly Gln Gly Thr Leu
            100                 105                 110

Val Thr Val Ser Ser
        115

<210>  11
<211>  133
<212>  PRT
<213>  Artificial

<220>
<223>  Variable region of human antibody

<400>  11

Gln Val Gln Leu Val Gln Ser Gly Gly Gly Leu Val Gln Pro Gly Arg
1               5                   10                  15

Ser Leu Arg Leu Ser Cys Ile Ala Ser Gly Phe Ser Phe Asp Asp Tyr
            20                  25                  30

Ala Met His Trp Val Arg Gln Ile Pro Gly Lys Gly Leu Glu Trp Val
        35                  40                  45

Ser Gly Ile Ser Trp Asn Ser Gly Ala Arg Gly Tyr Ala Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Val Gly Asp Thr Ala Phe Tyr Tyr Cys
                85                  90                  95

Ala Arg Asp Met Gly Ala Gly Pro Asn Asp Tyr Asp Thr Ser Gly Asn
            100                 105                 110

Tyr Pro Leu His Leu Tyr Gly Met Asp Val Trp Gly His Gly Thr Thr
        115                 120                 125

Val Thr Val Ser Ser
    130

<210>  12
<211>  119
<212>  PRT
<213>  Artificial

<220>
<223>  variable region of human antibody

<400>  12

Gln Val Gln Leu Gln Glu Ser Gly Pro Gly Leu Val Lys Pro Ser Gln
1               5                   10                  15

Thr Leu Ser Leu Thr Cys Ser Val Ser Gly Gly Ser Ile Ser Asn Pro
            20                  25                  30

Asp His Tyr Trp Asn Trp Ile Arg Gln Pro Pro Gly Lys Gly Leu Glu
        35                  40                  45

Trp Ile Gly Tyr Ile Tyr Tyr Thr Gly Arg Thr Trp Tyr Ser Pro Ser
    50                  55                  60

Leu Lys Ser Arg Val Ser Leu Ser Val Asp Thr Ser Lys Ser Gln Phe
65                  70                  75                  80

Ser Leu Asn Val Asp Ser Val Ile Ala Ala Asp Thr Ala Val Tyr Tyr
                85                  90                  95

Cys Ala Arg Ser His Val Trp Phe Gly Glu Leu Tyr Trp Gly Gln Gly
            100                 105                 110

Ala Leu Val Thr Val Ser Ser
        115

<210>  13
<211>  118
<212>  PRT
<213>  Artificial

<220>
<223>  variable region of human antibody

<400>  13

Gln Val Gln Leu Val Gln Ser Gly Gly Gly Leu Val Lys Pro Gly Gly
1               5                   10                  15

Ser Leu Arg Leu Ser Cys Ala Gly Ser Gly Phe Ser Phe Ser Asp Tyr
            20                  25                  30

Tyr Met Thr Trp Ile Arg Gln Ala Pro Gly Lys Gly Leu Glu Trp Val
        35                  40                  45

Ser Ser Ile Asn Ser Gly Ser Thr Val Thr Asn Tyr Ala Asp Ser Val
    50                  55                  60

Gln Gly Arg Phe Thr Ile Ser Arg Asp Lys Asn Ser Val Tyr Leu Gln
65                  70                  75                  80

Met Asn Ser Leu Arg Thr Glu Asp Thr Ala Val Tyr Tyr Cys Ala Arg
                85                  90                  95

Asp Phe Gly Ile Lys Ala Gly Thr Ser Asp Tyr Trp Gly Gln Gly Thr
            100                 105                 110

Leu Val Thr Val Ser Ser
        115

<210>  14
<211>  124
<212>  PRT
<213>  Artificial

<220>
<223>  Variable region of human antibody

<400>  14

Gln Val Gln Leu Val Gln Ser Gly Ala Glu Val Lys Arg Pro Gly Ala
1               5                   10                  15

Ser Val Lys Val Ser Cys Lys Ala Ser Gly Tyr Asn Leu Ile Asp Phe
            20                  25                  30

Tyr Val Asn Trp Val Arg Gln Val Pro Gly Gln Gly Leu Glu Trp Met
        35                  40                  45

Gly Tyr Ile Asn Pro Asp Ser Gly Gly Ala His Tyr Glu Gly Lys Phe
    50                  55                  60

Gln Gly Arg Val Ile Met Thr Arg Asp Thr Ser Ile Asn Thr Ala Tyr
65                  70                  75                  80

Met Glu Leu Pro Ser Leu Thr Ser Asp Asp Thr Ala Val Tyr Phe Cys
                85                  90                  95

Ala Arg Gly Ala Ser Ser Gly Leu Leu Thr Tyr Tyr Tyr Gly Leu Asp
            100                 105                 110

Val Trp Gly Glu Gly Thr Thr Val Ile Val Ser Ser
        115                 120

<210>  15
<211>  327
<212>  DNA
<213>  Artificial

<220>
<223>  Variable region of human antibody

<400>  15
gacatccaga tgacccagtc tccatcgtcc ctgtctgcat ctgtgggaga cagagtcatc      60

atcacttgcc gggcaagtca gggcattacc agacacttaa attggtatca gcagaaatca     120

ggcaaagccc ccaaactcct gatctacgat gcgtccagtt tgcaaagtgg ggtcccatcc     180

aggttcagtg gcagtggatc tggggcagat ttcactctca ccatcagcag tgtgcaacct     240

gaagattttg caacttacta ctgtcaacag agttatagta accctccgac tttcggcccc     300

gggaccaaag tggatatcaa acgtgag                                         327

<210>  16
<211>  328
<212>  DNA
<213>  Artificial

<220>
<223>  Variable region of human antibody

<400>  16
gaaattgtgt tgacacagtc tccagccacc ctgtctgcat ctgtgggaga cagagtcacc      60

atcacttgcc gggcgagtca ggacattacc agttccttag gctggtatca gcagagagca     120

ggggaagccc cgaagctcct gctctttgct gcatccagat tggaaagtgg ggtcccgtcc     180

aggttcagtg gcagtggatc tgggacagaa tatactctca ccatcagcaa cctgcagcct     240

gaagattttg caacttatta ctgtctacac tattttggtt cccgtccgtg gacgttcggc     300

caagggacca aggtggagat caaacgtg                                        328

<210>  17
<211>  333
<212>  DNA
<213>  Artificial

<220>
<223>  Variable region of human antibody

<400>  17
cagtctgtgc tgacgcagcc gccctcagtg tctggggccc cagggcagag ggtcaccttc      60

tcctgcactg ggagcagctc caacatcggg gcaggttatg atgtaaactg gtaccagcag     120

cttccaggaa cagcccccaa agtcctcatc tatggcaaca ccaatcggcc ctcaggggtc     180

cctgatcgat tctctgcctc caagtctggc acctcagcct ccctggccat cactgggctc     240

caggctgagg atgaggctga ttattactgc cagtcctatg acagtagcct gaggggttat     300

gtcttcggaa cagggaccaa ggtcagcgtc cta                                  333

<210>  18
<211>  325
<212>  DNA
<213>  Artificial

<220>
<223>  Variable region of human antibody

<400>  18
gaaattgtgt tgacacagtc tccagccacc ctgtctgtgt ctccagggga tagagccacc      60

ctctcctgca gggccagtca gagtgttagc gacaagttag cctggtacca gcagaaacct     120

ggccaggctc ccaggctcct catctatggt gcatacacca gggccacagg taccccagcc     180

aggttcagtg gcagtgggtc tgggacagag ttcactctca gcatcacagg tctgcaggct     240

gaagattttg caatttatta ctgtcaccaa tacaataact ggccgctcac tttcggcgga     300

gggaccaagg tggayatcaa acgtg                                           325


<210>  19
<211>  330
<212>  DNA
<213>  Artificial

<220>
<223>  Variable region of human antibody

<400>  19
cagtctgtgc tgacgcagcc gccctcagtg tctgcggccc caggacagaa ggtcaccatc      60

tcctgctctg gacgcagctc caacgttggg gataattatg tgtcctggta ccagcagttc     120

ccaggaacag cccccaaact ccttatctat gacaatacta agcgcccctc agggattcct     180

gaccgattct ctggctccaa gtctggcacg tcagccaccc tgggcatcac cggactccag     240

actggagacg aggccgctta ttactgcgca acatgggata gcgttctgag tgctggggtt     300

ttcggcggag ggaccaagct gaccgtccta                                      330


<210>  20
<211>  324
<212>  DNA
<213>  Artificial

<220>
<223>  Variable region of human antibody

<400>  20
gacatccaga tgacccagtc tccatcctcc ctgtctgcat ctgtaggaga cagagtcacc      60

atcacttgcc gggcaagtca gaccattagc aactttttaa attggtatca gcagaaacca     120

ggaaaagccc ctaaactcct gatctatgct gcatccagat tgcaaagttg ggtcccatca     180

aggttcactg gcagtggatc tgggacagat ttcgctctca ccattagcag tctgcaacct     240

gaagattttg caacttacta ctgtcagcag actttcagtc cccgaatcac tttcggccct     300

gggaccaagg tggaaatgaa acgt                                            324


<210>  21
<211>  323

<212>  DNA
<213>  Artificial

<220>
<223>  Variable region of human antibody

<400>  21
gacatccaga tgacccagtc tccatcctcc ctgtctgcat ctataggaga cagagtcacc      60

atcacttgcc gggcgagtcc gggcattaac agttatttag cctggtatca gcacaaacca     120

gggaaagctc ctaagctcct gatctatgct gcctccactt tgcagtccgg ggtcccatct     180

cggttcagtg gcagtggttc tgggacagac ttcactctca ccatcagcag cctgcagcct     240

gaagatgttg caacttatta ctgtcagaag tataatagtg ccccgtggac gttcggccca     300

gggaccaagg tggaaatcaa acg                                             323


<210>  22
<211>  108
<212>  PRT
<213>  Artificial

<220>
<223>  Variable region of human antibody

<400>  22

Asp Ile Gln Met Thr Gln Ser Pro Ser Ser Leu Ser Ala Ser Val Gly
1               5                   10                  15


Asp Arg Val Ile Ile Thr Cys Arg Ala Ser Gln Gly Ile Thr Arg His
            20                  25                  30


Leu Asn Trp Tyr Gln Gln Lys Ser Gly Lys Ala Pro Lys Leu Leu Ile
        35                  40                  45


Tyr Asp Ala Ser Ser Leu Gln Ser Gly Val Pro Ser Arg Phe Ser Gly
    50                  55                  60


Ser Gly Ser Gly Ala Asp Phe Thr Leu Thr Ile Ser Ser Val Gln Pro
65                  70                  75                  80


Glu Asp Phe Ala Thr Tyr Tyr Cys Gln Gln Ser Tyr Ser Asn Pro Pro
                85                  90                  95


Thr Phe Gly Pro Gly Thr Lys Val Asp Ile Lys Arg
            100                 105


<210>  23
<211>  109
<212>  PRT
<213>  Artificial

<220>
<223>  Variable region of human antibody

<400>  23

Glu Ile Val Leu Thr Gln Ser Pro Ala Thr Leu Ser Ala Ser Val Gly
1               5                   10                  15

Asp Arg Val Thr Ile Thr Cys Arg Ala Ser Gln Asp Ile Thr Ser Ser
            20                  25                  30

Leu Gly Trp Tyr Gln Gln Arg Ala Gly Glu Ala Pro Lys Leu Leu Leu
        35                  40                  45

Phe Ala Ala Ser Arg Leu Glu Ser Gly Val Pro Ser Arg Phe Ser Gly
    50                  55                  60

Ser Gly Ser Gly Thr Glu Tyr Thr Leu Thr Ile Ser Asn Leu Gln Pro
65                  70                  75                  80

Glu Asp Phe Ala Thr Tyr Tyr Cys Leu His Tyr Phe Gly Ser Arg Pro
                85                  90                  95

Trp Thr Phe Gly Gln Gly Thr Lys Val Glu Ile Lys Arg
            100                 105

<210>  24
<211>  111
<212>  PRT
<213>  Artificial

<220>
<223>  Variable region of human antibody

<400>  24

Gln Ser Val Leu Thr Gln Pro Pro Ser Val Ser Gly Ala Pro Gly Gln
1               5                   10                  15

Arg Val Thr Phe Ser Cys Thr Gly Ser Ser Ser Asn Ile Gly Ala Gly
            20                  25                  30

Tyr Asp Val Asn Trp Tyr Gln Gln Leu Pro Gly Thr Ala Pro Lys Val
        35                  40                  45

Leu Ile Tyr Gly Asn Thr Asn Arg Pro Ser Gly Val Pro Asp Arg Phe
    50                  55                  60

Ser Ala Ser Lys Ser Gly Thr Ser Ala Ser Leu Ala Ile Thr Gly Leu
65                  70                  75                  80

Gln Ala Glu Asp Glu Ala Asp Tyr Tyr Cys Gln Ser Tyr Asp Ser Ser
                85                  90                  95

Leu Arg Gly Tyr Val Phe Gly Thr Gly Thr Lys Val Ser Val Leu
            100                 105                 110

<210>  25
<211>  107

<212>  PRT
<213>  Artificial

<220>
<223>  Variable region of human antibody

<400>  25

Glu Ile Val Leu Thr Gln Ser Pro Ala Thr Leu Ser Val Ser Pro Gly
1               5                   10                  15

Asp Arg Ala Thr Leu Ser Cys Arg Ala Ser Gln Ser Val Ser Asp Lys
            20                  25                  30

Leu Ala Trp Tyr Gln Gln Lys Pro Gly Gln Ala Pro Arg Leu Leu Ile
        35                  40                  45

Tyr Gly Ala Tyr Thr Arg Ala Thr Gly Thr Pro Ala Arg Phe Ser Gly
    50                  55                  60

Ser Gly Ser Gly Thr Glu Phe Thr Leu Ser Ile Thr Gly Leu Gln Ala
65                  70                  75                  80

Glu Asp Phe Ala Ile Tyr Tyr Cys His Gln Tyr Asn Asn Trp Pro Leu
                85                  90                  95

Thr Phe Gly Gly Gly Thr Lys Val Asp Ile Lys
            100                 105

<210>  26
<211>  110
<212>  PRT
<213>  Artificial

<220>
<223>  Variable region of human antibody

<400>  26

Gln Ser Val Leu Thr Gln Pro Pro Ser Val Ser Ala Ala Pro Gly Gln
1               5                   10                  15

Lys Val Thr Ile Ser Cys Ser Gly Arg Ser Ser Asn Val Gly Asp Asn
            20                  25                  30

Tyr Val Ser Trp Tyr Gln Gln Phe Pro Gly Thr Ala Pro Lys Leu Leu
        35                  40                  45

Ile Tyr Asp Asn Thr Lys Arg Pro Ser Gly Ile Pro Asp Arg Phe Ser
    50                  55                  60

Gly Ser Lys Ser Gly Thr Ser Ala Thr Leu Gly Ile Thr Gly Leu Gln
65                  70                  75                  80

Thr Gly Asp Glu Ala Ala Tyr Tyr Cys Ala Thr Trp Asp Ser Val Leu
                85                  90                  95

Ser Ala Gly Val Phe Gly Gly Gly Thr Lys Leu Thr Val Leu
            100                 105                 110

<210>  27
<211>  108
<212>  PRT
<213>  Artificial

<220>
<223>  Variable region of human antibody

<400>  27

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Thr Ile Ser Asn Phe
            20                  25                  30

Leu Asn Trp Tyr Gln Gln Lys Pro Gly Lys Ala Pro Lys Leu Leu Ile
        35                  40                  45

Tyr Ala Ala Ser Arg Leu Gln Ser Trp Val Pro Ser Arg Phe Thr Gly
    50                  55                  60

Ser Gly Ser Gly Thr Asp Phe Ala Leu Thr Ile Ser Ser Leu Gln Pro
65                  70                  75                  80

Glu Asp Phe Ala Thr Tyr Tyr Cys Gln Gln Thr Phe Ser Pro Arg Ile
                85                  90                  95

Thr Phe Gly Pro Gly Thr Lys Val Glu Met Lys Arg
            100                 105

<210>  28
<211>  107
<212>  PRT
<213>  Artificial

<220>
<223>  Variable region of human antibody

<400>  28

Asp Ile Gln Met Thr Gln Ser Pro Ser Ser Leu Ser Ala Ser Ile Gly
1               5                   10                  15

Asp Arg Val Thr Ile Thr Cys Arg Ala Ser Pro Gly Ile Asn Ser Tyr
            20                  25                  30

Leu Ala Trp Tyr Gln His Lys Pro Gly Lys Ala Pro Lys Leu Leu Ile
        35                  40                  45

Tyr Ala Ala Ser Thr Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Val Ala Thr Tyr Tyr Cys Gln Lys Tyr Asn Ser Ala Pro Trp
                85                  90                  95

Thr Phe Gly Pro Gly Thr Lys Val Glu Ile Lys
            100                 105

<210>  29
<211>  65
<212>  DNA
<213>  Artificial

<220>
<223>  an artificially synthesized primer sequence

<220>
<221>  misc_feature
<222>  (65)..(65)
<223>  n is a, c, g, or t

<400>  29
acagcaggtc agtcaagcag tagcagcagt tcgataagcg gccgccatgg accccccccc      60

cccdn                                                                  65

<210>  30
<211>  22
<212>  DNA
<213>  Artificial

<220>
<223>  an artificially synthesized primer sequence

<400>  30
acagcaggtc agtcaagcag ta                                               22

<210>  31
<211>  22
<212>  DNA
<213>  Artificial

<220>
<223>  an artificially synthesized primer sequence

<400>  31
agcagtagca gcagttcgat aa                                               22

<210>  32
<211>  20
<212>  DNA
<213>  Artificial

<220>
<223>  an artificially synthesized primer sequence

<400>  32
aggtgtgcac gccgctggtc                                                  20

<210>  33
<211>  20
<212>  DNA
<213>  Artificial

<220>
<223>  an artificially synthesized primer sequence

<400>  33
cgcctgagtt ccacgacacc                                                    20


<210>  34
<211>  20
<212>  DNA
<213>  Artificial

<220>
<223>  an artificially synthesized primer sequence

<400>  34
tcggggaagt agtccttgac                                                    20


<210>  35
<211>  19
<212>  DNA
<213>  Artificial

<220>
<223>  an artificially synthesized primer sequence

<400>  35
acacyagtgt ggccttgtt                                                     19


<210>  36
<211>  19
<212>  DNA
<213>  Artificial

<220>
<223>  an artificially synthesized primer sequence

<400>  36
gcttgragct cctcagagg                                                     19


<210>  37
<211>  19
<212>  DNA
<213>  Artificial

<220>
<223>  an artificially synthesized primer sequence

<400>  37
gggygggaac agagtgacc                                                     19


<210>  38
<211>  20
<212>  DNA
<213>  Artificial

<220>

<223>  an artificially synthesized primer sequence

<400>  38
gttattcagc aggcacacaa                                                    20


<210>  39
<211>  20
<212>  DNA
<213>  Artificial

<220>
<223>  an artificially synthesized primer sequence

<400>  39
gaggcagttc cagatttcaa                                                    20


<210>  40
<211>  21
<212>  DNA
<213>  Artificial

<220>
<223>  an artificially synthesized primer sequence

<400>  40
gggaagatga agacagatgg t                                                  21

## Description

### Technical Field

The present invention relates to devices for detecting or quantifying influenza viruses, kits for detecting or quantifying influenza viruses, methods for detecting or quantifying influenza viruses, human anti-influenza A virus nucleoprotein antibodies, and human anti-influenza B virus nucleoprotein antibodies.

### Background Art

Immunological measurement methods which use antigen-antibody reactions are widely used to measure an object of measurement in a sample, and numerous measurement methods have been developed, such as enzyme-linked immunosorbent assay (ELISA), radioimmunoassay (RIA), chemiluminescent enzyme immunoassay, nephelometric immunoassay, and measurement methods using surface plasmon resonance (SPR).

Recently, immunochromatography has received attention from the viewpoint of simplicity and speed, and many products that are based on this technique are already on the market. Immunochromatography is classified into flow-through type and lateral flow type based on the principle of measurement; however, the lateral flow type immunochromatography has become the mainstream in recent years. As lateral flow type immunochromatography, devices have been reported which comprise a region in which a labeled antibody is retained on a support in a manner to allow migration, the labeled antibody being an antibody retained on a support in a manner to allow migration, which binds to an object of measurement and to which a label is bound; and a region in which an antibody which binds to the object of measurement is immobilized.

Influenza has periodically repeated worldwide epidemic from old times and has each time caused many deaths; however, the causal virus was detected in 1931 and a way for elucidation of the causes was found. The pathogenic virus for influenza is classified into three types, type A, type B and type C, according to the antigenicity of the soluble nucleoprotein (hereinafter abbreviated as NP) found inside the virus. Moreover, it is classified into subtypes according to the antigenicity of two envelope glycoproteins, hemagglutinin (abbreviated as HA) and neuraminidase (abbreviated as NA), present on the viral surface.

From 1972, vaccines inactivated with formalin after ether treatment have been used for the prevention of influenza. In recent years, besides the vaccines used for prevention, anti-influenza agents have been found and are being widely used as therapeutic agents. Such therapeutic agents are, for example, amantadine hydrochloride applied to influenza B viruses, and zanavir and oseltamivir phosphate applied to influenza A viruses and type A.

When selecting these therapeutic agents, it is important to detect the influenza virus in the sample and determine whether the infection is due to an influenza virus and the viral type is type A or type B. Further, influenza type A viruses have a stronger infectivity and cause more serious symptoms as compared with influenza type B viruses; therefore, determination of the type of the infecting virus is important for early treatment.

Conventionally, detection of an influenza virus has been carried out using an anti-influenza virus antibody. Known antibodies against the influenza virus are antibodies that recognize the HA region, antibodies that recognize the NA region, antibodies that recognize the matrix protein (such as M1), antibodies that recognize the non-structural protein (such as NS1), antibodies specifically recognizing NP, and such. Moreover, antibodies described in Non-Patent Document 1 and Non-Patent Document 2 are known as human anti-influenza A virus nucleoprotein antibodies.

Recently, devices for immunochromatography for easily and rapidly detecting influenza viruses have been developed [see, Patent Documents 1 to 14) and numerous products have been distributed in the market for example, "ESPLINE Influenza A&B-N" (manufactured by Fujirebio Inc.); "QuickVue Rapid SP influ" (manufactured by DS Pharma Biomedical Co., Ltd.); "POCTEM Influenza A/B" (manufactured by Otsuka Pharmaceutical Co. Ltd. and Sysmex Corporation); "Clearview-Influenza A/B (manufactured by Sanwa Kagaku Co. Ltd); "Quick S-Influ A/B "Seiken"" (manufactured by Denka Seiken Co. Ltd); "Quick Ex-Flu "Seiken"" (manufactured by Denka Seiken Co. Ltd); "Rapid Testa FLU stick" (manufactured by Daichi Pure Chemicals Co. Ltd); "Capilia Flu A;B" (manufactured by Alfresa Pharma Corporation); and "Quick Chaser Flu A,B" (manufactured by Mizuho Medy Co. Lid.)].

### Prior Art Documents

### Patent Documents

Patent Document 1: WO 2005/007697, pamphlet
Patent Document 2: WO 2005/007698, pamphlet
Patent Document 3: Japanese Utility Model Registration No. 3088698
Patent Document 4: Japanese Patent Application *Kokai* Publication No. (JP-A) 2004-279208 (unexamined, published Japanese patent application)
Patent Document 5: JP-A (Kokai) 2004-279158
Patent Document 6: JP-A (Kokai) 2006-189317
Patent Document 7: JP-A (Kokai) 2006-194687
Patent Document 8: JP-A (Kokai) 2006-194688
Patent Document 9: JP-A (Kokai) 2007-93292
Patent Document 10: JP-A (Kokai) 2007-33293
Patent Document 11: JP-A (Kokai) 2006-153523
Patent Document 12: JP-A (Kokai) 2006-67979
Patent Document 13: JP-A (Kokai) 2000-55918
Patent Document 14: JP-A (Kokai) 2007-33293

### Non-Patent Documents

Non-Patent Document 1: Journal of General Virology, Vol. 64, p. 697-700 (1983)
Non-Patent Document 2: Nature Vol. 453, p. 667-672 (2008)

### Summary of the Invention

### [Problems to be Solved by the Invention]

However, the antibodies used in these devices are all non-human antibodies, so that the sensitivity was insufficient and the specificity to influenza virus which repeats mutations was insufficient for application. Thus, the development of devices for detecting or quantifying influenza viruses, kits for detecting or quantifying influenza viruses, methods for detecting or quantifying influenza viruses, which would lead to sufficient sensitivity and have wide specificity, as well as antibodies suitable for detecting or quantifying influenza viruses were desired.

### [Means for Solving the Problems]

The inventors conducted dedicated studies to solve these problems, and as a result, they discovered that human anti-influenza virus nucleoprotein antibodies can be used in devices for detecting or quantifying influenza viruses and in kits for detecting or quantifying influenza viruses. Furthermore, they discovered that antibodies having high affinity can be obtained from lymphocytes taken from humans inoculated with the influenza vaccine, and they thus completed the present invention. Accordingly, the present invention relates to the following [1] to [37]:
[1] a device for detecting or quantifying an influenza virus in a sample, which comprises a detection region in which a human anti-influenza virus nucleoprotein antibody is immobilized onto a support, a sample supply region, and a sample-migrating region;
[2] a device for detecting or quantifying an influenza virus in a sample, which comprises a detection region in which an anti-influenza virus nucleoprotein antibody (antibody 1) is immobilized onto a support, a sample supply region, and a sample-migrating region, wherein a labeled antibody in which a label is bound to an anti-influenza virus nucleoprotein antibody (antibody 2) is supplied from the sample supply region, and wherein at least one of antibody 1 and antibody 2 is a human anti-influenza virus nucleoprotein antibody;
[3] a device for detecting or quantifying an influenza virus in a sample, which comprises a detection region in which an anti-influenza virus nucleoprotein antibody (antibody 1) is immobilized onto a support, a labeled reagent region in which a labeled antibody in which a label is bound to an anti-influenza virus nucleoprotein antibody (antibody 2) is retained on a support in a manner to allow migration, a sample supply region, and a sample-migrating region, wherein at least one of antibody 1 and antibody 2 is a human anti-influenza virus nucleoprotein antibody;
[4] the device of [2] or [3], which further comprises at least one region selected from the group consisting of a developer solution supply region, an excess liquid absorbing region, and a sample supply confirming region;
[5] the device of any one of [1] to [4], wherein the human anti-influenza virus nucleoprotein antibody is a human anti-influenza A virus nucleoprotein antibody;
[6] the device of [5], wherein the human anti-influenza A virus nucleoprotein antibody is a human anti-influenza A virus nucleoprotein monoclonal antibody which specifically reacts with an influenza A virus nucleoprotein but does not react with an influenza B virus nucleoprotein;
[7] the device of [6], wherein the amino acid sequence of the heavy chain variable region of the human anti-influenza A virus nucleoprotein monoclonal antibody comprises the amino acid sequence of any one of (SEQ ID NOs: 8 to 13 and the amino acid sequence of the light chain variable region of the human anti-influenza A virus nucleoprotein monoclonal antibody comprises the amino acid sequence of any one of SEQ ID NOs; 22 to 27;
[8] the device of any one of [1] to [4], wherein the human anti-influenza virus nucleoprotein antibody is a human anti-influenza B virus nucleoprotein antibody;
[9] the device of [8], wherein the human anti-influenza B virus nucleoprotein antibody is a human anti-influenza B virus nucleoprotein monoclonal antibody which specifically reacts with an influenza B virus nucleoprotein but does not react with an influenza A virus nucleoprotein;
[10] the device of [9], wherein the amino acid sequence of the heavy chain variable region of the human anti-influenza B virus nucleoprotein monoclonal antibody comprises the amino acid sequence of SEQ ID NO: 14 and the amino acid sequence of the light chain variable region of the human anti-influenza B virus nucleoprotein monoclonal antibody comprises the amino acid sequence of SEQ ID NO: 28;
[11] a kit for detecting or quantifying an influenza virus, which comprises a solid phase in which a human anti-influenza virus nucleoprotein antibody is fixed onto a carrier;
[12] a kit for detecting or quantifying an influenza virus, which comprises a solid phase in which an anti-influenza virus nucleoprotein antibody (antibody 1) is fixed onto a carrier and a reagent comprising an anti-influenza virus nucleoprotein antibody (antibody 2), and wherein at least one of antibody 1 and antibody 2 is a human anti-influenza virus nucleoprotein antibody;
[13] a kit for detecting or quantifying an influenza virus, which comprises a solid phase in which an anti-influenza virus nucleoprotein antibody (antibody 1) is fixed onto a carrier and a reagent comprising a labeled antibody in which a label is bound to an anti-influenza virus nucleoprotein antibody (antibody 2), and wherein at least one of antibody 1 and antibody 2 is a human anti-influenza virus nucleoprotein antibody;
[14] a kit for detecting or quantifying an influenza virus, which comprises a solid phase in which a human anti-influenza virus nucleoprotein antibody is fixed onto a carrier and a reagent comprising a labeled antigen analog in which a label is bound to an influenza virus nucleoprotein antigen analog;
[15] a kit for detecting or quantifying an influenza virus, which comprises a solid phase in which an influenza virus nucleoprotein antigen analog is fixed onto a carrier and a reagent comprising a labeled antibody in which a label is bound to a human anti-influenza virus nucleoprotein antibody;
[16] the kit of any one of [11] to [15], wherein the human anti-influenza virus nucleoprotein antibody is a human anti-influenza A virus nucleoprotein antibody;
[17] the kit of [16], wherein the human anti-influenza A virus nucleoprotein antibody is a human anti-influenza A virus nucleoprotein monoclonal antibody which specifically reacts with an influenza A virus nucleoprotein but does not react with an influenza B virus nucleoprotein;
[18] the kit of [17], wherein the amino acid sequence of the heavy chain variable region of the human anti-influenza A virus nucleoprotein monoclonal antibody comprises the amino acid sequence of any one of SEQ ID NOs: 8 to 13 and the amino acid sequence of the light chain variable region of the human anti-influenza A virus nucleoprotein monoclonal antibody comprises the amino acid sequence of any one of SEQ ID NOs: 22 to 27;
[19] the kit of any one of [11] to [15], wherein the human anti-influenza virus nucleoprotein antibody is a human anti-influenza B virus nucleoprotein antibody;
[20] the kit of [19], wherein the human anti-influenza B virus nucleoprotein antibody is a human anti-influenza B virus nucleoprotein monoclonal antibody which reacts specifically with an influenza B virus nucleoprotein but does not react with an influenza A virus nucleoprotein;
[21] the kit of [20], wherein the amino acid sequence of the heavy chain variable region of the human anti-influenza B virus nucleoprotein monoclonal antibody comprises the amino acid sequence of SEQ ID NO: 14 and the amino acid sequence of the light chain variable region of the human anti-influenza B virus nucleoprotein monoclonal antibody comprises the amino acid sequence of SEQ ID NO: 28;
[22] a method for detecting or quantifying an influenza virus, comprising the steps of:
   (1) reacting a sample with a human anti-influenza virus nucleoprotein antibody fixed onto a carrier; and
   (2) measuring a physical change produced in step (1);
[23] a method for detecting or quantifying an influenza virus, comprising the steps of:
   (1) reacting a sample with an anti-influenza virus nucleoprotein antibody (antibody 1) fixed onto a carrier to form an antibody 1/influenza virus nucleoprotein complex on the carrier;
   (2) reacting the complex formed on the carrier in step (1) with an anti-influenza virus nucleoprotein antibody (antibody 2); and
   (3) measuring a physical change produced in step (2);
   wherein at least one of antibody 1 and antibody 2 is a human anti-influenza virus nucleoprotein antibody;
[24] the method of [22] or [23], wherein the physical change is turbidity change or mass change;
[25] a method for detecting or quantifying an influenza virus, comprising the steps of:
   (1) reacting a sample with an anti-influenza virus nucleoprotein antibody (antibody 1) fixed onto a carrier to form an antibody 1/inftuenza virus nucleoprotein complex on the carrier;
   (2) redacting the complex formed on the carrier in step (1) with a labeled antibody (labeled antibody 2) in which a label is bound to an anti-influenza virus nucleoprotein antibody (antibody 2) to form an antibody 1/influenza virus nucleoprotein/labeled antibody 2 complex on the carrier;
   (3) washing the carrier after step (2) to remove substances not bound to the carrier; and
   (4) measuring the label in the antibody 1/influenza virus nucleoprotein/labeled antibody 2 complex on the carrier after step (3);
   wherein at least one of antibody 1 and antibody 2 is a human anti-influenza virus nucleoprotein antibody;
[26] a method for detecting or quantifying an influenza virus, comprising the steps of:
   (1) reacting a sample with a labeled antibody (labeled antibody 2) in which a label is bound to an anti-influenza virus nucleoprotein antibody (antibody 2) to form a labeled antibody 2/influenza virus nucleoprotein complex;
   (2) reacting the complex formed in step (1) with an anti-influenza virus nucleoprotein antibody (antibody 1) fixed onto a carrier to form an antibody 1/influenza virus nucleoprotein/labeled antibody 2 complex on the carrier;
   (3) washing the carrier after step (2) to remove substances not bound to the carrier; and
   (4) measuring the label in the antibody 1/influenza virus nucleoprotein/labeled antibody 2 complex on the carrier after step (3);
   wherein at least one of antibody 1 and antibody 2 is a human anti-influenza virus nucleoprotein antibody;
[27] a method for detecting or quantifying an influenza virus, comprising the steps of:
   (1) reacting a sample with a human anti-influenza virus nucleoprotein antibody fixed onto a carrier in the co-presence of a labeled antigen analog in which a label is bound to an influenza virus nucleoprotein antigen analog, to form a human anti-influenza virus nucleoprotein antibody/influenza virus nucleoprotein complex and a human anti-influenza virus nucleoprotein antibody/labeled antigen analog complex on the carrier;
   (2) washing the carrier after step (1) to remove substances not bound to the carrier; and
   (3) measuring the label in the complexes on the carrier after step (2);
[28] a method for detecting or quantifying an influenza virus, comprising the steps of:
   (1) reacting a sample with an influenza virus nucleoprotein antigen analog fixed onto a carrier in the co-presence of a labeled antibody in which a label is bound to a human anti-influenza virus nucleoprotein antibody, to form an influenza virus nucleoprotein antigen analog/labeled antibody complex on the carrier;
   (2) washing the carrier after step (1) to remove substances not bound to the carrier; and
   (3) measuring the label in the complex on the carrier after step (2);
[29] the method of any one of [22] to [28], wherein the human anti-influenza virus nucleoprotein antibody is a human anti-influenza A virus nucleoprotein antibody;
[30] the method of [29], wherein the human anti-influenza A virus nucleoprotein antibody is a human anti-influenza A virus nucleoprotein monoclonal antibody which specifically reacts with an influenza A virus nucleoprotein but does not react with an influenza B virus nucleoprotein;
[31] the method of [30], wherein the amino acid sequence of the heavy chain variable region of the human anti-influenza A virus nucleoprotein monoclonal antibody comprises the amino acid sequence of any one of SEQ ID NOs: 8 to 13 and the amino acid sequence of the light chain variable region of the human anti-influenza A virus nucleoprotein monoclonal antibody comprises the amino acid sequence of any one of SEQ ID NOs: 22 to 27;
[32] the method of any one of [22] to [28], wherein the human anti-influenza virus nucleoprotein antibody is a human anti-influenza B virus nucleoprotein antibody;
[33] the method of [32], wherein the human anti-influenza B virus nucleoprotein antibody is a human anti-influenza B virus nucleoprotein monoclonal antibody which specifically reacts with an influenza B virus nucleoprotein but does not react with an influenza A virus nucleoprotein;
[34] the method of [33], wherein the amino acid sequence of the heavy chain variable region of the human anti-influenza B virus nucleoprotein monoclonal antibody comprises the amino acid sequence of SEQ ID NO: 14 and the amino acid sequence of the light chain variable region of the human anti-influenza B virus nucleoprotein monoclonal antibody comprises the amino acid sequence of SEQ ID NO: 28;
[35] a human anti-influenza A virus nucleoprotein monoclonal antibody which specifically reacts with an influenza A virus nucleoprotein but does not react with an influenza B virus nucleoprotein, wherein the amino acid sequence of the heavy chain variable region comprises the amino acid sequence of any one of SEQ ID NOs: 8 to 13 and the amino acid sequence of the light chain variable region comprises the amino acid sequence of any one of SEQ ID NOs: 22 to 27;
[36] a human anti-influenza B virus nucleoprotein monoclonal antibody which specifically reacts with an influenza B virus nucleoprotein but does not react with an influenza A virus nucleoprotein; and
[37] a human anti-influenza B virus nucleoprotein monoclonal antibody which specifically reacts with an influenza B virus nucleoprotein but does not react with an influenza A virus nucleoprotein, wherein the amino acid sequence of the heavy chain variable region comprises the amino acid sequence of SEQ ID NO: 14 and the amino acid sequence of the light chain variable region comprises the amino acid sequence of SEQ ID NO: 28.

### Effects of the Invention

The present invention provides devices for detecting or quantifying influenza viruses, kits for detecting or quantifying influenza viruses, and methods for detecting or quantifying influenza viruses, which are highly sensitive and have wide specificity, as well as antibodies that are suitable for detecting or quantifying influenza viruses.

### Brief Description of the Drawings

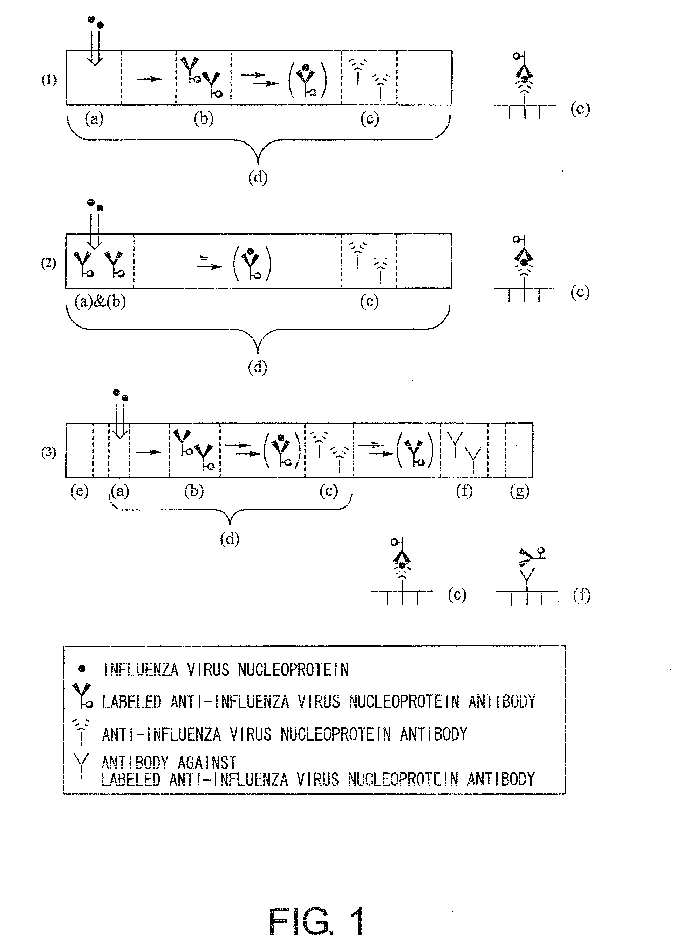

Fig. 1 shows the modes of reaction (immunochromatography reaction) of the devices of the present invention. (a) sample supply region; (b) labeled reagent region; (c) detection region; (d) sample-migrating region; (e) developer solution supply region; (f) sample supply confirming region; and (g) excess liquid absorbing region. (1) shows the mode of reaction of a device of the present invention comprising a sample supply region, a labeled reagent region, a sample-migrating region, and a detection region. (2) shows the mode of reaction of a device of the present invention in which the sample supply region of (1) functions as a labeled reagent region as well. (3) shows the mode of reaction of a device of the present invention in which (1) further comprises a developer solution supply region, a sample supply confirming region, and an excess liquid absorbing region.

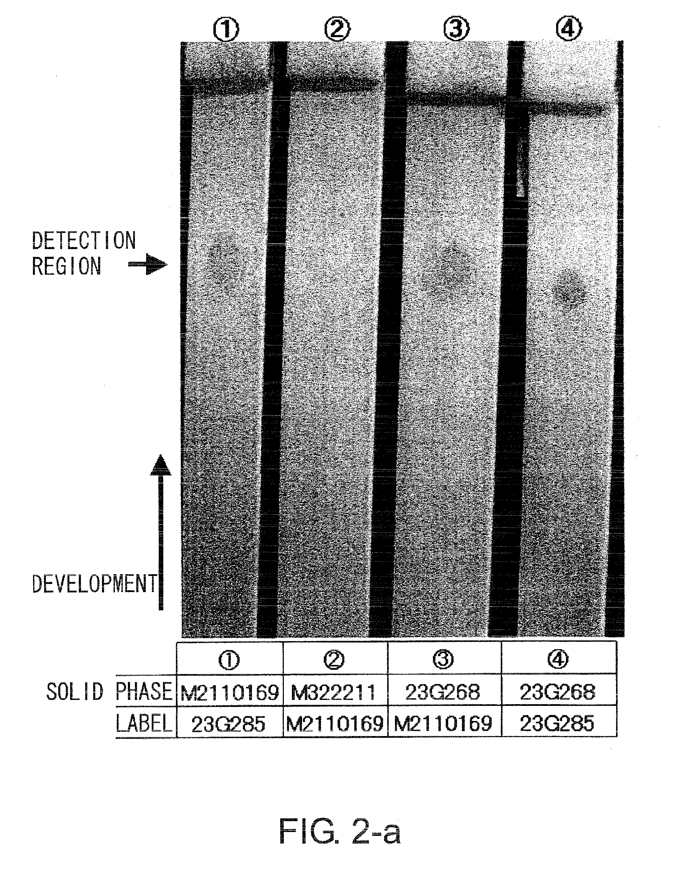

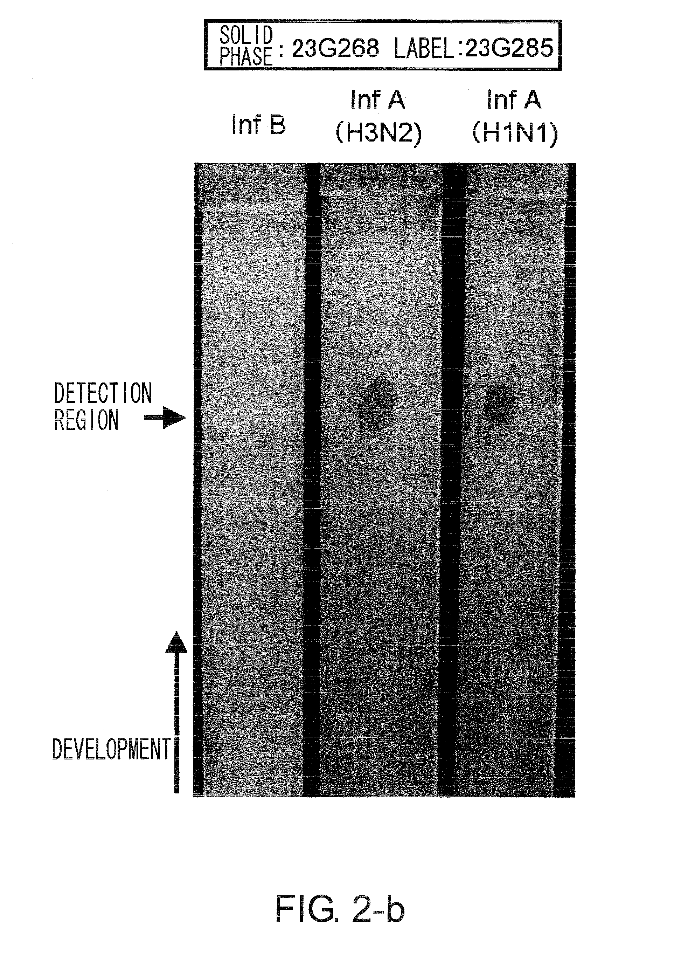

Fig. 2-a shows immunochromatograms of the devices of the present invention which use a human anti-influenza A virus nucleoprotein antibody (devices of Example 1). Fig. 2-a shows immunochromatograms of devices which use various combinations of antibodies used in the detection region (indicated as "solid phase" in the figure) and antibodies used for labeling (indicated as "label" in the figure).
Fig. 2-b shows immunochromatograms of the devices of the present invention which use a human anti-influenza A virus nucleoprotein antibody (devices of Example 1). Fig. 2-b shows immunochromatograms of devices which use 23G268 as antibody used in the detection region (indicated as "solid phase" in the figure) and 23G285 as antibody used for labeling (indicated as "label" in the figure).
Fig. 3 shows immunochromatograms of the devices of the present invention which use a human anti-influenza B virus nucleoprotein antibody (devices of Example 1).
Fig. 4-a shows an antibody γ chain expression vector and antibody λ chain expression vector used in the production of the human anti-influenza virus nucleoprotein antibodies of the present invention. Fig. 4-a shows a vector for expressing the antibody heavy chain.

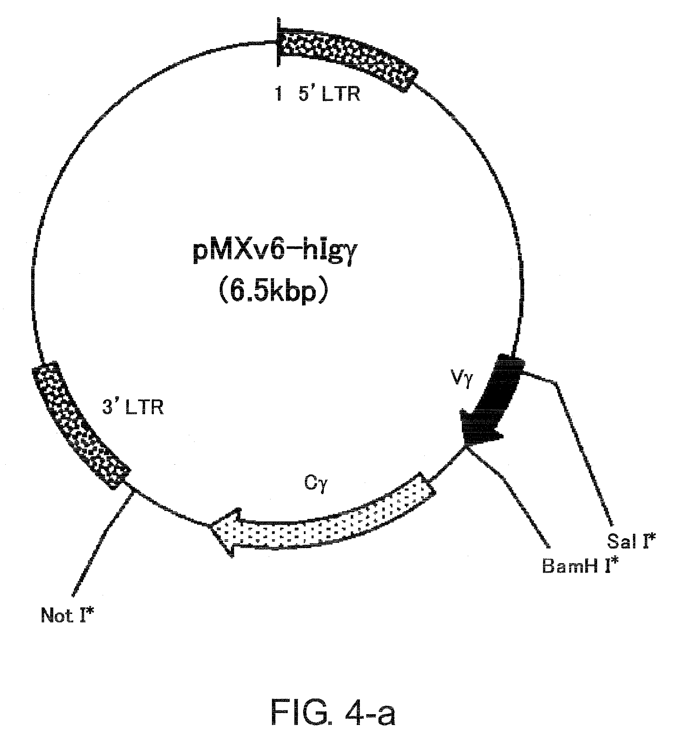

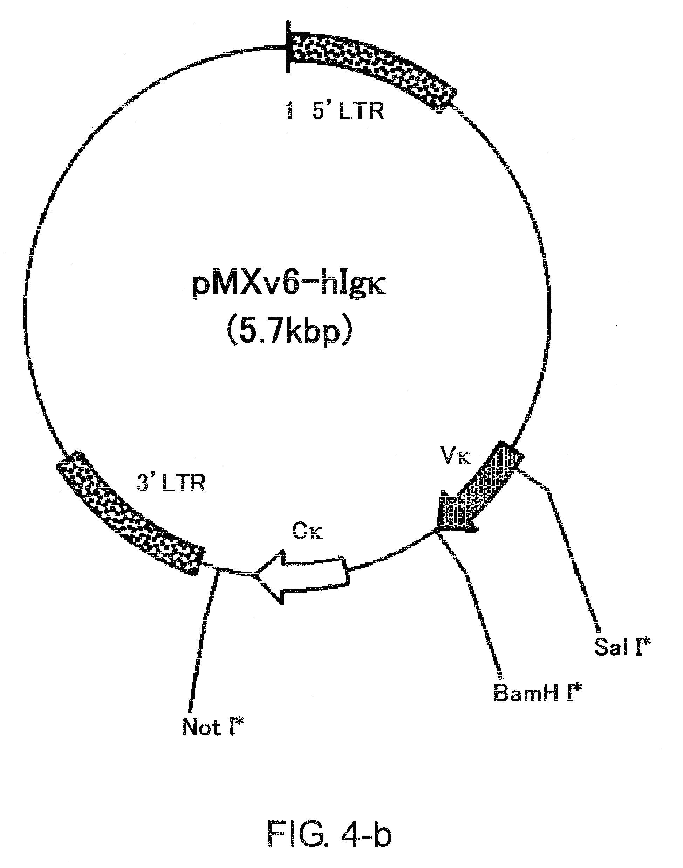

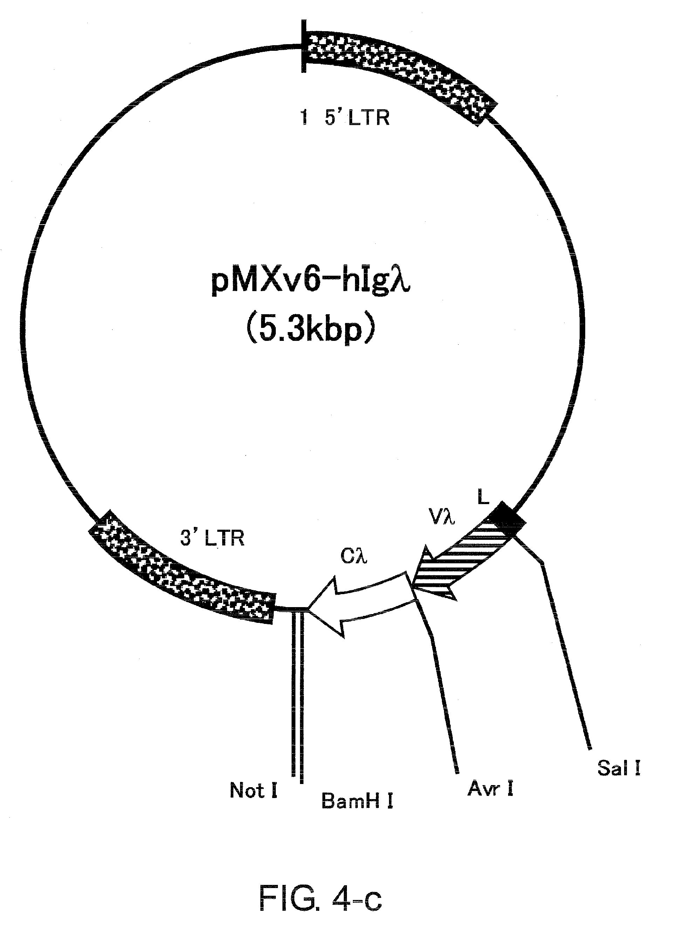

Fig. 4-b shows an antibody γ chain expression vector and antibody λ chain expression vector used in the production of the human anti-influenza virus nucleoprotein antibodies of the present invention. Fig. 4-b shows vector 1 for expressing an antibody light chain.
Fig. 4-c shows an antibody γ chain expression vector and antibody λ chain expression vector used in the production of the human anti-influenza virus nucleoprotein antibodies of the present invention. Fig. 4-c shows vector 2 for expressing an antibody light chain.

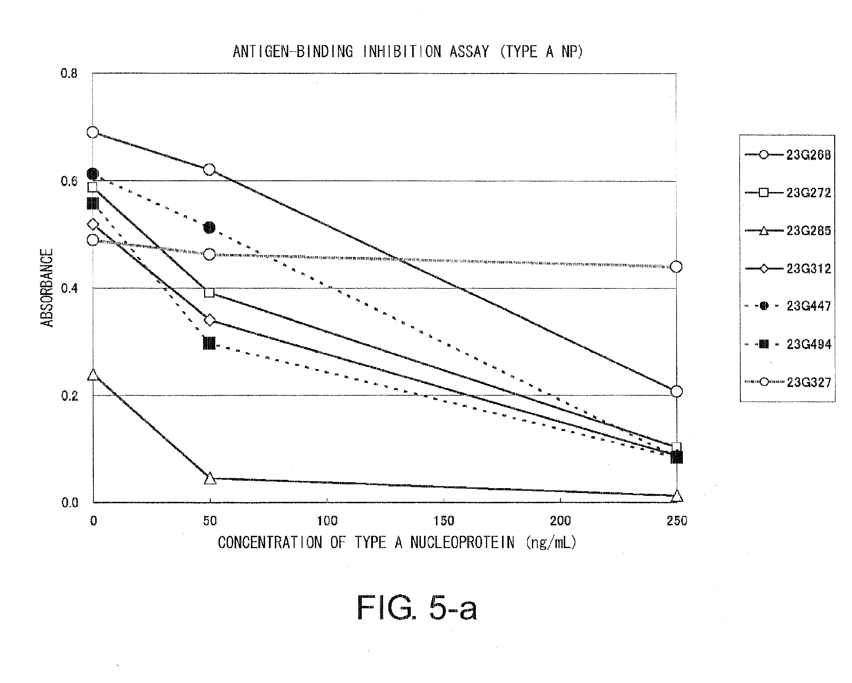

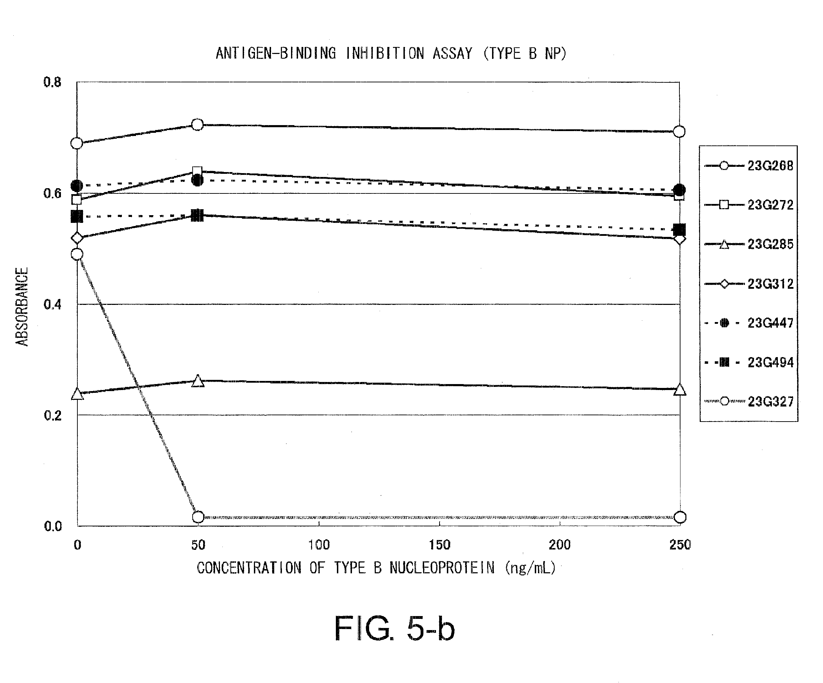

Fig. 5-a shows the result of a competitive inhibition assay (specific binding assay) of the human anti-influenza A virus nucleoprotein antibodies of the present invention to the influenza virus nucleoprotein antigen.
Fig. 5-b shows the result of a competitive inhibition assay (specific binding assay) of the human anti-influenza B virus nucleoprotein antibody of the present invention to the influenza virus nucleoprotein antigen.

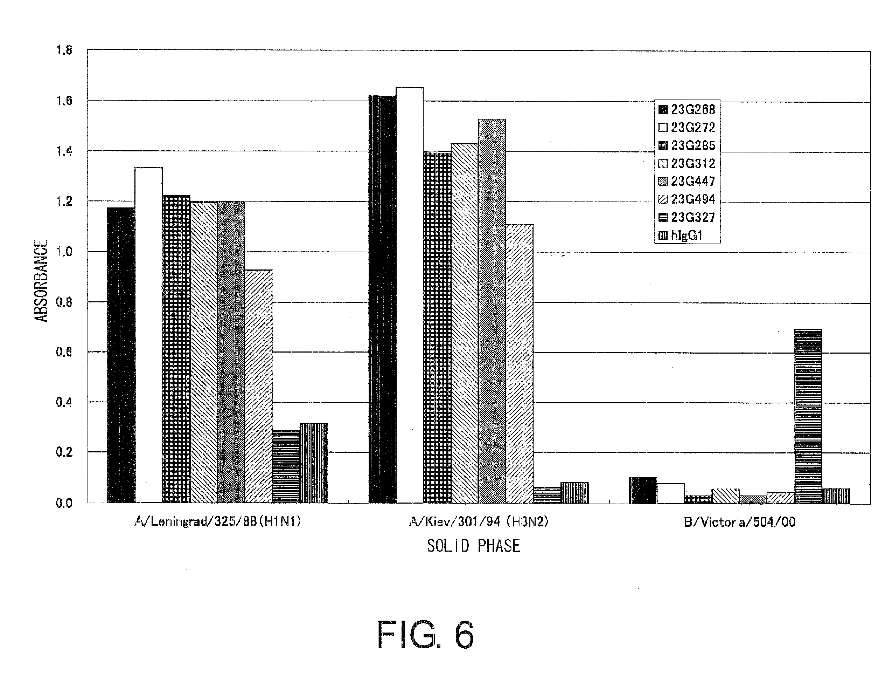

Fig. 6 shows the specificity of the human anti-influenza virus nucleoprotein antibodies of the present invention.

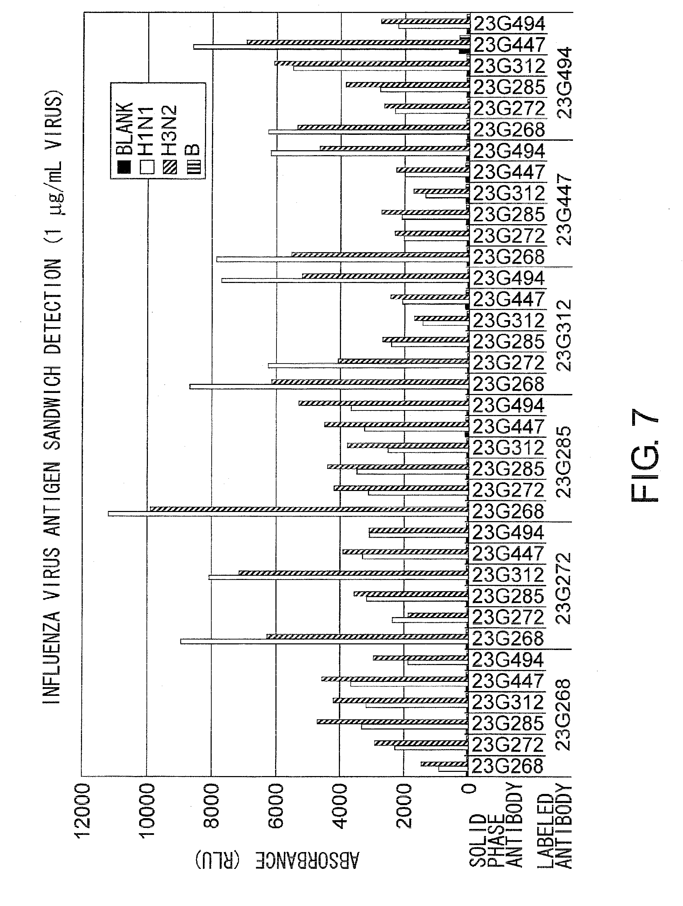

Fig. 7 shows the relationships between the combinations of the human anti-influenza A virus nucleoprotein antibodies of the present invention and sensitivity as well as specificity.

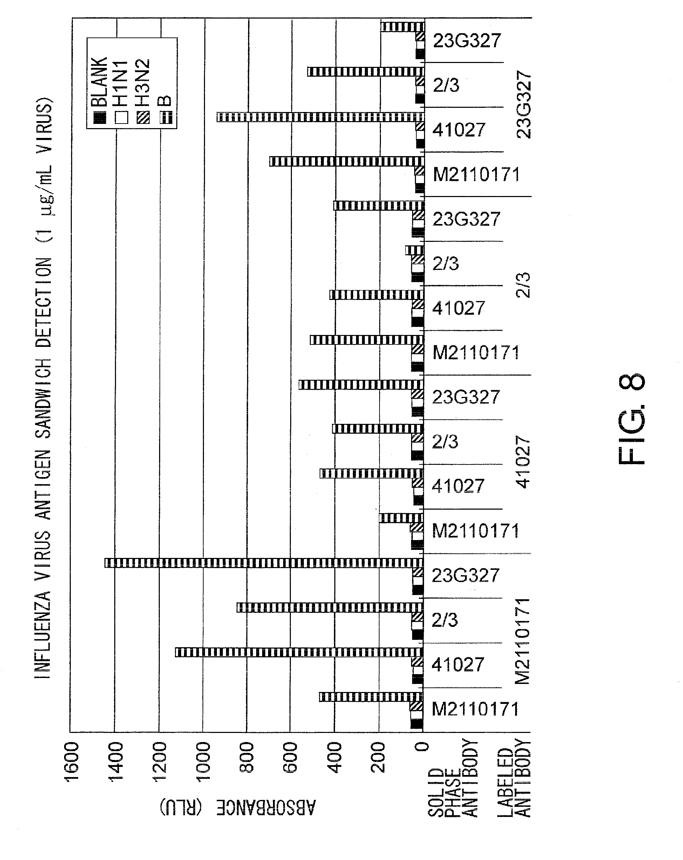

Fig. 8 shows the relationships between the combinations of the human anti-influenza B virus nucleoprotein antibody of the present invention with commercially available mouse anti-influenza B virus antibodies and sensitivity as well as specificity.

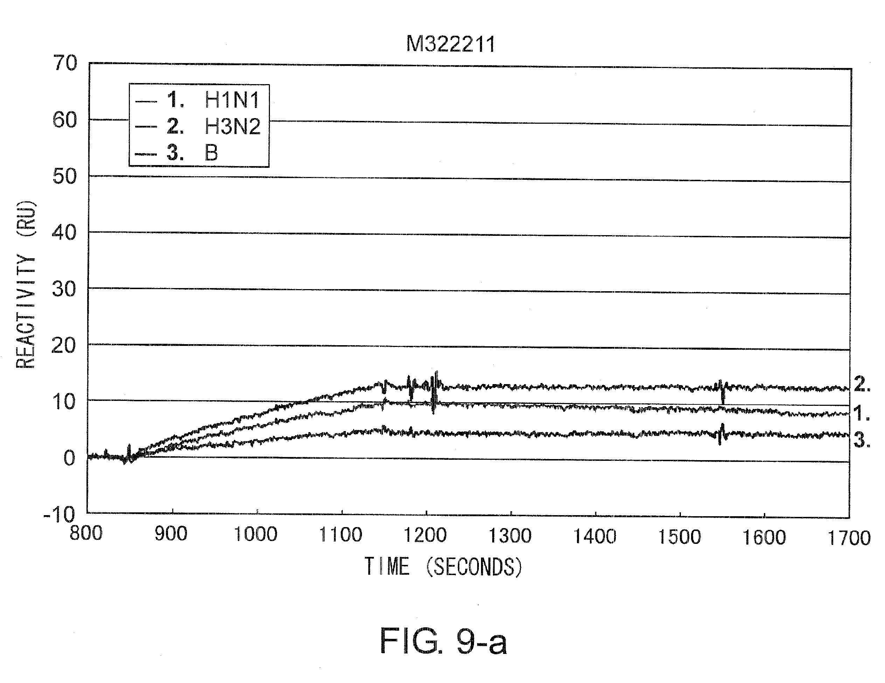

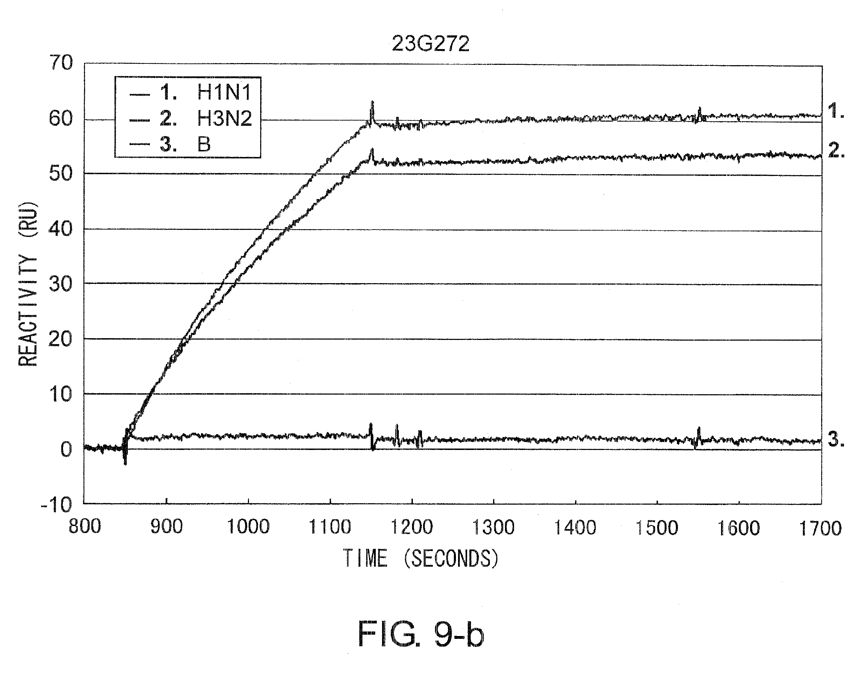

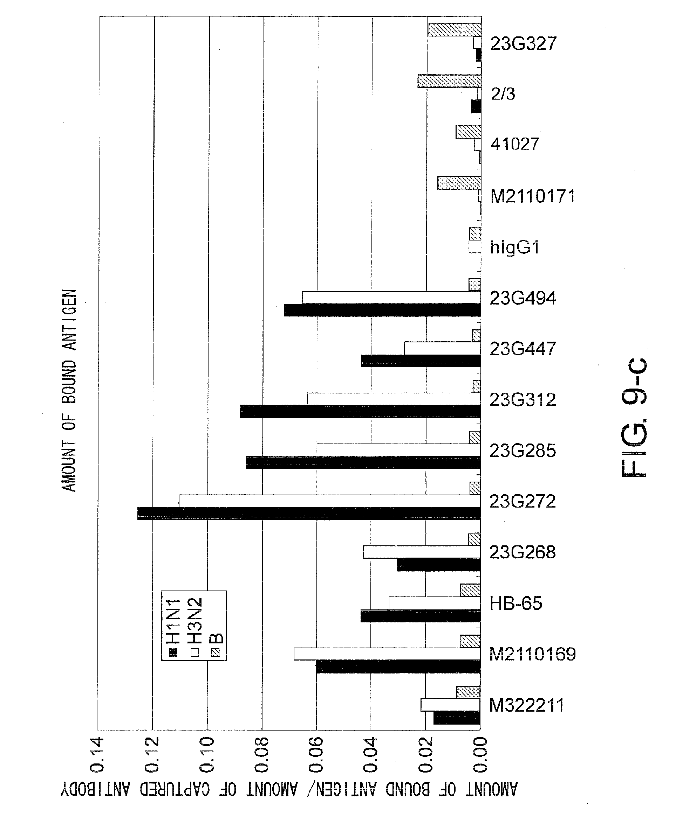

Fig. 9-a is a sensorgram showing the binding ability of the commercially available mouse anti-influenza A virus nucleoprotein antibody M322211 to influenza viruses.
Fig. 9-b is a sensorgram showing the binding ability of the human anti-influenza A virus nucleoprotein antibody 23G272 of the present invention to influenza viruses.
Fig. 9-c shows the amount of bound influenza viruses for each of the antibodies of the present invention.

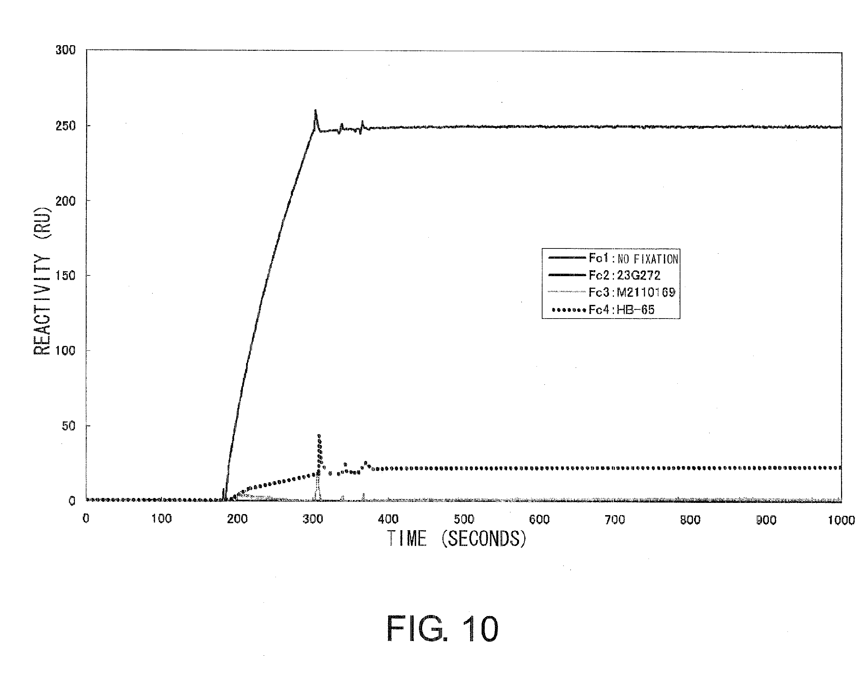

Fig. 10 shows the antigen-binding ability for each of the anti-influenza A nucleoprotein antibodies of the present invention.

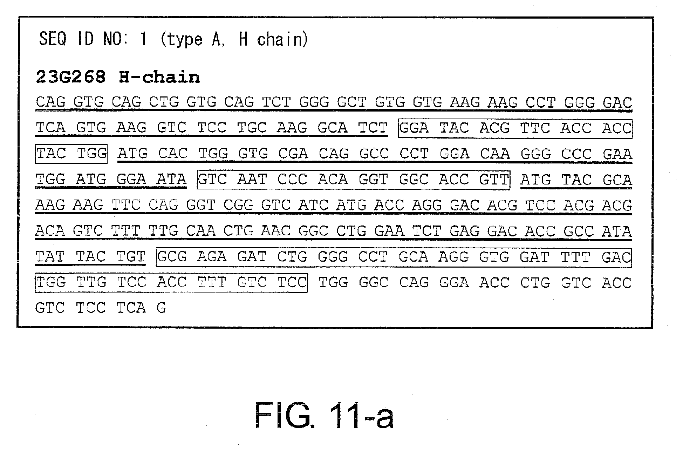

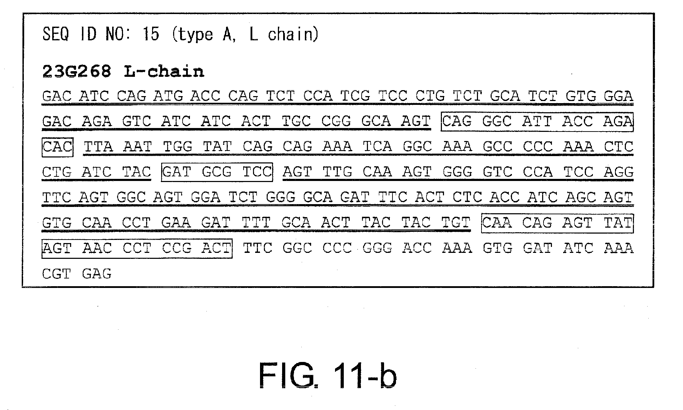

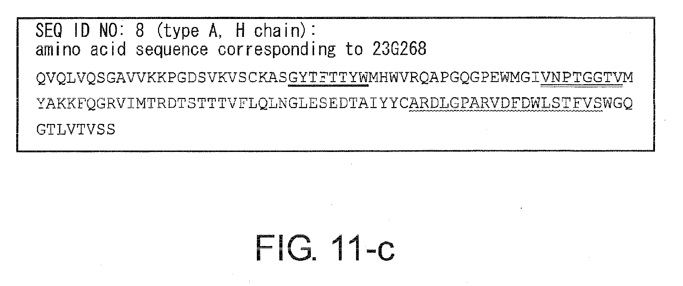

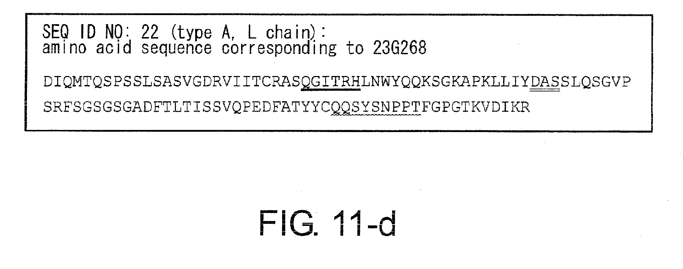

Fig. 11-a shows DNA encoding the H chain of the human anti-influenza A virus protein antibody 23G268 (SEQ ID NO: 1). The underlines indicate the DNA sequences corresponding to frameworks 1, 2, and 3 and the boxes indicate the DNA sequences corresponding to CDRs 1, 2, and 3.
Fig. 11-b shows DNA encoding the L chain of the human anti-influenza A virus protein antibody 23G268 (SEQ ID NO: 15). The underlines indicate the DNA sequences corresponding to frameworks 1, 2, and 3 and the boxes indicate the DNA sequences corresponding to CDRs 1, 2, and 3.
Fig. 11-c shows the amino acid sequence corresponding to the H chain of the human anti-influenza A virus protein antibody 23G268 (SEQ ID NO: 8). The underline, the double underline, and the wavy line respectively indicate CDRs 1, 2, and 3.
Fig. 11-d shows the amino acid sequence corresponding to the L chain of the human anti-influenza A virus protein antibody 23G268 (SEQ ID NO: 22). The underline, the double underline, and the wavy line respectively indicate CDRs 1, 2, and 3.

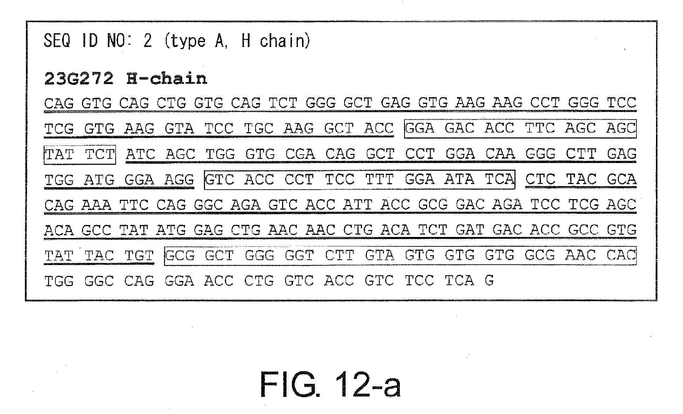

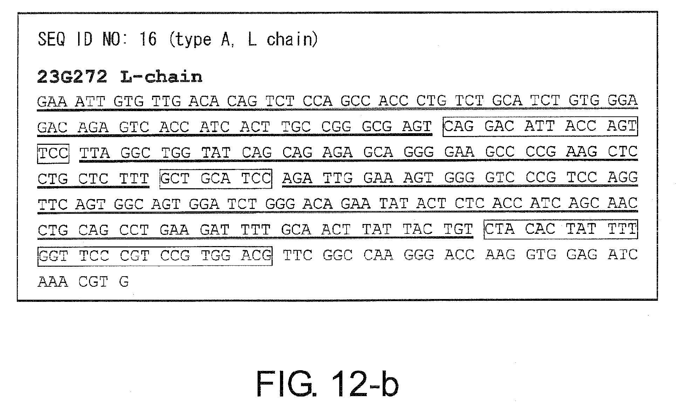

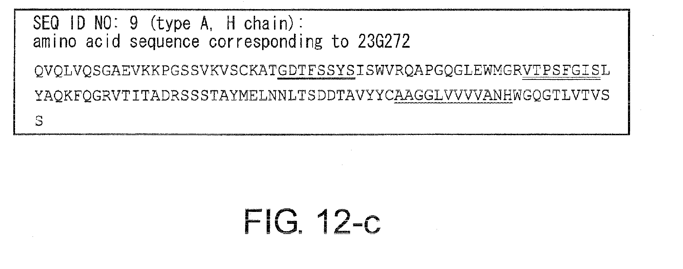

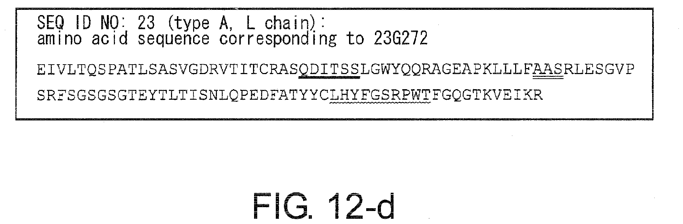

Fig. 12-a shows DNA encoding the H chain of the human anti-influenza A virus protein antibody 23G272 (SEQ ID NO: 2). The underlines indicate the DNA sequences corresponding to frameworks 1, 2, and 3 and the boxes indicate the DNA sequences corresponding to CDRs 1, 2, and 3.
Fig. 12-b shows DNA encoding the L chain of the human anti-influenza A virus protein antibody 23G272 (SEQ ID NO: 16). The underlines indicate the DNA sequences corresponding to frameworks 1, 2, and 3 and the boxes indicate the DNA sequences corresponding to CDRs 1, 2, and 3.
Fig. 12-c shows the amino acid sequence corresponding to the H chain of the human anti-influenza A virus protein antibody 23G272 (SEQ ID NO: 9). The underline, the double underline, and the wavy line respectively indicate CDRs 1, 2, and 3.
Fig. 12-d shows the amino acid sequence corresponding to the L chain of the human anti-influenza A virus protein antibody 23G272 (SEQ ID NO: 23). The underline, the double underline, and the wavy line respectively indicate CDRs 1, 2, and 3.

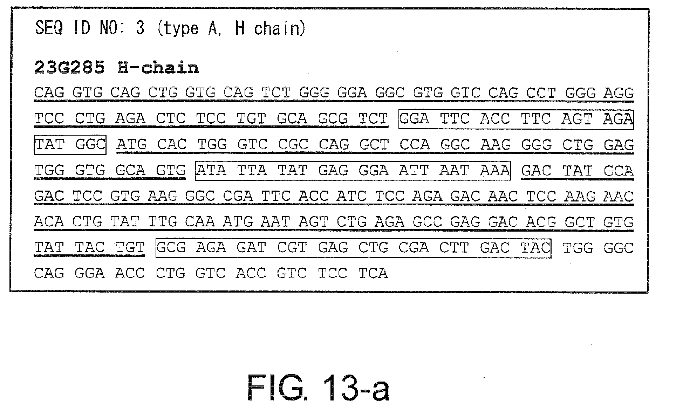

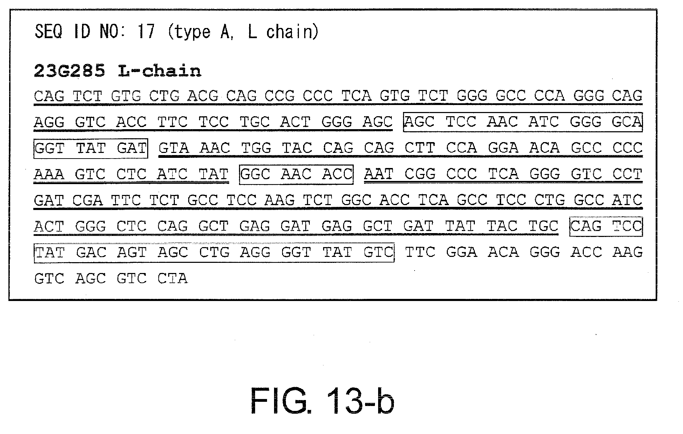

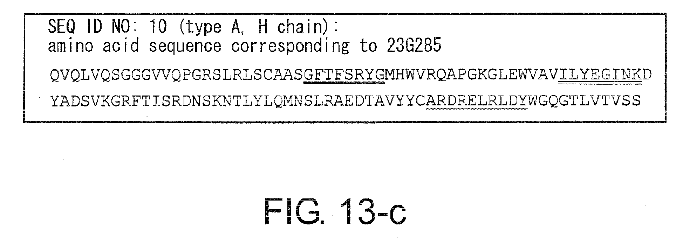

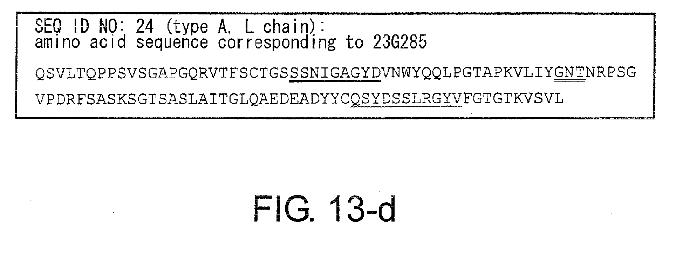

Fig. 13-a shows DNA encoding the H chain of the human anti-influenza A virus protein antibody 23G285 (SEQ ID NO: 3). The underlines indicate the DNA sequences corresponding to frameworks 1, 2, and 3 and the boxes indicate the DNA sequences corresponding to CDRs 1, 2, and 3.
Fig. 13-b shows DNA encoding the L chain of the human anti-influenza A virus protein antibody 23G285 (SEQ ID NO: 17). The underlines indicate the DNA sequences corresponding to frameworks 1, 2, and 3 and the boxes indicate the DNA sequences corresponding to CDRs 1, 2, and 3.
Fig. 13-c shows the amino acid sequence corresponding to the H chain of the human anti-infiuenza A virus protein antibody 23G285 (SEQ ID NO: 10). The underline, the double underline, and the wavy line respectively indicate CDRs 1, 2, and 3.
Fig. 13-d shows the amino acid sequence corresponding to the L chain of the human anti-influenza A virus protein antibody 23G285 (SEQ ID NO: 24). The underline, the double underline, and the wavy line respectively indicate CDRs 1, 2, and 3.

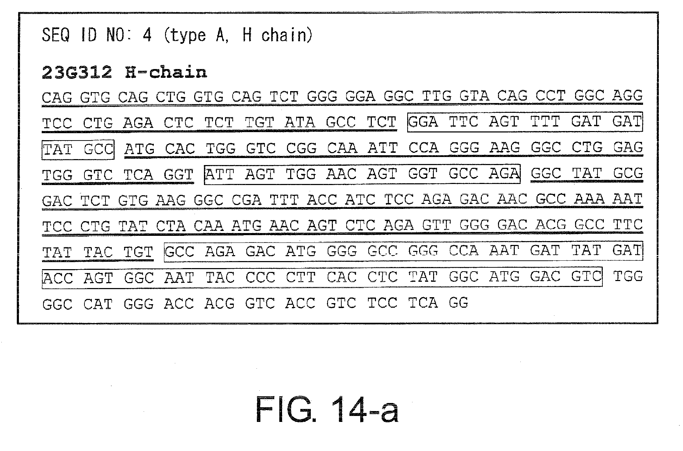

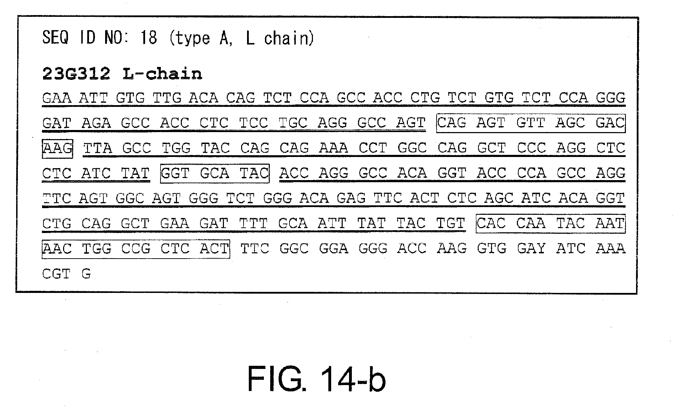

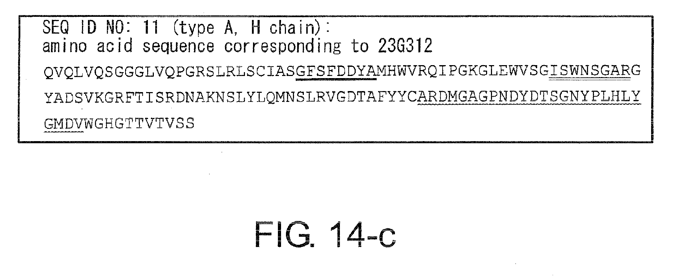

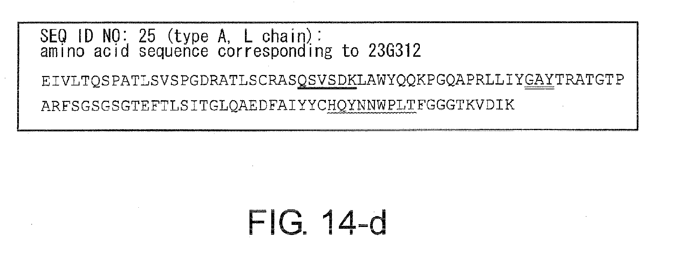

Fig. 14-a shows DNA encoding the H chain of the human anti-influenza A virus protein antibody 23G312 (SEQ ID NO: 4). The underlines indicate the DNA sequences corresponding to frameworks 1, 2, and 3 and the boxes indicate the DNA sequences corresponding to CDRs 1, 2, and 3.
Fig. 14-b shows DNA encoding the L chain of the human anti-influenza A virus protein antibody 23G312 (SEQ ID NO: 18). The underlines indicate the DNA sequences corresponding to frameworks 1, 2, and 3 and the boxes indicate the DNA sequences corresponding to CDRs 1, 2, and 3.
Fig. 14-c shows the amino acid sequence corresponding to the H chain of the human anti-influenza A virus protein antibody 23G312 (SEQ ID NO: 11). The underline, the double underline, and the wavy line respectively indicate CDRs 1, 2, and 3.
Fig. 14-d shows the amino acid sequence corresponding to the L chain of the human anti-influenza A virus protein antibody 23G312 (SEQ ID NO: 25). The underline, the double underline, and the wavy line respectively indicate CDRs 1, 2, and 3.

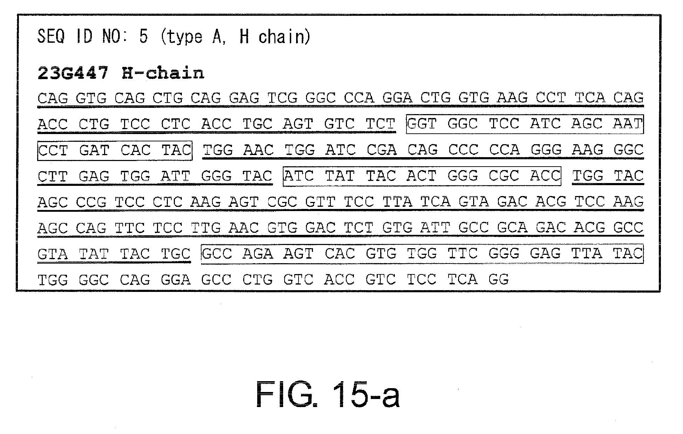

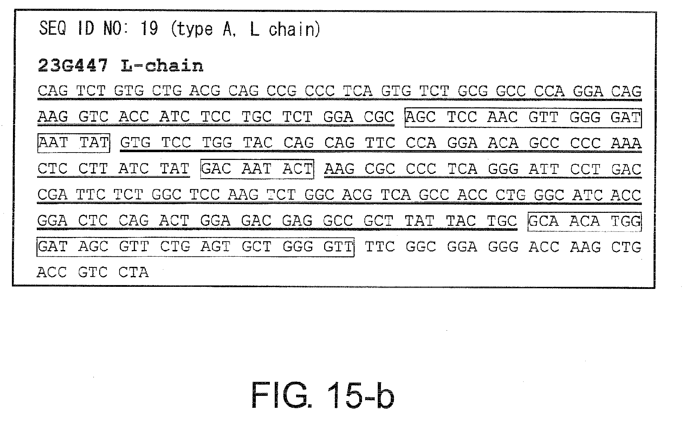

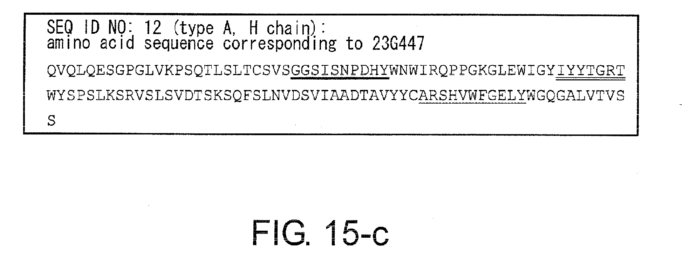

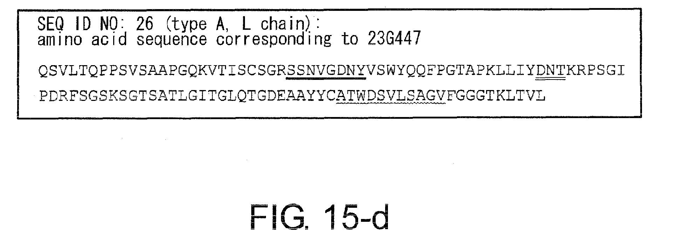

Fig. 15-a shows DNA encoding the H chain of the human anti-influenza A virus protein antibody 23G447 (SEQ ID NO: 5). The underlines indicate the DNA sequences corresponding to frameworks 1, 2, and 3 and the boxes indicate the DNA sequences corresponding to CDRs 1, 2, and 3.
Fig. 15-b shows DNA encoding the L chain of the human anti-influenza A virus protein antibody 23G447 (SEQ ID NO: 19). The underlines indicate the DNA sequences corresponding to frameworks 1, 2, and 3 and the boxes indicate the DNA sequences corresponding to CDRs 1, 2, and 3.
Fig. 15-c shows the amino acid sequence corresponding to the H chain of the human anti-influenza A virus protein antibody 23G447 (SEQ ID NO:12). The underline, the double underline, and the wavy line respectively indicate CDRs 1, 2, and 3.
Fig. 15-d shows the amino acid sequence corresponding to the L chain of the human anti-influenza A virus protein antibody 23G447 (SEQ ID NO: 26). The underline, the double underline, and the wavy line respectively indicate CDRs 1, 2, and 3.

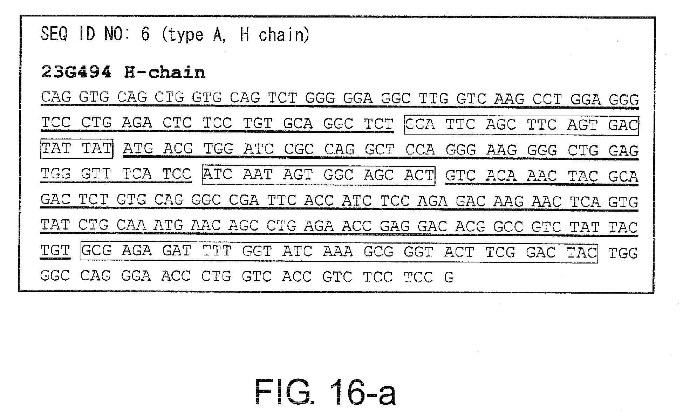

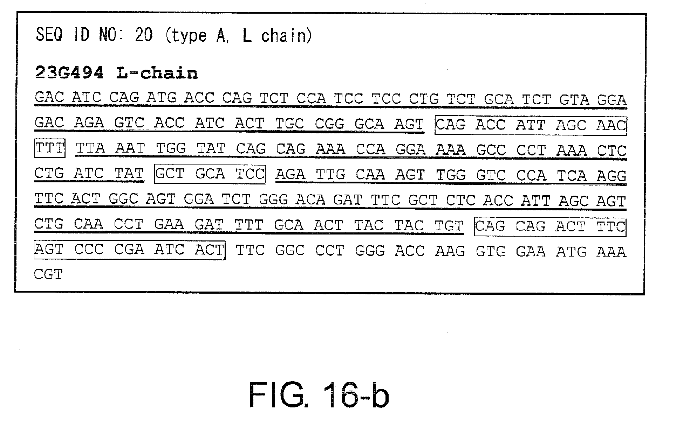

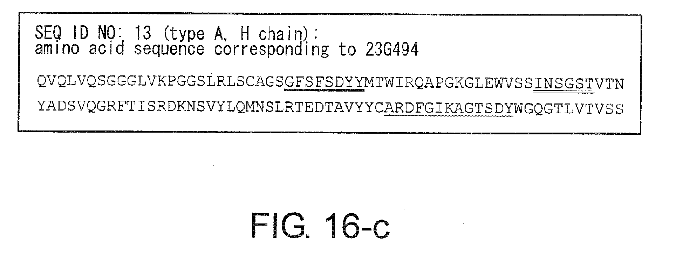

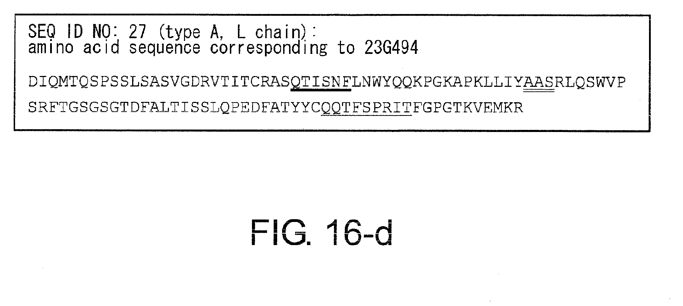

Fig. 16-a shows DNA encoding the H chain of the human anti-influenza A virus protein antibody 23G494 (SEQ ID NO: 6). The underlines indicate the DNA sequences corresponding to frameworks 1, 2, and 3 and the boxes indicate the DNA sequences corresponding to CDRs 1, 2, and 3.
Fig. 16-b shows DNA encoding the L chain of the human anti-influenza A virus protein antibody 23G494 (SEQ ID NO: 20). The underlines indicate the DNA sequences corresponding to frameworks 1, 2, and 3 and the boxes indicate the DNA sequences corresponding to CDRs 1, 2, and 3.
Fig. 16-c shows the amino acid sequence corresponding to the H chain of the human anti-influenza A virus protein antibody 23G494 (SEQ ID NO: 13). The underline, the double underline, and the wavy line respectively indicate CDRs 1, 2, and 3.
Fig. 16-d shows the amino acid sequence corresponding to the L chain of the human anti-influenza A virus protein antibody 23G494 (SEQ ID NO: 27). The underline, the double underline, and the wavy line respectively indicate CDRs 1, 2, and 3.

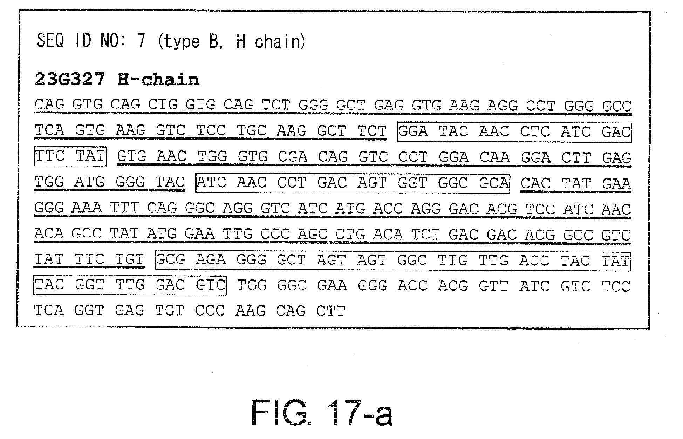

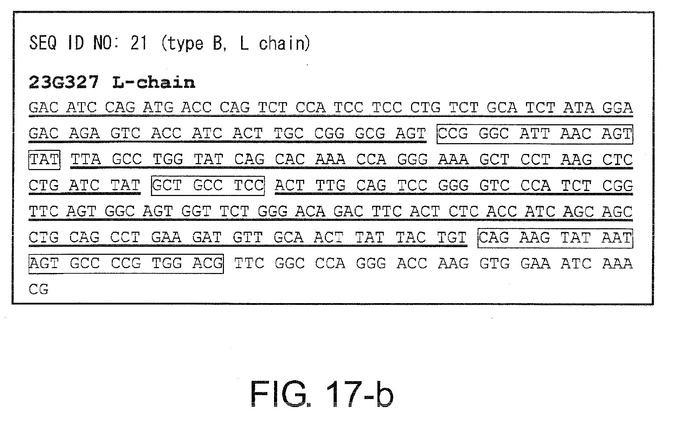

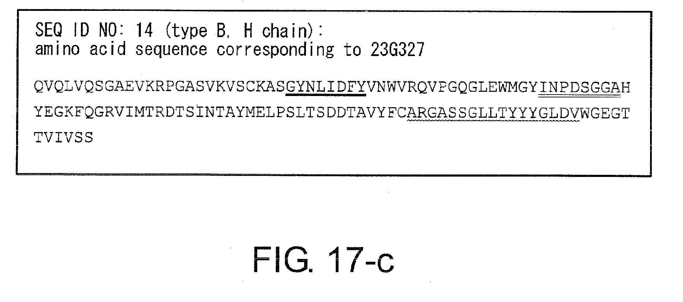

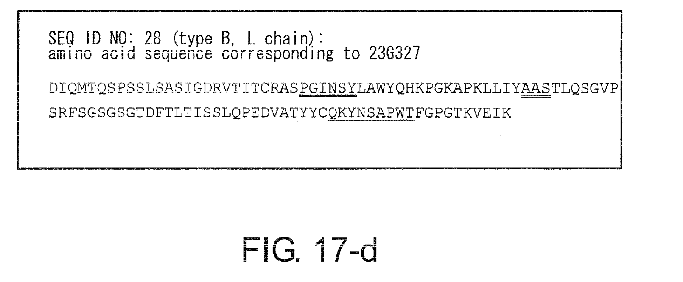

Fig. 17-a shows DNA encoding the H chain of the human anti-influenza B virus protein antibody 23G327 (SEQ ID NO: 7). The underlines indicate the DNA sequences corresponding to frameworks 1, 2, and 3 and the boxes indicate the DNA sequences corresponding to CDRs 1, 2, and 3.
Fig. 17-b shows DNA encoding the L chain of the human anti-influenza B virus protein antibody 23G327 (SEQ ID NO: 21). The underlines indicate the DNA sequences corresponding to frameworks 1, 2, and 3 and the boxes indicate the DNA sequences corresponding to CDRs 1, 2, and 3.
Fig. 17-c shows the amino acid sequence corresponding to the H chain of the human anti-influenza B virus protein antibody 23G327 (SEQ ID NO: 14). The underline, the double underline, and the wavy line respectively indicate CDRs 1, 2, and 3.
Fig. 17-d shows the amino acid sequence corresponding to the L chain of the human anti-influenza B virus protein antibody 23G327 (SEQ ID NO: 28). The underline, the double underline, and the wavy line respectively indicate CDRs 1, 2, and 3.

### Mode for Carrying Out the invention

### [Devices for detecting or quantifying influenza viruses]

The devices of the present invention for detecting or quantifying influenza viruses are devices which can be used for detecting or quantifying an influenza virus, and specifically, they are devices suitable for immunochromatography and such. The devices of the present invention may be either a flow-through type or a lateral flow type.

An embodiment of the devices of the present invention for detecting or quantifying an influenza virus includes devices which comprise a detection region, a sample supply region, and a sample-migrating region, and in which a support in the detection region has a human anti-influenza virus nucleoprotein antibody immobilized on to it. The devices are suitable for quartz crystal microbalance (QCM) sensors or carbon nanotube (CNT) sensors. After supplying a sample to the sample supply region, the sample is developed through the sample-migrating region, a reaction between the antibody and the influenza virus nucleoprotein present in the sample progresses in the detection region, and following this, physical changes occur in the detection region. Examples of the physical changes include turbidity change and mass change. By measuring such physical changes, the influenza virus nucleoprotein in the sample can be detected or quantified. Since influenza virus nucleoproteins are present in influenza viruses, detection or quantification of influenza virus nucleoproteins enables detection or quantification of the influenza viruses.

Another embodiment of the devices of the present invention for detecting or quantifying influenza viruses includes devices which comprise a detection region, a sample supply region, and a sample-migrating region, wherein in the detection region, an anti-influenza virus nucleoprotein antibody (antibody 1) is immobilized onto a support constituting this region, a labeled antibody in which a label is bound to an anti-influenza virus nucleoprotein antibody (antibody 2) is supplied from the sample supply region, and at least one of antibody 1 and antibody 2 is a human anti-influenza virus nucleoprotein antibody. Although these devices do not comprise a labeled reagent region, the labeled antibody is supplied from the sample supply region together with the sample. The influenza virus nucleoprotein/labeled antibody complexes produced through the reaction between the labeled antibody and the influenza virus nucleoprotein in the sample reach the detection region by passing through the sample-migrating region. The complexes react with the immobilized antibody 1 in the detection region and produce antibody 1/influenza virus nucleoprotein/labeled antibody complexes in the detection region. By detecting the labels in the produced antibody 1/inftuenza virus nucleoprotein/labeled antibody complexes, the influenza virus can be detected or quantified.

Another embodiment of the devices of the present invention for detecting or quantifying influenza viruses includes devices which comprise a detection region, a sample supply region, a sample-migrating region, and a labeled reagent region, wherein in the detection region, an anti-influenza virus nucleoprotein antibody (antibody 1) is immobilized onto a support constituting this region, a labeled antibody in which a label is bound to an anti-influenza virus nucleoprotein antibody (antibody 2) is retained in the labeled reagent region in a manner to allow migration, and at least one of antibody 1 and antibody 2 is a human anti-influenza virus nucleoprotein antibody. In this device, the sample which is supplied to the sample supply region is developed through the sample-migrating region and reaches the labeled reagent region. In the labeled reagent region, the influenza virus nucleoprotein in the sample reacts with the labeled antibody and produces influenza virus nucleoprotein/labeled antibody complexes. The produced complexes are further developed through the sample-migrating region and reach the detection region. Reaction between these complexes and the immobilized antibody 1 takes place in the detection region, and produce antibody 1/inftuenza virus nucleoprotein/labeled antibody complexes in the detection region. Detection of the labels in the produced antibody 1/influenza virus nucleoprotein/labeled antibody complexes enables detection or quantification of the influenza virus in the sample. In this device, the sample supply region can function as the labeled reagent region as well [see Figs. 1(1) and 1(2)].

Furthermore, devices of the present invention for detecting or quantifying an influenza virus also include devices comprising a detection region having a human anti-influenza virus nucleoprotein antibody immobilized onto a support, a sample supply region, and a sample-migrating region, wherein a labeled antigen analog in which a label is bound to an influenza virus nucleoprotein antigen is supplied from the sample supply region; and devices comprising a detection region having a human anti-influenza virus nucleoprotein antibody immobilized on to a support, a labeled reagent region having a labeled antigen analog in which a label is bound to an influenza virus nucleoprotein antigen retained on a support in a manner to allow migration, a sample supply region, and a sample-migrating region.

Additionally, devices of the present invention for detecting or quantifying an influenza virus also include devices comprising a detection region having an influenza virus nucleoprotein antigen analog immobilized onto a support, a sample supply region, and a sample-migrating region, wherein a labeled antibody in which a label is bound to a human anti-influenza virus nucleoprotein antibody is supplied from the sample supply region; and devices comprising a detection region having an influenza virus nucleoprotein antigen analog immobilized onto a Support, a labeled reagent region having a labeled antibody in which a label is bound to a human anti-influenza virus nucleoprotein antibody retained on a support in a manner to allow migration, a sample supply region, and a sample-migrating region.

Samples used in the detection or quantification of influenza viruses using a device of the present invention are not particularly limited as long as they are samples collected from humans, which may contain an influenza virus, and examples include biological samples such as, nasal cavity swab, nasal cavity aspirate, pharyngeal swab, and oral rinse. In the present invention, these biological samples can be used as is, and pretreated biological samples can also be used. Pretreatment agents used for the pretreatment are not particularly limited as long as they are pretreatment agents that destroy the nucleus of influenza viruses and enable the reactions with the anti-influenza virus nucleoprotein antibodies, and examples include surfactants. Examples of surfactants include non-ionic surfactants, cationic surfactants, anionic surfactants, and zwitterionic surfactants, and non-ionic surfactants are preferred. An example of a non-ionic surfactant is a polyoxyethylene-type surfactant (for example, Nonidet P-40). Furthermore, aqueous solvents, buffers, antiseptics, salts, sugars, metal ions, proteins, or such may be included in the pretreatment agent as needed. Examples of aqueous solvents include deionized water, distilled water, and membrane-filtered water. Examples of buffers include lactate buffer, citrate buffer, acetate buffer, succinate buffer, phthalate buffer, phosphate buffer, triethanolamine buffer, diethanolamine buffer, lysine buffer, barbituric buffer, imidazole buffer, malate buffer, oxalate buffer, glycine buffer, borate buffer, carbonate buffer, and Good buffer. Examples of Good buffers include Tris [tris(hydroxymethyl)aminomethane] buffer, MES (2-morpholinoethanesulfonic acid) buffer, Bis-Tris [bis(2-hydroxyethyl)iminotris(hydroxymethyl)methane] buffer, ADA [N-(2-acetamide)iminodiacetic acid] buffer, PIPES [piperazine-N,N' -bis(2-ethanesulfonic acid)] buffer, ACES {2-[N-(2-acetamide)amino]ethanesulfonic acid} buffer, MOPSO (3-morpholino-2-hydroxypropanesulfonic acid) buffer, BES {2-[N,N-bis(2-hydroxyethyl)amino]ethanesulfonic acid} buffer, MOPS (3-morpholinopropanesulfonic acid) buffer, TES <2-{N-[tris(hydroxymethyl)methyl]amino}ethanesulfonic acid> buffer, HEPES [N-(2-hydroxyethyl)-N'-(2-sulfoethyl)piperazine] buffer, DIPSO {3-[N,N-bis(2-hydroxyethyl)amino]-2-hydroxypropanesulfonic acid} buffer, TAPSO <2-hydroxy-3-{[N-tris(hydroxymethyl)methy]amino}propanesulfonic acid> buffer, POPSO [piperazine-N,N'-bis(2-hydroxypropane"3-suIfonic acid)] buffer, HEPPSO [N-(2-hydroxyethyl)-N'-(2-hydroxy-3-sulfopropyl)piperazine] buffer, EPPS [N -(2-hydroxyethyl)- N' -(3 -sulfopropyl)piperazine] buffer, Tricine [N-tris(hydroxymethyl)methylglycine] buffer, Bicine [N,N-bis(2-hydroxyethyl)glycine] buffer, TAPS {3-[N-tris(hydroxymethyl)methyl]aminopropanesulfonic acid} buffer, CHES [2-(N-cyclohexylamino)ethanesulfonic acid] buffer, CAPSO [3-(N-cyclohexylamino)-2-hydroxypropanesulfonic acid] buffer, and CAPS [3-(N-cyclohexylamino)propanesulfonic acid].

Examples of the antiseptics include sodium azide and antibiotics. Examples of the salts include alkali metal halide salts such as sodium chloride and potassium chloride. Examples of the sugars include mannitol, sorbitol, and sucrose. Examples of the metal ions include magnesium ion, manganese ion, and zinc ion. Examples of the proteins include bovine serum albumin (BSA), casein, Block Ace^{™} (manufactured by Dainippon Pharmaceutical), and animal serum.

An example of the devices of the present invention is explained below based on Fig. 1(1).

When a sample is added to the sample supply region, the sample develops on the support by capillary action, and reaches the labeled reagent region. In this labeled reagent region, the influenza virus nucleoprotein in the sample reacts with labeled antibody 2, in which a label is bound to an anti-influenza virus nucleoprotein antibody (antibody 2), in the labeled reagent region to produce an influenza virus nucleoprotein/labeled antibody complex (complex 1). The produced complex 1 is further developed on the support by capillary action, and reaches the detection region. Complex 1 that has reached the detection region reacts with the immobilized anti-influenza virus nucleoprotein antibody (antibody 1) in this region, and an antibody 1/influenza virus nucleoprotein/labeled antibody 2 complex (complex 2) is produced, immobilized on the support. Next, by measuring the label of the produced complex 2, the influenza virus in the sample can be detected or quantified. Herein, at least one of antibody 1 and antibody 2 is a human anti-influenza virus nucleoprotein antibody. Examples of the combination of antibody 1 and antibody 2 include the combinations of human anti-influenza virus nucleoprotein antibody/non-human anti-infiuenza virus nucleoprotein antibody, non-human anti-influenza virus nucleoprotein antibody/human anti-influenza virus nucleoprotein antibody, and human anti-influenza virus nucleoprotein antibody/human anti-influenza virus nucleoprotein antibody.

Examples of influenza viruses that are the object of detection or quantification include influenza A viruses and influenza B viruses. Since influenza virus nucleoproteins are proteins existing in the nucleus of influenza viruses and are therefore one of the constituents of an influenza virus, influenza viruses in a sample can be detected or quantified by measuring the influenza virus nucleoproteins.

When detecting or quantifying an influenza A virus using a device of the present invention, an anti-influenza A virus nucleoprotein antibody (antibody A1) is used as antibody 1 and an anti-influenza A virus nucleoprotein antibody (antibody A2) is used as antibody 2. One of antibody A1 and antibody A2 is a human antibody, and more specifically, a human anti-influenza A virus nucleoprotein antibody. Examples of combinations of antibody A1 and antibody A2 include the combination of human anti-influenza A virus nucleoprotein antibody and human anti-influenza A virus nucleoprotein antibody, the combination of human anti-influenza A virus nucleoprotein antibody and non-human anti-influenza A virus nucleoprotein antibody, and the combination of non-human anti-influenza A virus nucleoprotein antibody and human anti-influenza A virus nucleoprotein (antibody. Human anti-influenza A virus nucleoprotein antibody may be a monoclonal antibody or a polyclonal antibody, and it is preferably a monoclonal antibody. In the case of monoclonal antibodies, the region in the influenza A virus nucleoprotein which is recognized by antibody A1 and the region of influenza A vitus nucleoprotein which is recognized by antibody A2 may be the same or may be different, and are preferably different. When detecting or quantifying an influenza A virus using a device of the present invention, a mobile complex (complex A1) of influenza A virus nucleoprotein/labeled antibody A2 is produced in the labeled reagent region, and an immobilized complex (complex A2) of antibody A1/infuenza A virus nucleoprotein/labeled antibody A2 is produced in the detection region.

When detecting or quantifying an influenza B virus using a device of the present invention, an anti-influenza B virus nucleoprotein antibody (antibody B1) is used as antibody 1 and an anti-influenza B virus nucleoprotein antibody (antibody B2) is used as antibody 2. One of antibody B1 and antibody B2 is a human antibody, and more specifically a human anti-influenza B virus nucleoprotein antibody. Examples of combinations of antibody B1 and antibody B2 include the combination of a human anti-influenza B virus nucleoprotein antibody and a human anti-influenza B virus nucleoprotein antibody, the combination of a human anti-influenza B virus nucleoprotein antibody and a non-human anti-influenza B virus nucleoprotein antibody, and the combination of a non-human anti-influenza B virus nucleoprotein antibody and a human anti-influenza B virus nucleoprotein antibody. Human anti-influenza B virus nucleoprotein antibody may be a monoclonal antibody or a polyclonal antibody, and it is preferably a monoclonal antibody. In the case of monoclonal antibodies, the region in the influenza B virus nucleoprotein which is recognized by antibody B1 and the region of influenza B virus nucleoprotein which is recognized by antibody B2 may be the same or may be different, and are preferably different. When detecting or quantifying an influenza B virus using a device of the present invention, a mobile complex (complex B1) of influenza B virus nucleoprotein/ labeled antibody B2 is produced in the labeled reagent region, and an immobilized complex (complex B2) of antibody B1/influenza B virus nucleoprotein/labeled antibody B2 is produced in the detection region.

Examples of human anti-influenza A virus nucleoprotein antibodies include the human anti-influenza A virus nucleoprotein antibodies described below. Examples of human anti-influenza B virus nucleoprotein antibodies include the human anti-influenza B virus nucleoprotein antibodies described below.

The devices of the present invention for detecting or quantifying influenza viruses may further comprise one or more regions selected from the group consisting of a developer solution supply region, an excess liquid absorbing region, and a sample supply confirming region. The developer solution added to the developer solution supply region is developed on the support by capillary action, and makes the sample migrate to the labeled reagent region and also makes complex 1 (complex A1; complex B1) produced in the labeled reagent region migrate to the detection region. The developer solution supply region can function as the sample supply region as well. The excess liquid excluding complex 2 (complex A2; complex B2) produced in the detection region is absorbed in the excess liquid absorbing region. Furthermore, addition of the sample can be confirmed by providing a sample supply confirming region containing anti-human IgG antibody immobilized on a support.

### - Support

The support in the devices of the present invention is not particularly limited as long as it is a material that allows a solution to develop in the sample-migrating region, and examples include glass fiber, cellulose, nylon, cross-linked dextran, various types of chromatography papers, nitrocellulose, and metals such as gold. The size of this support is not limited, and a strip having a width of 3 mm to 10 mm or so and a length of 30 mm to 100 mm or so is preferred. A support having a thickness of 100 µm to 1 mm may be used. Furthermore, the support can be used after blocking a part or all of the support, for example, with sucrose, casein, or animal serum such as bovine serum albumin (BSA) and casein to prevent non-specific adsorption of sample-derived proteins to the support at the time of measurement.

### - Detection region

An anti-influenza virus nucleoprotein antibody is immobilized in the detection region of a device of the present invention. The detection region may be formed separately from the support, and it is preferably formed on the support. The anti-influenza virus nucleoprotein antibodies immobilized in the detection region may be monoclonal antibodies or polyclonal antibodies, and they are preferably monoclonal antibodies. At least one of the anti-influenza virus nucleoprotein antibody immobilized in the detection region and the anti-influenza virus nucleoprotein antibody forming the labeled anti-influenza virus nucleoprotein antibody retained in the labeled reagent region in a manner to allow migration is a human antibody, and both antibodies are preferably monoclonal antibodies. Examples of the anti-influenza virus nucleoprotein antibody immobilized in the detection region include the below-described human anti-influenza A virus nucleoprotein antibodies, human anti-influenza B virus nucleoprotein antibodies, and fragments of these human antibodies [Fab, F(ab')₂, F(ab')].

Examples of methods for immobilizing the anti-influenza virus nucleoprotein antibodies to the detection region include methods of physically adsorbing the anti-influenza virus nucleoprotein antibody directly to the support and methods of fixing the antibody to the support by chemical bonds such as covalent bonds. Furthermore, the anti-influenza virus nucleoprotein antibody can be bound to insoluble carrier particles and these may be included in the support. Examples of insoluble carrier particles include polystyrene latex particles, magnetic particles, and glass fibers. Examples of methods for binding the anti-influenza virus nucleoprotein antibody to insoluble carrier particles include the aforementioned physical adsorption and chemical binding.

The detection region exists downstream of the labeled reagent region, the sample supply region, the sample-migrating region, and the developer solution supply region, and upstream of the excess liquid absorbing region.

### - Labeled reagent region

In the labeled reagent region in the devices of the present invention, labeled antibodies, in which a label is bound to an anti-influenza virus nucleoprotein antibody, are retained in a manner to allow migration. The labeled reagent region may be formed separately from the support, and it is preferably formed on the support. The anti-influenza virus nucleoprotein antibody which forms the labeled anti-influenza virus nucleoprotein antibody retained in the labeled reagent region in a manner to allow migration may be a monoclonal antibody or a polyclonal antibody, and it is preferably a monoclonal antibody. Examples of the anti-influenza virus nucleoprotein antibody retained in the labeled reagent region in a manner to allow migration include the below-described human anti-influenza A virus nucleoprotein antibodies, human anti-influenza B virus nucleoprotein antibodies, and fragments of these human antibodies [Fab, F(ab')₂, F(ab')]. Examples of methods for constructing the labeled reagent region include methods of spotting a labeled antibody-containing reagent to the support and methods of layering a water-absorbing pad impregnated with a labeled antibody-containing reagent onto the support. Examples of water-absorbing pads include the water-absorbing pad used in the sample supply region described below.

Examples of labels forming the labeled antibodies include enzymes such as peroxidase, alkaline phosphatase, and β-D-galactosidase, metal colloid particles such as gold colloid particles and selenium colloid particles, colored latex particles, luminescent substances, and fluorescent substances.

Examples of methods for preparing labeled antibodies include methods of linking a label and an antibody by a covalent bond and methods of linking a label and an antibody by a non-covalent bond. Specifically, examples include known methods such as the glutaraldehyde method, periodate method, maleimide method, pyridyl-disulfide method, and methods using various types of crosslinking agents [see Tanpakushitsu Kakusan Koso (Protein, Nucleic Acid and Enzyme), (31 Suppl), pp. 37-45 (1985)]. Examples of crosslinking agents that may be used include N-succinimidyl-4-maleimidobytyrate (GMBS), N-succinimidyl-6-maleimidohesanoate and N-succinimidyl-4-(N-maleimidomethyl)cyclohexane-1-carboxylate. In the methods utilizing covalent bonds, functional groups present on the antibody may be used, and in addition, for example, functional groups such as amino groups, carboxyl groups, hydroxyl groups, sulfhydryl groups can be introduced by conventional methods and then the labeled antibody can be prepared by the aforementioned method. Furthermore, examples of methods by non-covalent binding include physical adsorption methods.

### - Sample supply region

The sample supply region in the devices of the present invention is positioned upstream of the detection region, and the sample is supplied through this region. The sample supply region is formed on the support, and the region may also be formed by layering a water-absorbing pad on the support. The support and the water-absorbing pad which form the sample supply region may appropriately include salts, surfactants, and such. Examples of the salts and surfactants include the aforementioned salts and surfactants. Examples of the water-absorbing pads include, for example, glass fibers, cellulose, nonwoven fabric, and polyvinyl alcohol.

Furthermore, the sample supply region may function as the aforementioned labeled reagent region as well, and in this case, the sample can be absorbed efficiently by layering a water-absorbing pad impregnated with a labeled antibody-containing reagent onto the support. The water-absorbing pad is not particularly limited as long as it is a material that absorbs the labeled antibody and influenza virus nucleoprotein and shows low adsorption, and examples include those described above.

### - Sample-migrating region

The sample-migrating region in the devices of the present invention exists throughout the whole support, and is the region where the sample, the labeled antibodies, and the below-described developer solution migrate. The sample supplied to the sample supply region is transported through the sample-migrating region to the detection region together with the labeled antibody. Furthermore, the sample may be transported by the developer solution supplied from the below-described developer solution supply region to the detection region through the sample-migrating region.

### - Developer solution supply region

The developer solution supply region in the devices of the present invention is provided at one end of the support, and is the region from which the developer solution is supplied. By soaking with a developer solution the developer solution supply region of a device in which a sample has been supplied to the sample supply region, the sample can migrate on the support due to the developer solution and be transported through the sample-migrating region to the detection region. In this process, an influenza virus nucleoprotein in the sample reacts with the labeled antibody in the labeled reagent region, and the produced mobile labeled antibody/influenza virus nucleoprotein complex is further transported to the detection region and produces a anti-influenza virus nucleoprotein antibody/influenza virus nucleoprotein/labeled antibody complex in the detection region. The sample can be developed on the support without using a developer solution, and by using a developer solution, the sample can be developed efficiently. The developer solution is an aqueous solvent which develops a sample on a support; in addition, this aqueous solvent may include additives such as the aforementioned buffers, surfactants, antiseptics, salts, sugars, metal ions, and proteins as necessary.

### - Sample supply confirming region

The sample supply confirming region in the devices of the present invention is provided upstream of the excess liquid absorbing region, and is a region for confirming with certainty that the sample has been added to the sample supply region of the device.

In the device of the present invention which uses two antibodies (antibody 1 and antibody 2), when a sample is supplied to the sample supply region, unreacted labeled antibodies develop through the sample-migrating region together with the influenza virus nucleoprotein/labeled antibody complex formed by the reaction between the influenza virus nucleoproteins in the sample and the labeled antibodies (labeled anti-influenza virus nucleoprotein antibodies). Thus, when a sample is supplied certainly, unreacted labeled antibodies develop through the sample-migrating region. The sample supply confirming region is a region that captures these unreacted labeled antibodies, and can be constructed by immobilizing and fixing antibodies against the labeled antibodies. Examples of antibodies that react with the labeled antibodies include antibodies against the anti-influenza virus nucleoprotein antibodies, and antibodies against the label. Examples of methods for immobilizing antibodies that react with the labeled antibodies include methods of immobilizing antibodies in the detection region mentioned above. Furthermore, a sample supply confirming region can also be constructed by immobilizing and fixing antibodies against a substance included in common in a sample (for example, an enzyme) (see (3) of Fig. 1).

Similarly, in the devices of the present invention in which a single antibody is used, the sample supply confirming region can be constructed by immobilizing and fixing an antibody against a substance included in common in a sample (for example, an enzyme), an antibody against a labeled antigen analog (labeled influenza virus nucleoprotein antigen analog), or an antibody against the labeled antibody (labeled human anti-influenza virus nucleoprotein antibody).

### - Excess liquid absorbing region

The excess liquid absorbing region in the devices of the present invention is provided most downstream on the support. In summary, the sample and the labeled antibody developed together with the developer solution on the support are captured in the detection region to produce the anti-influenza nucleoprotein antibody/influenza nucleoprotein/labeled antibody complex, and this region is for absorbing the excess liquid after production of the complex. The excess liquid absorbing region can be formed from the support, and it can also be formed by attaching a water-absorbing substance to the support, or it can also be formed by layering a water-absorbing substance onto the support. Examples of the water-absorbing substance include highly water-absorbing filter paper and sponge.

### [Detection and quantification of influenza viruses using the devices]

Detection and quantification of influenza viruses using the devices of the present invention can be carried out, for example, as indicated below.

### - Embodiment 1

A sample supplied to the sample supply region develops through the detection migrating region and reaches the detection region. At the detection region, influenza virus nucleoproteins in the sample react with human anti-influenza virus nucleoprotein antibodies to form influenza virus nucleoprotein/human anti-influenza virus nucleoprotein antibody complexes. Influenza viruses can be detected or quantified by measuring a physical change accompanying this complex formation. Examples of the physical change herein include a mass change.

### - Embodiment 2

A sample and labeled antibodies (labeled anti-influenza virus nucleoprotein antibodies) supplied to the sample supply region develop through the detection migrating region and reach the detection region. Influenza virus nucleoprotein/labeled antibody complexes produced during the supplying or developing step react with anti-influenza virus nucleoprotein antibodies in the detection region to produce anti-influenza virus nucleoprotein antibody/influenza virus nucleoprotein/labeled antibody complexes. Influenza viruses can be detected or quantified by measuring the label in these anti-influenza virus nucleoprotein antibody/influenza virus nucleoprotein/labeled antibody complexes produced in the detection region.

### - Embodiment 3

A sample supplied to the sample supply region reacts with labeled anti-influenza virus nucleoprotein antibodies (labeled antibody 2) in the labeled reagent region and produces influenza virus nucleoprotein/labeled antibody 2 complexes (complex 1). Then, complex 1 which develops through the sample-migrating region to the detection region reacts with anti-influenza virus nucleoprotein antibodies (antibody 1) in the detection region, to produce antibody 1/anti-influenza virus nucleoprotein/labeled antibody 2 complexes (complex 2) in an immobilized form in the detection region, and by measuring the label of complex 2 produced in the detection region, influenza viruses can be detected or quantified. Herein at least one of antibody 1 and the anti-influenza virus nucleoprotein antibody (antibody 2) constituting the labeled anti-influenza virus nucleoprotein antibody (labeled antibody 2) is a human antibody. A developer solution may also be used for development of samples supplied to the sample supply region and for development of complex 1 produced in the labeled reagent region to the detection region.

### - Embodiment 4

A sample and labeled antigen analogs (labeled influenza virus nucleoprotein antigen analogs) supplied to the sample supply region develop through the detection migrating region and reach the detection region. In the detection region, the influenza virus nucleoprotein antigens in the sample and the labeled antigen analogs react competitively toward the human anti-influenza virus nucleoprotein antibodies, such that human anti-influenza virus nucleoprotein antibody/influenza virus nucleoprotein antigen complexes and human anti-influenza virus nucleoprotein antibody/labeled antigen analog complexes are produced. Influenza viruses can be detected or quantified by measuring the label in these complexes produced in the detection region.

### - Embodiment 5

A sample supplied to the sample supply region develops through the sample-migrating region and reaches the labeled reagent region. The sample that has reached the labeled reagent region is further developed with labeled antigen analogs (labeled influenza virus nucleoprotein antigen analogs) through the sample-migrating region and reaches the detection region. In the detection region, the influenza virus nucleoprotein antigens in the sample and the labeled antigen analogs react competitively toward the human anti-influenza virus nucleoprotein antibodies, such that human anti-influenza virus nucleoprotein antibody/influenza virus nucleoprotein antigen complexes and human anti-influenza virus nucleoprotein antibody/labeled antigen analog complexes are produced. Influenza viruses can be detected or quantified by measuring the label in these complexes produced in the detection region.

### - Embodiment 6

A sample and labeled antibodies (labeled human anti-influenza virus nucleoprotein antibodies) supplied to the sample supply region develop through the detection migrating region. Influenza virus nucleoprotein/labeled antibody complexes produced during the supplying or developing step reach the detection region together with unreacted labeled antibodies. In the detection region, the unreacted labeled antibodies react with influenza virus nucleoprotein antigen analogs to produce influenza virus nucleoprotein antigen analog/labeled antibody complexes. Influenza viruses can be detected or quantified by measuring the label in these influenza virus nucleoprotein antigen analog/labeled antibody complexes produced in the detection region.

### - Embodiment 7

A sample supplied to the sample supply region develops through the sample-migrating region and reaches the labeled reagent region. In the labeled reagent region, influenza virus nucleoproteins react with labeled antibodies (labeled human anti-influenza virus nucleoprotein antibodies) and produce influenza virus nucleoprotein/labeled antibody complexes. The produced complexes further develop together with the unreacted labeled antibodies through the sample-migrating region and reach the detection region. In the detection region, the unreacted labeled antibodies react with influenza virus nucleoprotein antigen analogs to produce influenza virus nucleoprotein antigen analog/labeled antibody complexes. Influenza viruses can be detected or quantified by measuring the label in these influenza virus nucleoprotein antigen analog/labeled antibody complexes produced in the detection region.

Herein, the label in the complexes produced in the detection region can be measured using known methods. When the label is a gold colloid particle or a colored latex particle, measurement can be carried out by measuring the absorbance of the band produced on the support due to production of the complexes. When the label is an enzyme, the label can be measured by allowing an enzyme substrate to act on the enzyme. The method of detection can be selected according to the enzyme and the enzyme substrate used.

The enzyme substrate can be included in the developer solution, and it may also be supported in the substrate reagent region provided in the device in a manner to allow migration. When the enzyme is peroxidase, examples of its substrate include chromogenic substrates and luminescent substrates. Examples of chromogenic substrates include combinations of a leuco-type chromogen with hydrogen peroxide, and combinations of hydrogen peroxide and a coupling-type chromogen containing a combination of two compounds. Examples of the leuco-type chromogen include 2,2'-azino-bis(3-ethylbenzothiazoline-6-sulfonic acid) (ABTS), 3,3',5,5'-tetramethylbenzidine (TMB), diaminobenzidine (DAB), 10-N-carboxymethylcarbamoyl-3,7-bis(dimethylamino)-10H-phenothiazine (CCAP), 10-N-methylcarbamoyl-3,7-bis(dimethylamino)-10H-phenothiazine (MCDP), N-(carboxymethylaminocarbonyl)-4,4'-bis(dimethylamino)diphenylamine sodium salt (DA-64), 10-N-(carboxymethylaminocarbonyl)-3,7-bis(dimethylamino)-10H-phenothiazine sodium salt (DA-67), 4,4'-bis(dimethylamino)diphenylamine, and bis[3-bis(4-chlorophenyl)methyl-4-dimethylaminophenyl]amine (BCMA). Examples of the coupling-type chromogens containing a combination of two compounds include a combination of a coupler and an aniline or a phenol. Examples of the coupler include 4-aminoantipyrine (4-AA) and 3-methyl-2-benzothiazolinonehydrazone. Examples of the aniline include N-ethyl-N-(3-methylphenyl)-N"-succinylethylenediamine (EMSE), N-(3,5-dimethoxyphenyl)-N'-succinylethylenediamine sodium salt (DOSE), N-ethyl-N-(2-hydroxy-3-sulfopropyl)-3-methylaniline, N-ethyl-N-(3-sulfopropyl)aniline, N-ethyl-N-(3-sulfopropyl)-3,5-dimethoxyaniline, N-(3-sulfopropyl)-3,5-dimethoxyaniline, N-ethyl-N-(3-sulfopropyl)-3,5-dimethylaniline,N-ethyl-N-(3-sulfiopropyl)-3-methylanilhe, N-ethyl-N-(2-hydroxy-3-sulfopropyl)-3-methoxylaniline, N-ethyl-N-(2-hydroxy-3-sulfopropyl)aniline, N-ethyl-N-(2-hydroxy-3-sulfbpropyl)-3-methylaniline sodium salt dihydrate (TOOS), N-ethyl-N-(2-hydroxy-3-sulfopropyl)-3,5-dimethoxyanline, N-(2-hydroxy-3-sulfopropyl)-3,5-dimethoxyaniline sodium salt (HSDA), N-ethyl-N-(2-hydroxy-3-sulfopropyl)-3,5-dimethylaniline, N-(3-sulfopropyl)aniline, N-ethyl-N-(3-sulfopropyl)-3-methoxylaniline, and N-ethyl-N-(2-hydroxy-3-sulfopropyl)-4-fluoro-3,5-dimethoxylaniline sodium salt (F-DAOS). Examples of the phenol include phenol and 3-hydroxy-2,4,6-triiodobenzoic acid. Examples of chromogenic substrates include the combination of luminol and hydrogen peroxide and the combination of isoluminol and hydrogen peroxide.

When the enzyme is alkaline phosphatase, its substrate is, for example, a chromogenic substrate, a fluorescent substrate, or a luminescent substrate. Detection or quantification is enabled by measuring the absorbance when the substrate is a chromogenic substrate, the fluorescence intensity when the substrate is a fluorescent substrate, and the luminescence intensity when the substrate is a luminescent substrate. Examples of the chromogenic substrate include 5-bromo-4-chloro-3-indolyl phosphate (BCIP) and 4-nitrophenyl phosphate. Examples of the fluorescent substrate include 4-methylumbelliferyl-phosphate (4MUP) and 4-methylumbelliphenyl-β-D-galactoside (4MUG) for β-D-galactosidase. Examples of the luminescent substrate include 3-(2'-spiroadamantane)-4-methoxy-4-(3'-phosphoryloxy)phenyl-1,2-dioxetane disodium salt (AMPPD), 2-chloro-5-{4-methoxyspiro[1,2-dioxetane-3,2'-(5'-chloro)tricyclo[3.3.1.13,7]decan]-4-yl}phenyl phosphate disodium salt (CDP-Star^{™}), 3-{4-methoxyspiro[1,2-dioxetane-3,2'-(5'-chloro)tricyclo[3.3.1.13,7]decan]-4-yl}phenyl phosphate disodium salt (CSPD^{™}), and [10-methyl-9(10H)-acridinylidene]phenoxymethylphosphate disodium salt (Lumigen^{™} APS-5).

When the enzyme is β-D-galactosidase, examples of the substrate of this enzyme include luminescent substrates such as 3-(2'-spiroadamantane)-4-methoxy-4-(3"-β-D-galactopyranosyl)phenyl-1,2-dioxetane (AMGPD).

Furthermore, devices of the present invention should only include a Human anti-influenza virus nucleoprotein antibody as its constituent, and the method of detection in the detection region is not particularly limited. Not only the aforementioned chromogenic methods (absorbance method), fluorescence methods, and luminescence methods can be used as detection method, and methods for detecting a mass change can also be used. Examples of methods for detecting a mass change include surface plasmon resonance (SPR) methods and methods that use a piezoelectric vibrator (see for example, WO2005/015217 amphlet).

Quantification of an influenza virus in a sample using a device of the present invention can be carried out as follows. First, influenza virus nucleoproteins prepared from influenza viruses having known concentrations are used as samples, these samples are supplied to the sample supply region of a device of the present invention, the information at the detection region (for example, the absorbance) is measured, and a calibration curve showing the relationship between the influenza virus concentration and the amount of information is produced. Subsequently, similar measurements are taken using actual object samples, and the amount of information obtained is correlated to the previously-prepared calibration curve to determine the concentration.

### [Methods for detecting or quantifying influenza viruses]

The methods of the present invention for detecting or quantifying influenza viruses are methods in which at least one or more human anti-influenza virus nucleoprotein antibodies are used. Influenza viruses can be detected or quantified using the kits of the present invention for detecting or quantifying influenza viruses which will be described later.

The methods of the present invention for detecting or quantifying influenza viruses are not particularly limited as long as they are methods that can detect or quantify influenza viruses, and examples include radioimmunoassay (RIA), immunoradiometric assay (IRMA), enzyme-linked immunosorbent assay (ELISA), homogeneous enzyme immunoassay, fluorescent immunoassay (FIA), immunofluorimetric assay (IFMA), fluorescence polarization assay, chemiluminescent immunoassay (CLIA), chemiluminescent enzyme immunoassay (CLEIA), nephelometric immunoassay, and surface plasmon resonance (SPR) method.

Examples of the methods of the present invention for detecting or quantifying an influenza virus include the methods indicated below.

### - Method 1

A method for detecting or quantifying an influenza virus, comprising the steps of:
(1) reacting a sample with a human anti-influenza virus nucleoprotein antibody fixed on a carrier; and
(2) measuring a physical change produced in step (1).

Examples of the physical change in the amount of physical change in this method include turbidity change and mass change. This method can be applied to nephelometric immunoassay which measures the turbidity change of the reaction solution accompanying the antigen antibody reaction, or to surface plasmon resonance (SPR) method which measures the mass change accompanying the antigen antibody reaction.

### - Method 2

A method for detecting or quantifying an influenza virus, comprising the steps of:
(1) reacting a sample with an anti-influenza virus nucleoprotein antibody (antibody 1) fixed on a carrier to form an antibody 1/influenza virus complex on the carrier;
(2) reacting the complex formed on the carrier in step (1) with an anti-influenza virus nucleoprotein antibody (antibody 2); and
(3) measuring the amount of physical change induced in step (2);
wherein at least one of antibody 1 and antibody 2 is a human anti-influenza virus nucleoprotein antibody.

Antibody 1 and antibody 2 may be a monoclonal antibody or a polyclonal antibody, and preferably both are monoclonal antibodies.

Examples of the physical change in the amount of physical change in this method include turbidity change and mass change. This method can be applied to nephelometric immunoassay which measures the turbidity change of the reaction solution accompanying the antigen antibody reaction, or to surface plasmon resonance (SPR) method which measures the mass change accompanying the antigen antibody reaction. Step (1) and step (2) may be performed simultaneously.

### - Method 3

A method for detecting or quantifying an influenza virus, comprising the steps of:
(1) reacting a sample with an anti-influenza virus nucleoprotein antibody (antibody 1) fixed on a carrier to form an antibody l/influenza virus nucleoprotein complex on the carrier;
(2) reacting the complex formed on the carrier in step (1) with a labeled antibody (labeled antibody 2) in which a label is bound to an anti-influenza virus nucleoprotein antibody (antibody 2) to form an antibody 1/influenza virus nucleoprotein/labeled antibody 2 complex on the carrier;
(3) washing the carrier after step (2) to remove substances not bound to the carrier; and
(4) measuring the label in the antibody 1/influenza virus nucleoprotein/labeled antibody 2 complex on the carrier after step (3);
wherein at least one of antibody 1 and antibody 2 is a human anti-influenza virus nucleoprotein antibody.

The above-described method is an embodiment of a sandwich method. Regarding antibody 1 and antibody 2 in the above-described method, the regions of the influenza virus nucleoprotein recognized by the respective antibodies may be the same or different, and the regions are preferably different. Furthermore, antibody 1 and antibody 2 in the above-described method may individually be a monoclonal antibody or polyclonal antibody, and preferably both are monoclonal antibodies.

In the above-described method, when the difference between the amount of information derived from the label in the complex produced in step (4) and the amount of information derived from the label in the unreacted labeled antibody present in the reaction system in step (2) is significant, the washing step of step (3) can be omitted (homogeneous method). Furthermore, step (1) and step (2) may be performed simultaneously, and a washing step may be inserted between step (1) and step (2).

Examples of the methods for measuring the label in step (4) of the above-described method include the aforementioned methods.

### - Method 4

A method for detecting or quantifying an influenza virus, comprising the steps of:
(1) reacting a sample with a labeled antibody (labeled antibody 2) in which a label is bound to an anti-influenza virus nucleoprotein antibody (antibody 2) to form a labeled antibody 2/influenza virus nucleoprotein complex;
(2) reacting the complex formed in step (1) with an anti-influenza virus nucleoprotein antibody (antibody 1) fixed on a carrier to form an antibody 1/influenza virus nucleoprotein/labeled antibody 2 complex on the carrier;
(3) washing the carrier after step (2) to remove substances not bound to the carrier; and
(4) measuring the label in the antibody 1/influenza virus nucleoprotein/labeled antibody 2 complex on the carrier after step (3);
wherein at least one of antibody 1 and antibody 2 is a human anti-influenza virus nucleoprotein antibody.

The above-described method is another embodiment of a sandwich method. Regarding antibody 1 and antibody 2 in the above-described method, the regions of the influenza virus nucleoprotein recognized by the respective antibodies may be the same or different, and the regions are preferably different. Furthermore, antibody 1 and antibody 2 in the above-described method may individually be a monoclonal antibody or a polyclonal antibody, and preferably both are monoclonal antibodies.

In the above-described method, when the difference between the amount of information derived from the label in the complex produced in step (2) and the amount of information derived from the label in the unreacted labeled antibody present in the reaction system in step (2) is significant, the washing step of step (3) can be omitted (homogeneous method). Furthermore, step (1) and step (2) may be performed simultaneously, and a washing step may be inserted between step (1) and step (2).

Examples of the method for measuring the label in step (4) of the above-described method include the aforementioned methods.

### - Method 5

A method for detecting or quantifying an influenza virus, comprising the steps of:
(1) reacting a sample with a human anti-influenza virus nucleoprotein antibody fixed on a carrier in the co-presence of a labeled antigen analog in which a label is bound to an influenza virus nucleoprotein antigen analog, to form a human anti-influenza virus nucleoprotein antibody/influenza virus nucleoprotein Complex and a human anti-influenza virus nucleoprotein antibody/labeled antigen analog complex on the carrier;
(2) washing the carrier after step (1) to remove substances not bound to the carrier; and
(3) measuring the label in the complex on the carrier after step (2).

The above-described method is an embodiment of a competition method. The human anti-influenza virus nucleoprotein antibody in the above-described method may be a monoclonal antibody or a polyclonal antibody, and it is preferably a monoclonal antibody.

Examples of the method for measuring the label in step (3) of the above-described method include the aforementioned methods.

### - Method 6

A method for detecting or quantifying an influenza virus, comprising the steps of:
(1) reacting a sample with an influenza virus nucleoprotein antigen analog fixed on a carrier in the co-presence of a labeled antibody in which a label is bound to a human anti-influenza virus nucleoprotein antibody, to form an influenza virus nucleoprotein antigen analog/labeled antibody complex on the carrier;
(2) washing the carrier after step (1) to remove substances not bound to the carrier; and
(3) measuring the label in the complex on the carrier after step (2).

The above-described method is another embodiment of the competition method. The human anti-influenza virus nucleoprotein antibody in the above-described method may be a monoclonal antibody or a polyclonal antibody, and it is preferably a monoclonal antibody.

Examples of the method for measuring the label in step (3) of the above-described method include the aforementioned methods.

The reaction conditions in the methods of the present invention can be set appropriately by those skilled in the art, and for example, the reaction temperature is 0°C to 50°C, and preferably 4°C to 40°C, and the reaction time is 1 minute to 24 hours, and preferably 3 minutes to 12 hours. Furthermore, when a washing step is included, washing of the carrier can be carried out using, for example, phosphate buffered saline (PBS).

The solid phase used in the methods of the present invention has an anti-influenza virus nucleoprotein antibody or a labeled antigen analog immobilized and fixed onto a carrier. The carrier is not particularly limited as long as it can allow immobilization and fixing of an anti-influenza virus nucleoprotein antibody or a labeled antigen analog, and is water insoluble, and examples include polystyrene plates such as microtiter plates, granulated substances (beads) made of glass or synthetic resin, globular substances (balls) made of glass or synthetic resin, latex, magnetic particles, nitrocellulose membrane, nylon membrane, and tube made of synthetic resin. Examples of the methods for immobilizing and fixing an anti-influenza virus nucleoprotein antibody or a labeled antigen analog to the carrier include direct methods and indirect methods. Examples of direct methods include methods of linking a functional group on the carrier with a functional group in the anti-influenza virus nucleoprotein antibody or in the labeled antigen analog through covalent bonds. Herein, examples of the combination of the functional group on the carrier and the functional group in the anti-influenza virus nucleoprotein antibody or the labeled antigen analog include amino group - carboxyl group, and carboxyl group - amino group.

Examples of indirect methods include methods of indirectly linking a functional group on the carrier with a functional group in the anti-influenza virus nucleoprotein antibody or in the labeled antigen analog through a linker, and methods of using interactions between substances. Herein, examples of the combination of the functional group on the carrier and the functional group in the anti-influenza virus nucleoprotein antibody or the labeled antigen analog include amino group - amino group, amino group - carboxyl group, amino group - sulfhydryl group, carboxyl group - amino group, carboxyl group - carboxyl group, and carboxyl group - sulfhydryl group, and such. The functional group may be introduced by chemical modification. Examples of methods that utilize interactions between substances include methods that utilize the avidin-biotin interaction and methods that utilize the sugar-lectin interaction. When utilizing the avidin-biotin interaction, for example, by reacting an avidin-adsorbed carrier with a biotinylated anti-influenza virus nucleoprotein antibody or a biotinylated labeled antigen analog, the anti-influenza virus nucleoprotein antibody or the labeled antigen analog can be immobilized and fixed onto the carrier.

Furthermore, a solid phase prepared by further blocking a carrier having an anti-influenza virus nucleoprotein antibody or labeled antigen analog immobilized and fixed on to it with bovine serum albumin, casein, and such may be used as the solid phase used in the methods of the present invention.

Examples of the human anti-influenza virus nucleoprotein antibodies used in the methods of the present invention include the below-described human anti-influenza A virus nucleoprotein antibodies and the human anti-influenza B virus nucleoprotein antibodies.

Examples of the labels used in the methods of the present invention include the aforementioned labels. Examples of the methods for measuring the labels in the methods of the present invention include the aforementioned methods for measuring the labels.

The labeled anti-influenza virus nucleoprotein antibodies used in the methods of the present invention can be prepared from a label and an anti-influenza virus nucleoprotein antibody according to the aforementioned methods.

The influenza virus nucleoprotein antigen analogs used in the methods of the present invention are substances that compete with an influenza virus nucleoprotein in the sample to react the human anti-influenza virus nucleoprotein antibody fixed on the carrier, and examples include not only the influenza virus nucleoprotein itself but also substances containing an epitope found in the influenza virus nucleoprotein recognized by the anti-influenza virus nucleoprotein antibody.

The labeled antigen analogs (labeled influenza virus nucleoprotein antigen analogs) used in the methods of the present invention can be prepared by methods similar to the aforementioned methods for preparing labeled antibodies.

### [Kits for detecting or quantifying influenza viruses]

The kits of the present invention for detecting or quantifying an influenza virus are kits used for the methods of the present invention for detecting or quantifying an influenza virus.

Examples of the kits of the present invention for detecting or quantifying an influenza virus include the kits described below.

### - Kit 1

A kit for detecting or quantifying an influenza virus, which comprises a solid phase produced by fixing a human anti-influenza virus nucleoprotein antibody to a carrier.

The human anti-influenza virus nucleoprotein antibody may be a monoclonal antibody or a polyclonal antibody.

This kit is suitable for methods such as nephelometric immunoassays and surface plasmon resonance (SPR) methods.

### - Kit 2

A kit for detecting or quantifying an influenza virus, which comprises a solid phase produced by fixing an anti-influenza virus nucleoprotein antibody (antibody 1) to a carrier, and
a reagent containing an anti-influenza virus nucleoprotein antibody (antibody 2), and wherein at least one of antibody 1 and antibody 2 is a human anti-influenza virus nucleoprotein antibody.

Antibody 1 and antibody 2 may be a monoclonal antibody or a polyclonal antibody, and preferably, both are monoclonal antibodies.

This kit is suitable for methods such as nephelometric immunoassays and surface plasmon resonance (SPR) methods.

### -Kit 3

A kit for detecting or quantifying an influenza virus, which comprises
a solid phase produced by fixing an anti-influenza virus nucleoprotein antibody (antibody 1) to a carrier, and
a reagent containing a labeled antibody in which a label is bound to an anti-influenza virus nucleoprotein antibody (antibody 2),
and wherein at least one of antibody 1 and antibody 2 is a human anti-influenza virus nucleoprotein antibody.

Antibody 1 and antibody 2 may be monoclonal antibodies or polyclonal antibodies, and preferably, both are monoclonal antibodies.

This kit is suitable for immunoassays that are based on the sandwich method.

### - Kit 4

A kit for detecting or quantifying an influenza virus, which comprises
a solid phase produced by fixing a human anti-influenza virus nucleoprotein antibody to a carrier, and
a reagent containing a labeled antigen analog in which a label is bound to an influenza virus nucleoprotein antigen analog.

This kit is suitable for immunoassays that are based on competition methods.

### - Kit 5

A kit for detecting or quantifying an influenza virus, which comprises a solid phase produced by fixing an influenza virus nucleoprotein antigen analog to a carrier, and a reagent containing a labeled antibody in which a label is bound to a human anti-influenza virus nucleoprotein antibody.

This kit is suitable for immunoassays that are based on competition methods.

Examples of the solid phases used in the kits of the present invention for detecting or quantifying an influenza virus include the aforementioned solid phases.

Examples of the human anti-influenza virus nucleoprotein antibodies used in the kits of the present invention include the below-described human anti-influenza A virus nucleoprotein antibodies and the human anti-influenza B virus nucleoprotein antibodies.

Examples of the labels used in the kits of the present invention include the aforementioned labels. Examples of methods for measuring labels in the kits of the present invention include the aforementioned methods for measuring labels.

The kits of the present invention may include as necessary not only the aforementioned aqueous solvents but also the aforementioned additives such as buffers, surfactants, antiseptics, salts, sugars, metal ions, and proteins. These additives can be used as additive-containing reagents separately from the solid phase and reagents constituting the kits of the present invention, and they may also be used by including them into either of or both of the solid phase and the reagents constituting the kits of the present invention.

### [Human anti-influenza virus nucleoprotein antibodies]

The human anti-influenza virus nucleoprotein antibodies of the present invention are antibodies that can be used for the devices of the present invention for detecting or quantifying an influenza virus, kits of the present invention for detecting or quantifying an influenza virus, and methods of the present invention for detecting or quantifying an influenza virus. Examples include human anti-influenza A virus nucleoprotein antibodies and human anti-influenza B virus nucleoprotein antibodies. Furthermore, the human anti-influenza virus nucleoprotein antibodies of the present invention may be monoclonal antibodies or polyclonal antibodies, and they are preferably monoclonal antibodies.

The human anti-influenza A virus nucleoprotein monoclonal antibodies of the present invention are preferably antibodies that react specifically with an influenza A virus nucleoprotein but do not react with an influenza B virus nucleoprotein. Furthermore, the human anti-influenza B virus nucleoprotein monoclonal antibodies of the present invention are preferably antibodies that react specifically with an influenza B virus nucleoprotein but do not react with an influenza A virus nucleoprotein.

### - Methods for producing human anti-influenza virus nucleoprotein monoclonal antibodies

As described above, the human anti-influenza virus nucleoprotein antibodies of the present invention are preferably human anti-influenza virus nucleoprotein monoclonal antibodies. Human anti-influenza virus nucleoprotein monoclonal antibodies can be produced by known monoclonal antibody production methods [hybridoma methods (see for example, In Vitro Cell Dev Biol. (1985) 21(10):593-596), viral infection methods (see for example, J. gen. Virol. (1983), 64, 697-700), phage display methods (see for example, WO 2001/062907 pamphlet), lymphocyte microarray methods, and such], and the lymphocyte microarray methods are preferred.

Lymphocyte microarray methods are techniques relating to monoclonal antibody production method jointly developed by the University of Toyama and SC World, Inc. [Japanese

Patent Application *Kokai* Publication No. (JP-A) 2004-173681 (unexamined, published Japanese patent application); JP-A (Kokai) 2004-187676; JP-A (Kokai) 2005-261339; JP-A (Kokai) 2007-14267; Anal. Chem. 77(24), p.8050-8056 (2005); Cytometry A 71(11), p.961-967 (2007); Cytometry A 71(12), p.1003-1010 (2007)] and include the following steps:
[1] the step of preparing B lymphocytes from human blood;
[2] the step of selecting B lymphocytes that react with an object of measurement;
[3] the step of obtaining genes relating to antibodies that react with the object of measurement from the selected B lymphocytes;
[4] the step of expressing the antibodies using the obtained genes; and
[5] the step of selecting antibodies having the desired properties.

Hereinafter, each of the above-mentioned steps regarding production of human anti-influenza virus nucleoprotein monoclonal antibodies is described.

### [1] The step of preparing B lymphocytes from human blood

Human B lymphocytes can be prepared from human peripheral blood, and in particular, they can be prepared from human peripheral blood having a history of influenza virus infection or history of influenza vaccination, in which the presence of influenza virus nucleoprotein antibodies can be confirmed in the serum. Desirably, they are prepared by collecting blood after a certain period of time has elapsed, preferably one to 30 days after, or more preferably five to ten days after influenza vaccination. Specific examples of the methods for preparation include methods that use density gradient centrifugation, cell sorter, magnetic beads, or such.

### [2] The step of selecting B lymphocytes that react with an object of measurement

Selection of a single B lymphocyte specific to an antigen can be carried out, for example, by a method using a microwell array chip. In the method using a microwell array chip, for example, the antigen is added to each microwell of a microwell array chip for antigen-specific B lymphocyte detection, which has multiple microwells containing a single test B lymphocyte, then B lymphocytes that reacted with the antigen are detected, and by collecting the detected antigen-specific B lymphocytes from the microwells, a single B lymphocyte specific to the antigen can be obtained. Hereinafter, this method is described in more detail.

Microwell array chips having multiple microwells and in which each microwell can contain a single test B lymphocyte can be used. By including a single test B lymphocyte in each microwell, antigen-specific B lymphocytes can be identified at the cellular level. That is, when this microwell array chip is used, the test B lymphocyte included in a microwell is a single cell; therefore, test B lymphocyte that reacts with the antigen can be identified as a single cell, and as a result, antigen-specific B lymphocyte can be detected as a single cell. Then, the detected single antigen-specific B lymphocyte is collected and its gene is cloned. There are no particular limitations on the shape or dimensions of the microwells, and the shape of the microwell can be, for example, cylindrical, or besides that, it may be cuboidal, inverted cone shaped, inverted pyramid (inverted triangular pyramid, inverted quadrangular pyramid, inverted five-sided pyramid, inverted six-sided pyramid, inverted multi-sided pyramid having seven or more sides) shaped, or it may be a shape produced by combining two or more of these shapes. For example, a part may be cylindrical and the rest may be inverted cone shaped. Furthermore, in the case of inverted cones or inverted pyramids, the base is the opening of the microwell; however, the shape may be one in which a portion of the apex of the inverted cone or inverted pyramid is cut off (in which case the bottom of the microwell will be flat). For cones and cuboids, the bottom of the microwell is normally flat, but it can also be a curved surface (convex or concave). The bottom of a microwell can be made into a curved surface also for shapes in which a portion of the apex of an inverted cone shape or inverted pyramid is cut off. The test B lymphocyte is stored together with the culture solution in the microwell. Examples of the culture solution include any one of the following:
1. 137 mmol/L NaCl, 2.7 mmol/L KCI, 1.8 mmol/L CaCl₂, 1 mmol/L MgCl₂, 1 mg/mL glucose, 1 mg/mL BSA, 20 mmol/L HEPES (pH7.4)
2. 10% FCS (fetal calf serum)=containing RPMI1640 medium
3. 1 mg/mL BSA-containing RPMI1640 medium
4. 10% FCS (fetal calf serum)-containing Dulbecco's MEM medium
5. 1 mg/mL BSA-containing Dulbecco's MEM medium

### Detection of cells that react with the antigen can be carried out as follows.

For Example, when an antigen binds to the antigen receptor (immunoglobulin) of a B lymphocyte, initially intracellular signal transduction takes place, and subsequently, cell proliferation and antibody production take place. Therefore, by detecting intracellular signal transduction, cell proliferation, or antibody production by various methods, cells that react with the antigen can be detected. Detection of cells that react with the antigen by detecting intracellular signal transduction can be carried out, for example, by observing the change in intracellular Ca ion concentration using a Ca ion-dependent fluorescent dye. Change in intracellular Ca ion concentration is observed using Fura-2, Fluo-3, or Fluo-4 as the fluorescent dye, and a fluorescence microscope or a microarray scanner as the detecting device.

### [3] The step of obtaining genes relating to antibodies that react with the object of measurement from the selected B lymphocytes

The collected antigen-specific B lymphocytes are dissolved using a cell lysis agent, then PCR is used to clone the antigen-specific immunoglobulin (antibody) genes. As the cell lysis agent, known substances can be used as they are, and examples include the following: 1x 1st strand buffer [GIBCO-BRL, provided with SuperScriptII], 0.2 mmol/L dNTP, 0.25% NP-40, 0.1 mg/mL BSA, 10 mmol/L DTT, Random Primer 0.05 µmol/L, 1U/µL RNasin.

The antigen receptor gene in B lymphocytes is the same as the antibody gene, and as a protein, it is called immunoglobulin. Antigen receptors exist on the B lymphocyte cell surface (membrane-type immunoglobulin), and the antibodies are normally produced as secretory proteins (secretory immunoglobulins). Their difference is in the C-terminal side of the protein. Membrane-type immunoglobulins have a membrane domain which is buried in the cell membrane and a portion that protrudes to the cytoplasm side. Secretory immunoglobulins are also produced from the same genes. However, due to alternative splicing, the C-terminal side of the protein of the secretory immunoglobulins is different from that of the membrane-type immunoglobulin so that secretory immunoglobulins do not have any membrane domain. As a result, secretory immunoglobulins are produced as a secretory protein, and the antigen-binding sites of these two proteins are the same. Therefore, in the case of B lymphocytes, cloning of the antibody gene and cloning of the antigen receptor gene are the same.

Preparation of cDNA by reverse transcriptase is carried out using the solution obtained by dissolving the collected antigen-specific B lymphocytes using a cell lysis agent.

Next, tailing reaction at the 3'-end of the cDNA by DNA polymerase is carried out to perform tailing of adenine, guanine, cytosine, or thymine to the 3'-end of the cDNA.

The desired antigen-specific immunoglobulin gene can be amplified by performing PCR twice using a primer mix for immunoglobulin genes.

Preparation of cDNA by reverse transcriptase can be carried out by a common procedure [for example, Sambrook J, Russell DW in Molecular Cloning: A Laboratory Manual 3rd ed (Cold Spring Harbor Laboratory Press, New York, 2001)].

In the present invention, the RT reaction is preferably directly carried out using a cell lysate, instead of performing a reverse transcription reaction using RNA extracted and purified from cells.

### (Amplification of an antibody gene by PCR method)

In the amplification of an antibody gene by PCR method, the V region genes of the antibody gene is amplified preferably by performing the PCR reaction twice.

An antibody molecule is composed of a combination of H chains and L chains, and the H chain of a human antibody gene is composed of approximately 200 types of V-region gene fragments, approximately 20 types of D-fragments, and 6 types of J-fragments in the germ cell line. Upon differentiation into B lymphocytes, one type each of V-, D-, and J-fragments are combined to form a single antigen-binding site by gene rearrangement (V/D/J rearrangement). This is the same for the L chain.

Each B lymphocyte expresses one type of antibody molecule on the cell surface. To amplify an antigen-specific antibody gene from antigen-specific B lymphocytes, it is necessary to use primers that match each of the V-fragment sequences.

### [4] The step of expressing the antibodies using the obtained genes

As described above, cDNAs encoding the heavy (H) and light (L) chains of the antibody are obtained from cells using genetic engineering techniques, a recombinant vector in which the obtained cDNAs are inserted downstream of a promoter of the vector for antibody expression is produced, and antibody-expressing cells are obtained by introducing this vector into host cells. By culturing these cells in a suitable medium or by administering them to animals to transform the cells into ascites carcinoma, and separating and purifying the culture solution or ascitic fluid, the antibodies can be prepared. Such vector for antibody expression is an expression vector for animal cells in which genes encoding a constant region heavy chain (CH) and constant region light chain (CL), which are the constant regions (C regions) of a human antibody, are incorporated, and is constructed by inserting each of the genes encoding CH and CL of a human antibody into the expression vector for animal cells.

As human antibody C region, Cγ1 and Cγ4 may be used for the human antibody H chain, and Cκ may be used for the human antibody L chain. As genes encoding the antibody C regions, chromosomal DNAs consisting of exons and introns can be used, and alternatively, cDNAs may also be used. As an expression vector for animal cells, any vector may be used as long as it can incorporate and express a gene encoding an antibody C region.

Examples of the vector include pAGE107 [Cytotechnology, 3, 133 (1990)], pAGE103 [J. Biochem, 101, 1307 (1987)], pHSG274 [Gene, 27, 223 (1984)], pKCR [Proc. Natl., Acad. Sci., 78, 1527 (1981)], and pSG1βd2-4 [Cytotechnology, 4, 173 (1990)]. Examples of promoters and enhancers used in the vectors for expression in animals include the early promoter and enhancer of SV40 [J. Biochem, 101, 1307 (1987)], the LTR promoter and enhancer of Moloney mouse leukemia virus [Biochem. Biophys. Res. Comun., 149, 960 (1987)], and the promoter [Cell, 41, 479 (1985)] and enhancer [Cell, 33, 717 (1983)] of an immunoglobulin H chain.

For the vectors for expression, either of the type in which the antibody H-chain gene and the L-chain gene are present on separate vectors or the type in which the genes exist on the same vector (tandem type) may be used.

Transformant strains which stably produce the antibodies can be obtained by introducing the aforementioned expression vector into suitable host cells. Methods for introducing expression vectors into host cells include the electroporation method [JP-A (Kokai) H02-257891, Cytotechnology, 3, 133 (1990)]. As host cells for introducing the expression vectors, any cells may be used as long as they are host cells that can express the antibodies. Examples include mouse SP2/0-Ag cells (ATCC CRL1581), mouse P3X63-Ag8.653 cells (ATCC CRL1580), CHO cells in which the dihydrofolate reductase gene (hereinafter, referred to as DHFR gene) is defective [Proc. Natl. Acad. Sci., 77, 4216 (1980)], and rat YB2/3HL.P2.G11.16Ag.20 cells (ATCC CRL1662, hereinafter referred to as YB2/0 cells).

### [5] The step of selecting antibodies having the desired properties

Antibodies obtained in the previous step can be purified from the culture supernatant of the transformant strains using a protein A column (Antibodies, Chapter 8). Additionally, other purification methods used for ordinary proteins, for example, gel filtration, ion exchange chromatography, and ultrafiltration may be used separately or in combination. The molecular weight of the purified recombinant antibody H chain or L chain or of the whole antibody molecule is determined by polyacrylamide electrophoresis (SDS-PAGE) [Nature, 227, 680 (1970)], Western blotting (Antibodies, Chapter 12), or such.

The binding ability of the antibodies in this culture supernatant to the influenza virus nucleoprotein is measured by the ELISA method using a 96-well coated with the influenza virus nucleoprotein. Measurement by ELISA can be carried out as follows. Specifically, the influenza virus nucleoprotein diluted in PBS (10 µg/mL) is dispensed at 50 µL per well into a 96-well plate, and this is left to stand at 4°C overnight for adsorption. The wells are washed with PBS, and to remove non-specific binding, 3% BSA, 0.05% Tween-20-containing PBS is dispensed at 400 µL per well, and this is allowed to react at room temperature for two hours for blocking. As negative control wells, wells that are blocked without antigen adsorption are also prepared. Thereafter, the wells are washed with 0.1% Tween-20-containing PBS (PBS-T), the culture supernatant of the above-mentioned 293T cells is dispensed at 50 µL per well, and this is allowed to react at room temperature for two hours. The wells are washed with PBS-T, alkaline phosphatase (ALP)-labeled anti-human immunoglobulin diluted 1000 times is dispensed at 50 µL per well, and this is allowed to react at room temperature for two hours. The wells are washed with PBS-T, 1 mg/mL *p*-nitrophenyl phosphate (ALP substrate) dissolved in a buffer for ALP substrate [100 mmol/L sodium chloride, 5 mmol/L magnesium chloride, 100 mmol/L Tris buffer (pH9.5)] is dispensed at 50 µL per well, and after reaction at room temperature for 20 minutes, the absorbance at 405 nm is measured on a microplate reader.

Furthermore, to check the specificity of binding of a monoclonal antibody of the present invention to the antigen, when adding the antibody-containing cell culture supernatant to the wells, a soluble influenza virus nucleoprotein and the culture supernatant were pre-incubated. This mixed solution is added to the wells to examine whether binding of the antibodies to the recombinant nucleoprotein adsorbed onto the wells is competitively inhibited by the soluble recombinant nucleoprotein.

Specific examples of the human anti-influenza A virus nucleoprotein monoclonal antibody in the present invention include human anti-influenza A virus nucleoprotein monoclonal antibodies in which the amino acid sequence of the heavy chain variable region comprises the amino acid sequence of any one of SEQ ID NOs: 8 to 13 and the amino acid sequence of the light chain variable region comprises the amino acid sequence of any one of SEQ ID NOs: 22 to 27. A preferred embodiment is for example, a human anti-influenza A virus nucleoprotein monoclonal antibody comprising a heavy chain variable region and a light chain variable region composed of a combination of amino acid sequences selected from the group consisting of combinations of amino acid sequences having the following SEQ ID NOs:
- The amino acid sequence of the heavy chain variable region and the amino acid sequence of the light chain variable region are, respectively, SEQ ID NOs: 8 and 22, SEQ ID NOs: 9 and 23, SEQ ID NOs: 10 and 24, SEQ ID NOs: 11 and 25, SEQ ID NOs: 12 and 26, and SEQ ID NOs: 13 and 27.

An example of a preferred embodiment of the human anti-influenza B virus nucleoprotein monoclonal antibodies of the present invention is a human anti-influenza B virus nucleoprotein monoclonal antibody comprising a heavy chain variable region comprising the amino acid sequence of SEQ ID NO: 14 and a light chain variable region comprising the amino acid sequence of SEQ ID NO: 28.

As mentioned above, when two anti-influenza virus nucleoprotein antibodies are used in the present invention, a non-human anti-influenza virus nucleoprotein antibody can be used together with a human anti-influenza virus nucleoprotein antibody. Examples of non-human animals include mouse, rat, rabbit, and such. The non-human anti-influenza virus nucleoprotein antibody may be a monoclonal or polyclonal antibody, and it is preferably a monoclonal antibody. The non-human anti-influenza virus nucleoprotein monoclonal antibody may be produced for example, by the aforementioned known monoclonal antibody production methods. Examples of the non-human anti-influenza virus nucleoprotein monoclonal antibody include mouse anti-influenza A virus nucleoprotein monoclonal antibodies, mouse anti-influenza B virus nucleoprotein monoclonal antibodies, rat anti-influenza A virus nucleoprotein monoclonal antibodies, rat anti-influenza B virus nucleoprotein monoclonal antibodies, rabbit anti-influenza A virus nucleoprotein monoclonal antibodies, and rabbit anti-influenza B virus nucleoprotein monoclonal antibodies. In the present invention, not only the monoclonal antibodies produced by the aforementioned known monoclonal antibody production methods, but also commercial products can be used as the non-human anti-influenza virus nucleoprotein monoclonal antibody. Specific examples of commercially available products include M322211 and M2110169 (which are manufactured by Fitzgerald) and ATCC HB-65, which are commercially available mouse anti-influenza A virus nucleoprotein monoclonal antibodies, and M2110171 (manufactured by Fitzgerald) and 41027 (manufactured by Capricorn) which are commercially available mouse anti-influenza B virus nucleoprotein monoclonal antibodies.

All prior art references cited herein are incorporated by reference into this description.

Herein below, the present invention will be specifically described with reference to Examples, but it is not to be construed as being limited thereto.

### Examples

### [Example 1]

### Devices for detecting or quantifying influenza viruses

### - Preparation of alkaline phosphatase-labeled antibodies

Alkaline phosphatase-labeled antibodies were prepared using the Alkaline Phosphatase Labeling Kit (manufactured by Dojindo Laboratories) and two types of human anti-influenza virus nucleoprotein monoclonal antibodies [23G285 (type A) and 23G327 (type B)] obtained in Example 4 described below and two types of mouse anti-influenza virus nucleoprotein monoclonal antibodies [M2110169 (type A; manufactured by Fitzgerald) and M2110171 (type B; manufactured by Fitzgerald)].

### - Devices for detecting or quantifying influenza A virus using alkaline phosphatase-labeled antibodies

PBS solutions of mouse anti-influenza A virus nucleoprotein monoclonal antibodies M2110169 and M322211 (manufactured by Fitzgerald) and of human anti-influenza A virus nucleoprotein monoclonal antibody 23G268 were each dropped on a region of a filter paper (manufactured by Millipore) cut to 5 cm x 0.5 cm, the monoclonal antibodies were fixed, and these were used as detection region. From the lower side (the sample supply region) of the filter paper, the following were developed in this order: influenza A virus solution (H3N2: A/Panama/2007/99) diluted using a solution for reaction containing Nonidet P-40; a Tris solution of alkaline phosphatase-labeled mouse anti-influenza A virus nucleoprotein monoclonal antibody M2110169 or alkaline phosphatase-labeled human influenza A virus nucleoprotein monoclonal antibody 23G285; and BCIP/NBT (nitro-blue tetrazolium chloride) solution (manufactured by Sigma). The result is indicated in Fig. 2-a. In particular, in combinations of solid phase antibody (antibody immobilized in the detection region) and labeled antibody (antibody used for labeling) which use the human anti-influenza virus nucleoprotein antibody, a prominent blue spot was observed in the detection region. From this, it was revealed that human anti-influenza A virus nucleoprotein antibodies can be used for detecting influenza A viruses by immunochromatography, and that human anti-influenza A virus nucleoprotein antibodies are useful in highly sensitive detection of influenza A viruses.

Moreover, the performance of the present device was examined using a device comprising a detection region in which human anti-influenza A virus nucleoprotein antibody 23G268 is immobilized and fixed and a sample supply region at the lower side of the filter paper (Fig. 2-b). An influenza A virus solution (H3N2: A/Panama/2007/99) diluted in a solution for reaction containing Nonidet P-40, an influenza A virus solution (H1N1: ABeijingl262/95) diluted in a solution for reaction containing Nonidet P-40, and an influenza B virus solution (B/Victoria/504/00) diluted in a solution for reaction containing Nonidet P-40 were each supplied as sample from the sample supply region. Then, a Tris solution of alkaline phosphatase-labeled antibodies, in which alkaline phosphatase is bound to the human anti-influenza A virus nucleoprotein antibody 23 G285, was developed in the sample supply region of each of the devices, and a BCIP/NBT (nitro-blue tetrazolium chloride) solution was further developed from the developer solution supply region. As a result, as shown in Fig. 2-b, a blue spot was detected in the detection region only in the cases where influenza A virus solution (H3N2: A/Panama/2007/99) and influenza A virus solution (H1N1: A/Beijing/262/95) were used. Accordingly, it was revealed that type A influenza virus can be specifically detected by using the human anti-type A influenza virus nucleoprotein antibody 23G268 and the human anti-type A influenza virus nucleoprotein antibody 23G285.

### - Devices for detecting or quantifying influenza B virus using alkaline phosphatase-labeled antibodies

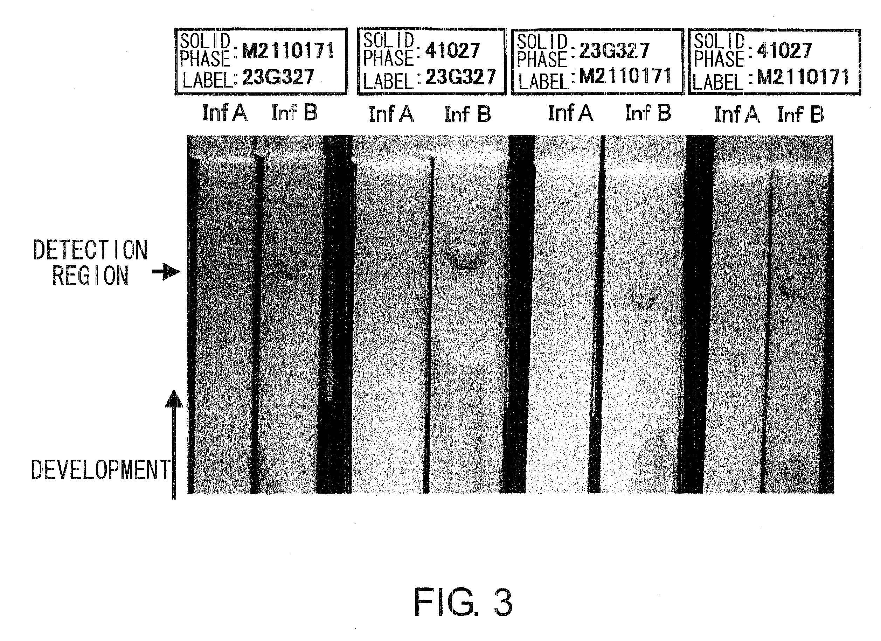

PBS solutions of mouse anti- influenza B virus nucleoprotein monoclonal antibodies M2110171 (manufactured by Fitzgerald) and 41027 (manufactured by Capricorn) were each dropped on a region of a filter paper (manufactured by Millipore) cut to 5 cm x 0.5 cm, the monoclonal antibodies were fixed, and these were used as detection region. From the lower side (the sample supply region) of the filter paper, an influenza A virus solution (H1N1: A/Beijing/262/95) diluted using a solution for reaction containing Nonidet P-40 and an influenza B virus solution (B/Victoria/504/00) diluted using a solution for reaction containing Nonidet P-40 were each supplied as sample. Then, a Tris solution of alkaline phosphatase-labeled antibodies, in which alkaline phosphatase is bound to human anti-influenza B virus nucleoprotein antibody 23G327, was supplied to the sample supply region of each of the devices. Further, a BCIP/NBT (nitro-blue tetrazolium chloride) solution was developed from the developer solution supply region. The result is indicated in Fig. 3. A blue spot was observed in the detection region in only the cases where the influenza B virus solution (B/Victoria/504/00) was used.

Moreover, when human anti-influenza B virus nucleoprotein antibody 23G327 was used as the antibody immobilized in the detection region and an alkaline phosphatase-labeled antibody, in which alkaline phosphatase is bound to mouse anti-influenza B virus nucleoprotein monoclonal antibody M2110171 (manufactured by Fitzgerald), was used as the labeled antibody, a blue spot was also observed in the detection region in only the cases where the influenza B virus solution (B/Victoria/504/00) was used.

When mouse anti-influenza B virus nucleoprotein antibody 41027 (manufactured by Capricorn) was used as the antibody immobilized in the detection region and an alkaline phosphatase-labeled antibody, in which alkaline phosphatase is bound to mouse anti-influenza B virus nucleoprotein monoclonal antibody M2110171 (manufactured by Fitzgerald), was used as the labeled antibody, a blue spot was similarly observed in the detection region in only the cases where the influenza B virus solution (B/Victoria/504/00) was used.

From the above, it was revealed that human anti-type B influenza virus nucleoprotein antibodies can be used to detect type B influenza viruses by immunochromatography.

### [Example 2]

### Preparation of gold colloid-labeled antibodies

Gold colloid-labeled antibodies were prepared using two types of human anti-influenza A virus nucleoprotein monoclonal antibodies [23G272 and 23G447] obtained in Example 4 described below and one type of mouse anti-influenza A virus nucleoprotein monoclonal antibody [M322211 (manufactured by Fitzgerald)] as labeled antibodies. Specifically, the PBS solutions of these antibodies were each replaced with Tris buffer to prepare Tris solutions of these antibodies, which were then mixed with 40 nm gold colloids (manufactured by BBInternational) and reacted for 15 minutes. After reaction and after blocking using a Tris solution containing 1% BSA, unbound monoclonal antibodies were removed by centrifugation, and the resultant was used as gold colloid-labeled antibodies.

### Detection of influenza A viruses

### Devices for detecting or quantifying influenza A viruses using gold colloid-labeled antibodies

Anti-influenza A virus nucleoprotein monoclonal antibodies [23 G312, 23G494, and M2110169 (manufactured by Fitzgerald)] were fixed as solid phase antibodies on a region of a filter paper (manufactured by Millipore) cut to 5 cm x 0.5 cm, and these were used as detection region. Specifically, PBS solutions of these anti-influenza A virus nucleoprotein monoclonal antibodies were each dropped and fixed to prepare detection regions. From the lower side (the sample supply region) of the filter paper, 0.1 mL of a solution, in which equal amounts of an influenza A virus solution (H1N1: A/Beijing/262/95) diluted using a solution for reaction containing Nonidet P-40 and a Tris solution of gold colloid-labeled antibodies prepared above were mixed, was developed. As a result, a prominent red spot was observed in the detection region with all antibody combinations (combinations of solid phase antibody and labeled antibody). When the time allowing visual observation of the spots was compared, in combinations which used human antibodies for the solid phase antibody and labeled antibody, the time was approximately twice faster for all combinations as compared to combinations which used mouse antibodies for the solid phase antibody and labeled antibody.

Further, to examine detection sensitivity, influenza A virus solutions (H1N1: A/Beijing/262/95 and H3N2: A/Panama/2007/99) prepared at various concentrations and gold colloid-labeled antibodies were developed using combinations of solid phase antibody and labeled antibody such as those shown in Table 1 and Table 2, and coloration of spots were visually observed.

**[Table 1]**

| | | | H1N1 (ng/mL) | | | |
|---|---|---|---|---|---|---|
| | SOLID PHASE ANTIBODY | LABELED ANTIBODY | 250 | 62.5 | 15.6 | 3.9 |
| MOUSE ANTIBODY | M2110169 | M322211 | ++ | ++ | + | - |
| HUMAN ANTIBODY | 23G312 | 23G272 | ++ | ++ | + | - |
| | 23G494 | 23G447 | ++ | ++ | ++ | - |

**[Table 2]**

| | | | H3N2(ng/mL) | | | |
|---|---|---|---|---|---|---|
| | SOLID PHASE ANTIBODY | LABELED ANTIBODY | 250 | 62.5 | 15.6 | 3.9 |
| MOUSE ANTIBODY | M2110169 | M322211 | ++ | + | - | - |
| HUMAN ANTIBODY | 23G312 | 23G272 | ++ | + | - | - |
| | 23G494 | 23G447 | ++ | ++ | + | - |

As a result, as indicated in Table 1 and Table 2, it was shown that, when human anti-influenza A virus nucleoprotein antibodies were used for both the solid-phase antibody and the labeled antibody, detection of comparable or higher sensitivity was possible with H1N1 and detection of four times or higher sensitivity was possible with H3N2, as compared with when commercially available mouse anti-influenza A virus nucleoprotein antibodies [M322211 and M2110169 (manufactured by Fitzgerald)] were used for both the solid-phase antibody and the labeled antibody.

Accordingly, it was revealed that human anti-influenza A virus nucleoprotein antibodies can be used to detect influenza A viruses using immunochromatography. In addition, it was revealed that influenza A viruses can be rapidly and highly sensitively detected when human anti-influenza A virus nucleoprotein antibodies are used as compared with when mouse anti-influenza A virus antibodies are used.

### [Example 3]

### Preparation of acridinium-labeled antibodies

Seven types of human anti-influenza virus nucleoprotein monoclonal antibodies [23G268, 23G272, 23G285, 23G312, 23G447, 23G494 (the forementioned: type A); and 23G327 (type B)] obtained in Example 4 described below and commercially available mouse anti-influenza virus nucleoproteins monoclonal antibodies were each reacted with acridinium ester (manufactured by Dojindo Laboratories), unreacted acridinium ester was removed using gel filtration, and acridinium-labeled anti-influenza virus nucleoprotein antibodies were obtained.

### Detection of influenza A viruses using monoclonal antibodies

Anti-influenza A virus nucleoprotein monoclonal antibodies diluted in PBS (10 µg/mL) were dispensed in each well at 50 µL per well of a 96 well microtiter plate (manufactured by Nunc) and left overnight at 4°C. After the solution in the wells was removed, 100 µL of a 1% BSA/PBS solution were dispensed and left to react for two hours at room temperature for blocking. 50 µL of three types of influenza virus solutions (H1N1: A/Beijing/262/95, H3N2: A/Panama/2007/99, and B: B/Victoria/504/00) diluted in PBS containing 0.5% Nonidet P-40 and 0.5% BSA were added to each well and left to react for one hour at room temperature. After washing with PBS containing 0.05% Tween 20, 50 µL of acridinium-labeled anti-influenza A virus nucleoprotein monoclonal antibody were added and left to react for one hour at room temperature. After washing with PBS containing 0.05% Tween 20, the amount of luminescence derived from acridinium was measured and the S/N ratio with the amount of luminescence for blank was determined. The result is indicated in Fig. 7. The result showed that the combination which uses 23G268 antibody as solid-phase antibody and 23G285 antibody as labeled antibody is most highly sensitive.

Moreover, the detection sensitivity was determined, considering an S/N ratio of 2.1 or above as positive. The result is shown in Table 3 [influenza A virus detection sensitivity (ng/mL)].

**[Table 3]**

| | SOLID PHASE ANTIBODY | LABELED ANTIBODY | DETECTION SENSITIVITY (ng/mL) | |
|---|---|---|---|---|
| | | | H1N1 | H3N2 |
| | HB-65 | M322211 | 50 | 50 |
| MOUSE ANTIBODY | M322211 | M2110169 | 12.5 | 6.25 |
| | M322211 | HB-65 | 12.5 | 12.5 |
| HUMAN ANTIBODY | 23G268 | 23G285 | 6.25 | 6.25 |

As apparent from Table 3, it was shown that, when 23G268 was used as the solid-phase antibody and 23G285 antibody was used as the labeled antibody, detection of comparable or higher sensitivity was possible with H3N2 and detection of two times or higher sensitivity was possible with H1N1, as compared with when commercially available mouse anti-influenza A virus nucleoprotein antibodies [M3322211, M2110169 (manufactured by Fitzgerald); and ATCC HB-65] were used for both the solid-phase antibody and the labeled antibody.

### Detection of influenza B viruses using monoclonal antibodies

Influenza B virus nucleoprotein monoclonal antibodies diluted in PBS (10 µg/mL) were dispensed in each well at 50 µL per well of a 96 well microtiter plate (manufactured by Nunc) and left overnight at 4°C. After the solution in the wells was removed, 100 µL of a 1 % BSA/PBS solution were dispensed and left to react for two hours at room temperature for blocking. 50 µL of three types of influenza virus solutions (H1N1: A/Beijing/262/95, H3N2: A/Panama/2007/99, and B: B/Victoria/504/00) diluted in PBS containing 0.5% Nonidet P-40 and 0.5% BSA were added to each well and left to react for one hour at room temperature. After washing with PBS containing 0.05% Tween 20, 50 µL of acridinium-labeled anti-influenza B virus nucleoprotein monoclonal antibody were added and left to react for one hour at room temperature. After washing with PBS containing 0.05% Tween 20, the amount of luminescence derived from acridinium was measured and the S/N ratio with the amount of luminescence for blank was determined. The result is indicated in Fig. 8. It was revealed that most highly sensitive detection was possible by using 23G237 as solid-phase antibody and M2110171 (manufactured by Fitzgerald) as labeled antibody.

Moreover, the detection sensitivity was determined, considering an S/N ratio of 2.1 or above as positive. The result is shown in Table 4 [influenza B virus detection sensitivity (ng/mL)].

**[Table 4]**

| | | LABEL | |
|---|---|---|---|
| | | M2110171 | 23G327 |
| SOLID PHASE | M2110171 | 125 | 31.3 |
| | 41027 | 62.5 | 15.6 |
| | 2/3 | 62.5 | 62.5 |
| | 23G327 | 31.3 | 125 |

It was shown from Table 4 that, by combining a commercially available mouse monoclonal antibody [M2110171 (manufactured by Fitzgerald); 41027 (manufactured by Capricorn); 2/3 (manufactured by Hytest)] and the human antibody 23G327 antibody of the present invention, detection of two to eight times higher sensitivity was possible as compared with when M2110171 was used for both the solid-phase antibody and the labeled antibody.

### Examination of the viral strain specificity

As indicated in Figs. 7 and 8, it was shown in the evaluation on detection of influenza viruses using monoclonal antibodies that detection was possible even when the same clone was used for the solid phase antibody and labeled antibody. Thus, evaluation on viral strain specificity was carried out using a solid phase antibody and labeled antibody from a same clone.

Influenza virus nucleoprotein monoclonal antibodies diluted in PBS (10 µg/mL) were dispensed in each well at 50 µL per well of a 96 well microtiter plate (manufactured by Nunc) and left overnight at 4°C. After the solution in the wells was removed, 100 µL of a 1% BSA/PBS solution were dispensed and left to react for two hours at room temperature for blocking. After various influenza virus strains [purchased from NIBSC (National Institute for Biological Standards and Control)] were diluted in PBS containing 0.5% Nonidet P-40 and 0.5% BSA to obtain 1 µg HA/mL, 50 µL were added to each well and left to react for one hour at room temperature. After washing with PBS containing 0.05% Tween 20, 50 µL of an acridinium-labeled anti influenza virus nucleoprotein monoclonal antibody, which is of the same clone as the solid-phase antibody, were added and left to react for one hour at room temperature. After washing with PBS containing 0.05% Tween 20, the amount of luminescence derived from acridinium was measured, and an S/N ratio with the amount of luminescence for blank of 2.1 or above was judged as positive (O) and an S/N ratio with the amount of luminescence for blank of less than 2.1 was judged as negative (X). Results with influenza virus A nucleoprotein monoclonal antibodies are indicated in Table 5-1 and Table 5-2. Results with influenza B virus nucleoprotein monoclonal antibodies are indicated in Table 6-1 and Table 6-2.

**[Table 5-1]**

| | | TYPE A NP ANTIBODY | | | | | | | | |
|---|---|---|---|---|---|---|---|---|---|---|
| | | HUMAN ANTIBODY | | | | | | MOUSE ANTIBODY | | |
| | VIRAL STRAIN | 23G268 | 23G272 | 23G285 | 23G312 | 23G447 | 23G494 | M322211 | M2110169 | HB-65 |
| | A/Beijing/262/95 | ○ | ○ | ○ | ○ | ○ | ○ | ○ | ○ | ○ |
| | A/Brazil/11/78 | ○ | ○ | ○ | ○ | ○ | ○ | ○ | ○ | ○ |
| | A/Chile/1/83 | ○ | ○ | ○ | ○ | ○ | ○ | ○ | ○ | ○ |
| | A/Johannesburg/82/96 | ○ | ○ | ○ | ○ | ○ | ○ | ○ | ○ | ○ |
| | A/New Caledonia/20/99 | ○ | ○ | ○ | ○ | ○ | ○ | ○ | ○ | ○ |
| H1N1 | A/New Caledonia/20/99(IVR-116) | ○ | ○ | ○ | ○ | ○ | ○ | ○ | ○ | ○ |
| | A/New Jersey/8/76 | ○ | ○ | ○ | ○ | ○ | ○ | ○ | ○ | ○ |
| | A/Solomon Islands/3/2006 | ○ | ○ | ○ | ○ | ○ | ○ | ○ | ○ | ○ |
| | A/Taiwan/1/86 | ○ | ○ | ○ | ○ | ○ | ○ | ○ | ○ | ○ |
| | A/Texas/36/91 | ○ | ○ | ○ | ○ | ○ | ○ | ○ | ○ | ○ |
| | A/USSR/92/77 | ○ | ○ | ○ | ○ | ○ | ○ | ○ | ○ | ○ |
| | A/Bangkok/1/79 | ○ | ○ | ○ | ○ | ○ | ○ | ○ | ○ | ○ |
| | A/Beijing/32/92 | ○ | ○ | ○ | ○ | ○ | ○ | ○ | ○ | ○ |
| | A/England/427/88 | ○ | ○ | ○ | ○ | ○ | ○ | ○ | ○ | ○ |
| | A/Leningrad/360/86 | ○ | ○ | ○ | ○ | ○ | ○ | ○ | ○ | ○ |
| | A/Mississippi/1/85 | ○ | ○ | ○ | ○ | ○ | ○ | ○ | ○ | ○ |
| | A/Guizhou/54/89 | ○ | ○ | ○ | ○ | ○ | ○ | ○ | ○ | ○ |
| | A/Johannesburg/33/94 | ○ | ○ | ○ | ○ | ○ | ○ | ○ | ○ | ○ |
| | A/New York/55/2004 | ○ | ○ | ○ | ○ | ○ | ○ | ○ | ○ | ○ |
| | A/OMS/5389/88 | ○ | ○ | ○ | ○ | ○ | ○ | ○ | ○ | ○ |
| H3N2 | A/Philippines/2/82 | ○ | ○ | ○ | ○ | ○ | ○ | ○ | ○ | ○ |
| | A/Shangdong/9/93 | ○ | ○ | ○ | ○ | ○ | ○ | ○ | ○ | ○ |
| | A/Shanghai/16/89 | ○ | ○ | ○ | ○ | ○ | ○ | ○ | ○ | ○ |
| | A/Shanghai/24/90 | ○ | ○ | ○ | ○ | ○ | ○ | ○ | ○ | ○ |
| | A/Sichuan/2/87 | ○ | ○ | ○ | ○ | ○ | ○ | ○ | ○ | ○ |
| | A/Sydney/5/97 | ○ | ○ | ○ | ○ | ○ | ○ | ○ | ○ | ○ |
| | A/Texas/1/77 | ○ | ○ | ○ | ○ | ○ | ○ | ○ | ○ | ○ |
| | A/Wisconsin/67/2005(NYMCX-161-B) | ○ | ○ | ○ | ○ | ○ | ○ | ○ | ○ | ○ |
| | A/Wisconsin/67/2005(NYMCX-161) | ○ | ○ | ○ | ○ | ○ | ○ | ○ | ○ | ○ |
| | A/Hiroshima/52/2005 | ○ | ○ | ○ | ○ | ○ | ○ | ○ | ○ | ○ |
| | A/Wyoming/03/03 | ○ | ○ | ○ | ○ | ○ | ○ | ○ | ○ | ○ |

**[Table 5-2]**

| | | | | | | | | | | |
|---|---|---|---|---|---|---|---|---|---|---|
| | A/Equine/Kentucky/1/81 | ○ | ○ | ○ | ○ | ○ | ○ | × | ○ | ○ |
| H3N8 | A/Equine/Miami/63 | ○ | ○ | ○ | ○ | ○ | ○ | × | ○ | ○ |
| | A/Equine/Newmarket/1/93 | ○ | ○ | ○ | ○ | ○ | ○ | × | ○ | ○ |
| | A/Equine/Newmarket/2/93 | × | ○ | ○ | × | × | × | × | ○ | ○ |
| H5N1 | A/Vietnam/1194/04 | ○ | ○ | ○ | ○ | ○ | ○ | ○ | ○ | ○ |
| | B/Malaysia/2506/2004 | × | × | × | × | × | × | × | × | × |
| | B/Jiangsu/10/2003 | × | × | × | × | × | × | × | × | × |
| | B/Ann Arbor/1/86 | × | × | × | × | × | × | × | × | × |
| | B/Beijing/7/87 | × | × | × | × | × | × | × | × | × |
| | B/Guangdong/120/2000 | × | × | × | × | × | × | × | × | × |
| | B/Harbin/7/94 | × | × | × | × | × | × | × | × | × |
| | B/Hong Kong/8/73 | × | × | × | × | × | × | × | × | × |
| B | B/Johannesburg/5/99 | × | × | × | × | × | × | × | × | × |
| | B/Norway/1/84 | × | × | × | × | × | × | × | × | × |
| | B/Panama/45/90 | × | × | × | × | × | × | × | × | × |
| | B/Shangdong/7/97 | × | × | × | × | × | × | × | × | × |
| | B/Singapore/222/79 | × | × | × | × | × | × | × | × | × |
| | B/Victoria/2/87 | × | × | × | × | × | × | × | × | × |
| | B/Yamagata/16/88 | × | × | × | × | × | × | ○ | ○ | ○ |
| | B/Yamanashi/166/98 | × | × | × | × | × | × | × | × | × |

| | | | | | | | | | | |
|---|---|---|---|---|---|---|---|---|---|---|
| (Table 5-2 is a continuation of Table 5-1.) | | | | | | | | | | |

**[Table 6-1]**

| | | TYPE B NP ANTIBODY | | | |
|---|---|---|---|---|---|
| | | HUMAN ANTIBODY | MOUSE ANTIBODY | | |
| | VIRAL STRAIN | 23G327 | M2110171 | 41027 | 2/3 |
| | A/Bejjng/262/95 | × | × | × | × |
| | A/Brazil/11/78 | × | × | × | × |
| | A/Chile/1/83 | × | × | × | × |
| | A/Johannesburg/82/96 | × | × | × | × |
| | A/New Caledonia/20/99 | × | × | × | × |
| H1N1 | A/New Caledonia/20/99(IVR-116) | × | × | × | × |
| | A/New Jersey/8/76 | × | × | × | × |
| | A/Solomon Islands/3/2006 | × | × | × | × |
| | A/Taiwan/1/86 | × | × | × | × |
| | A/Texas/36/91 | × | × | × | × |
| | A/USSR/92/77 | × | × | × | × |
| | A/Bangkok/1/79 | × | × | × | × |
| | A/Beijing/32/92 | × | × | × | × |
| | A/England/427/88 | × | × | × | × |
| | A/Leningrad/360/86 | × | × | × | × |
| | A/Mississippi/1/85 | × | × | × | × |
| | A/Guizhou/54/89 | × | × | × | × |
| | A/Johannesburg/33/94 | × | × | × | × |
| | A/New York/55/2004 | × | × | × | × |
| | A/OMS/5389/88 | × | × | × | × |
| | A/Philippines/2/82 | × | × | × | × |
| H3N2 | A/Shangdong/9/93 | × | × | × | × |
| | A/Shanghai/16/89 | × | × | × | × |
| | A/Shanghai/24/90 | × | × | × | × |
| | A/Sichuan/2/87 | × | × | × | × |
| | A/Sydney/5/97 | × | × | × | × |
| | A/Texas/1/77 | × | × | × | × |
| | A/Wisconsin/67/2005(NYMCX-161-B) | × | × | × | × |
| | A/Wisconsin/67/2005(NYMCX-161) | × | × | × | × |
| | A/Hiroshima/52/2005 | × | × | × | × |
| | A/Wyoming/03/03 | × | × | × | × |

**[Table 6-2]**

| | | | | | |
|---|---|---|---|---|---|
| | A/Equine/Kentucky/1/81 | × | × | × | × |
| H3N8 | A/Equine/Miami/63 | × | × | × | × |
| | A/Equine/Newmarket/1/93 | × | × | × | × |
| | A/Equine/Newmarket/2/93 | × | × | × | × |
| H5N1 | A/Vietnam/1194/04 | × | × | × | × |
| | B/Malaysia/2506/2004 | ○ | ○ | ○ | × |
| | B/Jiangsu/10/2003 | ○ | ○ | ○ | × |
| | B/AnnArbor/1/86 | ○ | ○ | ○ | × |
| | B/Beijing/7/87 | ○ | ○ | ○ | × |
| | B/Guangdong/120/2000 | ○ | ○ | ○ | ○ |
| | B/Harbin/7/94 | ○ | ○ | ○ | ○ |
| | B/Hong Kong/8/73 | ○ | ○ | ○ | ○ |
| B | B/Johannesburg/5/99 | ○ | ○ | ○ | ○ |
| | B/Norway/1/84 | ○ | ○ | ○ | ○ |
| | B/Panama/45/90 | ○ | ○ | ○ | ○ |
| | B/Shangdong/7/97 | ○ | ○ | ○ | ○ |
| | B/Singapore/222/79 | ○ | ○ | ○ | ○ |
| | B/Victoria/2/87 | ○ | ○ | ○ | × |
| | B/Yamagata/16/88 | ○ | ○ | ○ | ○ |
| | B/Yamanashi/166/98 | ○ | ○ | ○ | ○ |

| | | | | | |
|---|---|---|---|---|---|
| (Table 6-2 is a continuation of Table 6-1.) | | | | | |

From these results, it was revealed that, the human anti-influenza A virus nucleoprotein antibodies of the present invention do not react with a part of H3N8-type viruses which are equine influenza A virus strains; however, except for these viruses, the antibodies react with all the Human influenza A virus strains and the H5N1-type viruses which are avian influenza A virus strains, and do not react with type B virus strains. In contrast, it was revealed that commercially available mouse influenza A virus nucleoprotein antibodies react with a part of the type B virus strains.

Moreover, it was revealed that the human influenza B virus nucleoprotein antibody (23 G327) of the present invention reacts with none of the influenza A virus strains and specifically reacts with all of the influenza B virus strains. In contrast, it was revealed that the commercially available mouse influenza B virus nucleoprotein antibody 2/3 does not react with a part of the influenza B virus strains.

From the above, it was revealed that the human anti-influenza virus nucleoprotein antibodies of the present invention show high specificity to influenza virus strains as compared with commercially available mouse anti-influenza virus nucleoprotein antibodies.

### - Evaluation on the performance of the human anti-influenza virus nucleoprotein monoclonal antibodies

### Preparation of sensor chips

A sensor chip CM5 was set in Biacore 2000 and HBS-EP (manufactured by GE Healthcare Biosciences) was run as running buffer at a flow speed of 10 µL/minute. An activation solution (a mixture solution of equal amounts of 0.4 mol/L EDC and 0.1 mol/L NHS; both manufactured by GE Healthcare Biosciences) was run for seven minutes and allow to contact. Then, goat anti-human IgG or goat anti-mouse IgG (manufactured by Thermo Scientific) prepared at 10 µg/mL using a pH5.0 sodium acetate buffer was run for seven minutes and fixed on the CM5 surface. Then, to mask the remaining activated carboxylic acid, ethanolamine (1 mol/L, pH8.5) was run for seven minutes. Then, glycine-hydrochloric acid buffer (0.2 mol/L, pH 2.2) was run for one minute for washing. This procedure was performed in flow cell 2, and with regard to flow cell 1, activation with EDC and NHS was similarly carried out and masking was carried out with ethanolamine alone without performing contact with IgG..

### Detection of influenza viruses using monoclonal antibodies

By running anti-influenza A virus nucleoprotein monoclonal antibodies prepared at 10 µg/mL on the sensor chip prepared above at a flow speed of 5 µL/minute for ten minutes, the antibodies were captured on the sensor chip. At this time, the value calculated by subtracting the signal obtained in flow cell 1 (RU) from the signal obtained in flow cell 2 (RU) was taken as the amount of bound antibodies. Then, three types of influenza virus (H1N1: A/Beijing/262/95, H3N2: A/Panama/2007/99, and B: B/Victoria/504/00) solutions prepared at 10 µg/mL with HBS-EP were reacted at a flow speed of 5 µL/minute for five minutes, and HBS-EP (0.01 mol/L HEPES, 0.15 mol/L NaCl, 3 mmol/L EDTA, and 0.005% surfactant P20) buffer was further run at a flow speed of 5 µL/minute for five minutes. At this time, the value calculated by subtracting the aforementioned amount of bound antibodies from the value obtained by subtracting the signal obtained in flow cell 1 (RU) from the signal obtained in flow cell 2 (RU) was taken as the amount of bound antigens. After reaction, glycine-hydrochloric acid buffer (0.2 mol/L, pH 1.7) was run for one minute to regenerate the sensor chip. A sensorgram showing the interactions between a commercially available anti-influenza A virus nucleoprotein antibody (M322211) and three types of influenza virus (H1N1: A/Beijing/262/95, H3N2: A/Panama/2007/99, and B: B/Victoria/504/00) solutions is shown in Fig. 9-a. A sensorgram showing the interactions between a human anti-influenza A virus nucleoprotein antibody (23G272) obtained in the present invention and three types of influenza virus (H1N1: A/Beijing/262/95, H3N2: A/Panama/2007/99, and B: B/Victoria/504/00) solutions is shown in Fig. 9-b. Antigen dissociation reaction was not observed with the human antibody 23G272 of the present invention, revealing that the antibody is an antibody with strong affinity.

Moreover, similarly as for 23G272, no antigen dissociation reaction was observed with all the other human anti-influenza A virus nucleoprotein antibodies (23G268, 23G285, 23G312, 23G447, and 23G494) shown in Fig. 9-c, revealing that they are antibodies with strong affinity. At this time, the value obtained by subtracting the signal obtained in flow cell 1 (RU) from the signal obtained in flow cell 2 (RU) was calculated, and the ratio with the previously-calculated amount of bound antibodies was taken as the amount of bound antigens. The result is indicated in Fig. 9-c. From this result, the 23G268 antibody, 23G272 antibody, 23G285 antibody, 23G312 antibody, 23G447 antibody, and 23G494 antibody were shown to have a comparable or higher reactivity as with commercially available mouse anti-influenza A virus nucleoprotein antibodies. The 23G272 antibody showed twice or higher reactivity as compared with commercially available mouse anti-influenza A virus nucleoprotein antibodies, showing that the antibody has a property of a very fast binding reaction speed. The reactivity of the human anti-influenza B virus nucleoprotein antibody (23G327) was approximately 0.8 times that of one (2/3) of the commercially available mouse anti-influenza B virus nucleoprotein antibodies, so that the reactivity was somewhat inferior as compared with that of antibody 2/3. However, as shown in Table 6-2, antibody 2/3 does not react with a part of the influenza B virus strains and does not have reactivity to broad spectrum of influenza B virus strains. When three types of antibodies (23G327, M2110171, and 41027) which have reactivity to broad spectrum off influenza B virus strains were compared, 23G327 was the antibody with the highest reactivity, the reactivity being approximately 1.2 times that of M2110171 and approximately two times that of 41027. Accordingly, by judging overall from the viewpoints of the reactivity to the influenza B virus strains and broadness in the reactions with influenza B virus strains, the antibody 23G327 of the present invention was found to the most superior.

Further, 23G272 was fixed onto flow cell 2, M2110169 was fixed onto flow cell 3, and HB-65 was fixed onto flow cell 4 by a method similar to the aforementioned method for preparing a sensor chip. The fixed amount was 1519 RU for 23G272, 1773 RU for M2110169, and 1734 RU for HB-65. A/Beijing/262/95 adjusted to 10 µg/mL was reacted at a flow speed of 5 µL/minute for two minutes to the prepared sensor chips. The result is indicated in Fig. 10. 23G272 was found to have an amount of bound antigens often times or so as compared with HB-65. Moreover, M2110169 lost activity by the glycine-hydrochloric acid buffer and did not show any reaction with the viruses.

### [Example 4]

### [Preparation of influenza virus nucleoprotein]

Influenza virus nucleoprotein gene was synthesized based on the disclosed sequence information (A/Puerto Rio/8/34, or B/Ann Arbor/1/86), and was integrated into the pSUPEX vector. *Escherichia coli* (NY49 strain) was transformed using this vector, so as to have the transformant express an influenza virus nucleoprotein. The obtained influenza virus nucleoprotein was purified by affinity chromatography using an antibody against the influenza virus nucleoprotein.

### [Preparation of B lymphocytes]

A commercially available influenza HA vaccine was used to immunize human volunteers, and lymphocytes were prepared from the peripheral blood on the 9th day after immunization or one month after immunization. More specifically, human lymphocytes were separated from the peripheral blood by centrifugation using the Lymphosepar I solution (manufactured by immuno-Biological Laboratories Co., Ltd.), and then the B lymphocyte fraction was separated and purified by removing the non-B-lymphocyte fraction cells from the lymphocyte fraction using AutoMACS (Miltenyi Biotec, Bergisch Gladbac, Germany).

### [Loading of fluorescent dye onto B lymphocytes]

The prepared 2 x 10⁶ B lymphocytes were suspended in 1 µmol/L Fluo 4-AM (calcium-dependent fluorescent dye, Invitrogen)/BSA-containing RPMI buffer (loading buffer) [RPMI1640, 3 mg/mL BSA, 25 mmol/L HEPES (pH7.4)], and were incubated while slowly shaking at room temperature for 40 to 60 minutes. While Fluo4-AM is cell membrane permeable, once it is taken into the cell and the AM group is removed by an esterase, Fluo4-AM stays in the cytoplasm. The cells were washed using the loading buffer to remove the excess Fluo4-AM that was not introduced into the cells, and then suspended in RPMI1640/10% FCS solution. In all of the following experiments, RPMI1640 not containing Phenol Red was used. Fluo4-AM is a fluorescent dye whose fluorescence intensity increases upon calcium binding, and is used for detection of activation of a B lymphocyte by an antigen.

### [Microwell array chips]

Microwell array chips are produced using silicone (manufactured by Toyama Industrial Technology Center), and microwells having a diameter of 10 µm and a depth of 14 µm are arranged vertically and horizontally at pitches (distance between the centers of wells) of 25 µm. A single cluster is formed by 30 x 30 microwells (900 wells), and a chip in which ten of these clusters are aligned vertically and five of these clusters are aligned horizontally were used.

### [Seeding of lymphocytes to microwell array chips]

Microwell array chips were soaked in Hanks' buffered solution (HBSS), and air in the wells was removed through degassing by reduced pressure, and the microwells were filled with the solution. The microwell chip surface was subjected to blocking treatment with 0.2% LIPIDURE-BL-B03 (manufactured by NOF Corporation) for 10 to 15 minutes, then excess buffer was removed, the above-mentioned cell suspension solution was added, and this was left to stand for 10 minutes. Cells that did not enter the microwells on the chip were washed off using HBSS. Since the diameter of the lymphocyte is approximately 8 µm and the diameter of the microwells used is 10 µm, one lymphocyte enters a single microwell. A cover glass was placed on the above-mentioned seal, and the space between the chip and the coverglass was filled with HBSS.

### [Detection of recombinant nucleoprotein-specific B lymphocytes using microwell array chips]

B lymphocyte-seeded microwell array chip was loaded into a microchip CCD imager (Microchip VIEW 1100; manufactured by NanoSystem Solutions, Inc.), and the change over time of the fluorescence intensity of Fluo-4 prior to antigen stimulation was measured at 10-second intervals for 100 seconds. The measurement was stopped, the chip was taken out from the scanner, then HBSS between the chip and the coverglass was removed, and recombinant nucleoprotein dissolved in HBSS (10 to 100 µg/mL) was added in its place. The microwell array chip was immediately placed back into the measurement site of the microchip CCD imager, and the change over time of the fluorescence intensity of Fluo-4 in the cell after antigen addition was scanned at 10 second intervals and the data was saved. The degree of correlation of each of the changes over time of the intracellular calcium concentration with the typical pattern of the change over time of the intracellular calcium concentration observed when an antigen binds to a specific antibody-expressing cell was analyzed using a software, and cells showing an antigen-specific intracellular calcium response were identified.

### [Collection of B lymphocytes from microwells]

The detected B lymphocytes whose intracellular calcium increased in response to the recombinant nucleoprotein were collected using a micromanipulator under a fluorescence microscope. More specifically, first, HBSS between the coverglass and the chip was removed, and by placing air between the coverglass and chip, the coverglass was removed.
RPMI1640/10% FCS solution was added to the chip so that the chip does not become dry, the cells of interest were collected using a micromanipulator by observing the fluorescence of Fluo-4 in the cells under a fluorescence microscope.

### [Synthesis of antibody gene cDNA from B lymphocytes]

The collected B lymphocytes were transferred to a PCR tube containing in advance 2.5 µL of a reverse transcription reaction solution [1x 1^{st} strand buffer (Invitrogen, provided with SuperScript III), 0.25 pmol each GSP RT primer (Cg-RT, Cl-RT, and Ck-RT), 0.1 mmol/L each dNTP, 10 mmol/L DTT, 1 mg/mL Bovine Serum Albumin (BSA), 0.25% NP-40, 1.6 U RNaseOUT (Invitrogen), 0.2x Ampdirect Plus (Shimadzu Corporation), 16 U SuperScript III (Invitrogen)]. cDNA was synthesized from mRNA by carrying out the reaction at 25°C for ten minutes; 30°C for five seconds; 35°C for five seconds; 40°C for five seconds; 45°C for five seconds; 50°C for five seconds; and 55°C for one hour.

### [G-tailing reaction of the cDNA-3' end]

To 2.5 µL of the synthesized cDNA solution, 7.5 U of Terminal Deoxynucleotidyl Transferase (TdT) enzyme was added, and this was allowed to react in the presence of 10 mmol/L Tris-HCl (pH7.5), 10 mmol/L MgCl₂, 1 mM DTT, 2 mmol/L dGTP at 37°C for one hour.

### [First round of amplification of the antibody gene]

To 10 µL of a solution of cDNA after completion of the G-tailing reaction, 0.25 µL of Herculase II fusion DNA polymerase enzyme was added, and this was reacted in the presence of 1x Herculase II fusion DNA polymerase buffer, 0.25 mmol/L each dNTP, 25 pmol Oligo(dC) adaptor, and 8% DMSO, at 98°C fur two minutes; then (98°C for 20 seconds; 60°C for 20 seconds; and 72°C for 30 seconds) x 23 cycles; and 72°C for three minutes. From this PER reaction, the DNA from the oligo(dC) adaptor sequence to the constant region of the antibody gene is amplified.

### [Second round of amplification of the antibody gene]

Next, a second PCR reaction was carried out, and the V region cDNAs of the H chains and L chains of the obtained cells were amplified using separate tubes. Specifically, 3.3 µL of the PCR reaction solution obtained in the first round was used as template, 0.05 µL of Herculase II fusion DNA polymerase enzyme was added and let to react in the presence of 1x Herculase II fusion DNA polymerase buffer, 0.25 mmol/L each dNTP, 2.5 pmol AP1 primer, 2.5 pmol Cg-1st primer or Cl-1st & Ck-1 primer, and 8% DMSO at 98°C for two minutes; then (98°C for 20 seconds; 55°C for 20 seconds; 72°C for 30 seconds) x 30 cycles, and 72°C for three minutes.

### [Third round of amplification of the antibody gene]

Since two rounds of PCR does not lead to sufficient amplification of cDNA, a third round of amplification was carried out. 0.02 µL of the PCR reaction solution obtained in the second round was used as template, 0.05 µL of TaKaRa LA Taq polymerase enzyme was added and the reaction was carried out in the presence of 1x GC buffer, 0.2 mmol/L each dNTP, 3 pmol of AP2 primer, 3 pmol of Cg-nest primer or Cl-nest & Ck-nest primer, at 94°C for three minutes; (94°C for 20 seconds; 55°C for 20 seconds; 72°C for 90 seconds) x 30 cycles; and 72°C for three minutes. The third round of PCR amplifies the cDNA sequence from the variable region to the constant region of the antibody gene. The sequences of the primers used are shown in Table 7 (primers used in single cell 5'-RACE).

**[Table 7]**

| NAME OF SEQUENCE | SEQ ID NO | NUCLEOTIDE SEQUENCE |
|---|---|---|
| Oligo(dC) adaptor | SEQ ID NO: 29 | ACAGCAGGTCAGTCAAGCAGTAGCAGCAGTTCGATAA GCGGCCGCCATGGACCCCCCCCCCCCCC(AGT)(ACGT) |
| AP1 | SEQ ID NO: 30 | ACAGCAGGTCAGTCAAGCAGTA |
| AP2 | SEQ ID NO: 31 | AGCAGTAGCAGCAGTTCGATAA |
| Cg-RT | SEQ ID NO: 32 | AGGTGTGCACGCCGCTGGTC |
| Cg-1st | SEQ ID NO: 33 | CGCCTGAGTTCCACGACACC |
| Cg-nest | SEQ ID NO: 34 | TCGGGGAAGTAGTCCTTGAC |
| Cl-RT | SEQ ID NO: 35 | ACAC(CT)AGTGTGGCCTTGTT |
| Cl-1st | SEQ ID NO: 36 | GCTTG(AG)AGCTCCTCAGAGG |
| Cl-nest | SEQ ID NO: 37 | GGG(CT)GGGMCAGAGTGACC |
| Ck-RT | SEQ ID NO: 38 | GTTATTCAGCAGGCACACAA |
| Ck-1st | SEQ ID NO: 39 | GAGGCAGTTCCAGATTTCAA |
| Ck-nest | SEQ ID NO: 40 | GGGAAGATGAAGRCAGATGGT |

In the nucleotide sequences of the above Table 7, the parts in parentheses ( ) mean that one of the nucleotides in the parentheses is selected.

### [Determination of the nucleotide sequence of the amplified antibody gene]

PCR products were analyzed using an agarose gel, purified, and inserted into pGEM-T Easy vector (Promega), the nucleotide sequences of the antibody genes were determined, and they were confirmed to be translated into proteins. The nucleotide sequences and amino acid sequences of the cDNAs of the H-chain V regions are shown in SEQ ID NOs: 1 to 7 and SEQ ID NOs: 8 to 14, respectively. Furthermore, the nucleotide sequences and amino acid sequences of the cDNAs of the L-chain V regions are shown in SEQ ID NOs: 15 to 21 and SEQ ID NOs: 22 to 28, respectively.

### [Production of antibody proteins and analysis of antigen binding ability]

The H-chain and L-chain variable region genes of the antibodies amplified by the RT-PCR method were incorporated into a vector for antibody protein expression (Figs. 4-a, 4-b, and 4-c). More specifically, the gene fragment of the H-chain variable region of the amplified antibody was inserted into the Vγ portion of the pMXv6-hIgγ vector, and the gene fragment of the L-chain variable region of the amplified antibody was inserted into the Vκ portion of pMXv6-hIgκ or the Vλ portion of the pMXv6-hIgλ vector using restriction enzymes. To express the antibody proteins from the produced H-chain and L-chain expression vectors, both expression vectors were simultaneously gene transferred into human embryonic kidney-derived 293T cells. Gene transfer was performed according to conventional methods using Lipofectamine 2000 (Invitrogen). The cell supernatant was collected 5 to 7 days later. The binding ability of the antibody in this culture supernatant to the recombinant nucleoproteins were measured by the ELISA method using 96-well plates coated with the recombinant nucleoproteins. Measurements by ELISA were carried out as follows. Specifically, a recombinant nucleoprotein diluted with PBS (10 µg/mL) was dispensed into a 96-well plate at 50 µL per well, and adsorption was carried out by allowing this to stand at 4°C overnight. To remove non-specific binding, 3% BSA-supplemented PBS was dispensed at 150 µL per well and left to react at room temperature for one hour for blocking. As wells for negative control, wells without adsorption of an antigen which were subjected to blocking were also prepared. Thereafter, the culture supernatant of the above-mentioned 293T cells was dispensed at 50 µL per well, and this was allowed to react for two hours at room temperature. The wells were washed with TBS-T, horseradish peroxidase (HRP)-labeled anti-human immunoglobulin diluted 1/2000 times was dispensed at 50 µL per well, and this was allowed to react at room temperature for 1.5 hours. The wells were washed with TBS-T, 0.4 mg/mL *o*-phenylenediamine (OPD; HRP substrate) dissolved in a citrate-phosphate buffer (12 mmol/L citric acid, 26 mmol/L Na₂HPO₄, pH5.0) was dispensed at 50 µL per well, this was allowed to react at room temperature for 15 minutes, and then the absorbance at 492 nm was measured on a microplate reader.

Furthermore, to check the binding specificity of the monoclonal antibodies of the present invention to the antigens, the soluble recombinant nucleoprotein and the culture supernatant were pre-incubated when adding the antibody-containing cell culture supernatant to the wells. This mixed solution was added to the wells to examine whether the binding of the antibody to the recombinant nucleoprotein adsorbed onto the wells is competitively inhibited by a soluble recombinant nucleoprotein. The results are shown in Figs. 5-a and 5-b.

As shown in Figs. 5-a and 5-b, the produced 23G268 antibody, 23G272 antibody, 23G285 antibody, 23G312 antibody, 23G447 antibody, and 23G494 antibody bound specifically to the recombinant influenza A vitus nucleoprotein and did not bind to the recombinant influenza B virus nucleoprotein. Furthermore, to examine the specificity of binding, the six types of antibodies mentioned above were mixed with the soluble recombinant influenza A virus nucleoprotein and the mixtures were added to the wells in which the recombinant influenza A virus nucleoprotein is bound, and ELISA was performed similarly. As a result, binding of the antibodies to the recombinant influenza A virus nucleoprotein bound to the wells was observed to be competitively inhibited in a dose-dependent manner by the soluble recombinant influenza A virus nucleoprotein. This result indicated that the 23G268 antibody, 23G272 antibody, 23G285 antibody, 23G312 antibody, 23G447 antibody, and 23G494 antibody bind specifically to the recombinant A-type nucleoprotein.

On the other hand, the produced 23G327 antibody bound specifically to the recombinant influenza B virus nucleoproteins and did not bind to the recombinant influenza A virus nucleoprotein. Furthermore, to examine the specificity of binding, a mixture of the 23G327 antibody and the soluble recombinant influenza B virus nucleoprotein was added to the wells in which the recombinant influenza B virus nucleoprotein is fixed, and ELISA was performed similarly. As a result, binding of the 23G327 antibody to the recombinant influenza B virus nucleoprotein fixed to the wells was observed to be competitively inhibited in a dose-dependent manner by the soluble recombinant influenza B virus nucleoprotein. This result indicated that the 23G327 antibody binds specifically to the recombinant influenza B virus nucleoprotein.

### Examination of virus specificity by direct ELISA

Various types of influenza virus nucleoprotein antigens diluted in PBS (10 µg/mL) were dispensed at 50 µL per well into a 96-well microtiter plate (manufactured by Nunc), and left to stand at 4°C overnight. After removing the solution in the wells, 1% BSA/PBS solution was dispensed at 100 µL each, and blocking was accomplished by allowing this to react at room temperature for two hours.

After removing the solution in the wells, the culture supernatant obtained as described above or human IgG1 was diluted using 0.1% BSA/PBS solution and added at 50 µL per well, and then this was allowed to react at room temperature for one hour. After washing with PBS containing 0.05% Tween 20, HRP-labeled rabbit anti-human IgG antibodies were added, and this was allowed to react at room temperature for one hour. After washing with PBS containing 0.05% Tween20, 50 µL of TMB chromogen solution was added, and this was allowed to react at room temperature for 30 minutes. 50 µL of a reaction stopping solution (1 mol/L H₂SO₄) was added at the end, and the absorbance at 450 nm was measured on a microplate reader. The results are shown in Fig. 6. These results indicated that the 23G268 antibody, 23G272 antibody, 23G285 antibody, 23G312 antibody, 23G447 antibody, and 23G494 antibody bind specifically to the influenza A virus, and the 23G327 antibody reacts specifically to the influenza B virus.

### Industrial Applicability

The present invention provides devices and kits for diagnosis of influenza virus infections, as well as human anti-influenza virus nucleoprotein antibodies used for these devices and kits.

## Claims

1. A device for detecting or quantifying an influenza virus in a sample, which comprises a detection region in which a human anti-influenza virus nucleoprotein antibody is immobilized onto a support, a sample supply region, and a sample-migrating region.

2. A device for detecting or quantifying an influenza virus in a sample, which comprises a detection region in which an anti-influenza virus nucleoprotein antibody (antibody 1) is immobilized onto a support, a sample supply region, and a sample-migrating region, wherein a labeled antibody in which a label is bound to an anti-influenza virus nucleoprotein antibody (antibody 2) is supplied from the sample supply region, and wherein at least one of antibody 1 and antibody 2 is a human anti-influenza virus nucleoprotein antibody.

3. A device for detecting or quantifying an influenza virus in a sample, which comprises a detection region in which an anti-influenza virus nucleoprotein antibody (antibody 1) is immobilized onto a support, a labeled reagent region in which a labeled antibody in which a label is bound to an anti-influenza virus nucleoprotein antibody (antibody 2) is retained on a support in a manner to allow migration, a sample supply region, and a sample-migrating region, wherein at least one of antibody 1 and antibody 2 is a human anti-influenza virus nucleoprotein antibody.

4. The device of claim 2 or 3, which further comprises at least one region selected from the group consisting of a developer solution supply region, an excess liquid absorbing region, and a sample supply confirming region.

5. The device of any one of claims 1 to 4, wherein the human anti-influenza virus nucleoprotein antibody is a human anti-influenza A virus nucleoprotein antibody.

6. The device of claim 5, wherein the human anti-influenza A virus nucleoprotein antibody is a human anti-influenza A virus nucleoprotein monoclonal antibody which specifically reacts with an influenza A virus nucleoprotein but does not react with an influenza B virus nucleoprotein.

7. The device of claim 6, wherein the amino acid sequence of the heavy chain variable region of the human anti-influenza A virus nucleoprotein monoclonal antibody comprises the amino acid sequence of any one of SEQ ID NOs: 8 to 13 and the amino acid sequence of the light chain variable region of the human anti-influenza A virus nucleoprotein monoclonal antibody comprises the amino acid sequence of any one of SEQ ID NOs: 22 to 27.

8. The device of any one of claims 1 to 4, wherein the human anti-influenza virus nucleoprotein antibody is a human anti-influenza B virus nucleoprotein antibody.

9. The device of claim 8, wherein the human anti-influenza B virus nucleoprotein antibody is a human anti-influenza B virus nucleoprotein monoclonal antibody which specifically reacts with an influenza B virus nucleoprotein but does not react with an influenza A virus nucleoprotein.

10. The device of claim 9, wherein the amino acid sequence of the heavy chain variable region of the human anti-influenza B virus nucleoprotein monoclonal antibody comprises the amino acid sequence of SEQ ID NO: 14 and the amino acid sequence of the light chain variable region of the human anti-influenza B virus nucleoprotein monoclonal antibody comprises the amino acid sequence of SEQ ID NO: 28.

11. A kit for detecting or quantifying an influenza virus, which comprises a solid phase in which a human anti-influenza virus nucleoprotein antibody is fixed onto a carrier.

12. A kit for detecting or quantifying an influenza virus, which comprise a solid phase in which an anti-influenza virus nucleoprotein antibody (antibody 1) is fixed onto a carrier and a reagent comprising an anti-influenza virus nucleoprotein antibody (antibody 2), and wherein at least one of antibody 1 and antibody 2 is a human anti-influenza virus nucleoprotein antibody.

13. A kit for detecting or quantifying an influenza virus, which comprises a solid phase in which an anti-influenza virus nucleoprotein antibody (antibody 1) is fixed onto a carrier and a reagent comprising a labeled antibody in which a label is bound to an anti-influenza virus nucleoprotein antibody (antibody 2), and wherein at least one of antibody 1 and antibody 2 is a human anti-influenza virus nucleoprotein antibody.

14. A kit for detecting or quantifying an influenza virus, which comprises a solid phase in which a human anti-influenza virus nucleoprotein antibody is fixed onto a carrier and a reagent comprising a labeled antigen analog in which a label is bound to an influenza virus nucleoprotein antigen analog.

15. A kit for detecting or quantifying an influenza virus, which comprises a solid phase in which an influenza virus nucleoprotein antigen analog is fixed onto a carrier and a reagent comprising a labeled antibody in which a label is bound to a human anti-influenza virus nucleoprotein antibody.

16. The kit of any one of claims 11 to 15, wherein the human anti-influenza virus nucleoprotein antibody is a human anti-influenza A virus nucleoprotein antibody.

17. The kit of claim 16, wherein the human anti-influenza A virus nucleoprotein antibody is a human anti-influenza A virus nucleoprotein monoclonal antibody which specifically reacts with an influenza A virus nucleoprotein but does not react with an influenza B virus nucleoprotein.

18. The kit of claim 17, wherein the amino acid sequence of the heavy chain variable region of the human anti-influenza A virus nucleoprotein monoclonal antibody comprises the amino acid sequence of any one of SEQ ID NOs: 8 to 13 and the amino acid sequence of the light chain variable region of the human anti-influenza A virus nucleoprotein monoclonal antibody comprises the amino acid sequence of any one of SEQ ID NOs: 22 to 27.

19. The kit of any one of claims 11 to 15, wherein the human anti-influenza virus nucleoprotein antibody is a human anti-influenza B virus nucleoprotein antibody.

20. The kit of claim 19, wherein the human anti-influenza B virus nucleoprotein antibody is a Human anti-influenza B virus nucleoprotein monoclonal antibody which reacts specifically with an influenza B virus nucleoprotein but does not react with an influenza A virus nucleoprotein.

21. The kit of claim 20, wherein the amino acid sequence of the heavy chain variable region of the human anti-influenza B virus nucleoprotein monoclonal antibody comprises the amino acid sequence of SEQ ID NO: 14 and the amino acid sequence of the light chain variable region of the human anti-influenza B virus nucleoprotein monoclonal antibody comprises the amino acid sequence of SEQ ID NO: 28.

22. A method for detecting or quantifying an influenza virus, comprising the steps of:
(1) reacting a sample with a human anti-influenza virus nucleoprotein antibody fixed onto a carrier; and
(2) measuring a physical change produced in step (1).

23. A method for detecting or quantifying an influenza virus, comprising the steps of:
(1) reacting a sample with an anti-influenza virus nucleoprotein antibody (antibody 1) fixed onto a carrier to form an antibody 1/influenza virus nucleoprotein complex on the carrier;
(2) reacting the complex formed on the carrier in step (1) with an anti-influenza virus nucleoprotein antibody (antibody 2); and
(3) measuring a physical change produced in step (2);
wherein at least one of antibody 1 and antibody 2 is a human anti-influenza virus nucleoprotein antibody.

24. The method of claim 22 or 23, wherein the physical change is turbidity change or mass change.

25. A method for detecting or quantifying an influenza virus, comprising the steps of:
(1) reacting a sample with an anti-influenza virus nucleoprotein antibody (antibody 1) fixed onto a carrier to form an antibody 1/influenza virus nucleoprotein complex on the carrier;
(2) reacting the complex formed on the carrier in step (1) with a labeled antibody (labeled antibody 2) in which a label is bound to an anti-influenza virus nucleoprotein antibody (antibody 2) to form an antibody 1/influenza virus nucleoprotein/labeled antibody 2 complex on the carrier;
(3) washing the carrier after step (2) to remove substances not bound to the carrier; and
(4) measuring the label in the antibody 1/influenza virus nucleoprotein/labeled antibody 2 complex on the carrier after step (3);
wherein at least one of antibody 1 and antibody 2 is a human anti-influenza virus nucleoprotein antibody.

26. A method for detecting or quantifying an influenza virus, comprising the steps of:
(1) reacting a sample with a labeled antibody (labeled antibody 2) in which a label is bound to an anti-influenza virus nucleoprotein antibody (antibody 2) to form a labeled antibody 2/influenza virus nucleoprotein complex;
(2) reacting the complex formed in step (1) with an anti-influenza virus nucleoprotein antibody (antibody 1) fixed onto a carrier to form an antibody 1/influenza virus nucleoprotein/labeled antibody 2 complex on the carrier;
(3) washing the carrier after step (2) to remove substances not bound to the carrier; and
(4) measuring the label in the antibody 1/influenza virus nucleoprotein/labeled antibody 2 complex on the carrier after step (3);
wherein at least one of antibody 1 and antibody 2 is a human anti-influenza virus nucleoprotein antibody.

27. A method for detecting or quantifying an influenza virus, comprising the steps of:
(1) reacting a sample with a human anti-influenza virus nucleoprotein antibody fixed onto a carrier in the co-presence of a labeled antigen analog in which a label is bound to an influenza virus nucleoprotein antigen analog, to form a human anti-influenza virus nucleoprotein antibody/influenza virus nucleoprotein complex and a human anti-influenza virus nucleoprotein antibody/labeled antigen analog complex on the carrier;
(2) washing the carrier after step (1) to remove substances not bound to the carrier; and
(3) measuring the label in the complexes on the carrier after step (2).

28. A method for detecting or quantifying an influenza virus, comprising the steps of;
(1) reacting a sample with an influenza virus nucleoprotein antigen analog fixed onto a carrier in the co-presence of a labeled antibody in which a label is bound to a human anti-influenza virus nucleoprotein antibody, to form an influenza virus nucleoprotein antigen analog/labeled antibody complex on the carrier;
(2) washing the carrier after step (1) to remove substances not bound to the carrier; and
(3) measuring the label in the complex on the carrier after step (2).

29. The method of any one of claims 22 to 28, wherein the human anti-influenza virus nucleoprotein antibody is a human anti-influenza A virus nucleoprotein antibody.

30. The method of claim 29, wherein the human anti-influenza A virus nucleoprotein antibody is a human anti-influenza A virus nucleoprotein monoclonal antibody which specifically reacts with an influenza A virus nucleoprotein but does not react with an influenza B virus nucleoprotein.

31. The method of claim 30, wherein the amino acid sequence of the heavy chain variable region of the human anti-influenza A vims nucleoprotein monoclonal antibody comprises the amino acid sequence of any one of SEQ ID NOs: 8 to 13 and the amino acid sequence of the light chain variable region of the human anti-influenza A virus nucleoprotein monoclonal antibody comprises the amino acid sequence of any one of SEQ ID lVC7s: 22 to 27.

32. The method of any one of claims 22 to 28, wherein the human anti-influenza virus nucleoprotein antibody is a human anti-influenza B virus nucleoprotein antibody.

33. The method of claim 32, wherein the human anti-influenza B virus nucleoprotein antibody is a human anti-influenza B virus nucleoprotein monoclonal antibody which specifically reacts with an influenza B virus nucleoprotein but does not react with an influenza A virus nucleoprotein.

34. The method of claim 33, wherein the amino acid sequence of the heavy chain variable region of the human anti-influenza B virus nucleoprotein monoclonal antibody comprises the amino acid sequence of SEQ ID NO: 14 and the amino acid sequence of the light chain variable region of the human anti-influenza B virus nucleoprotein monoclonal antibody comprises the amino acid sequence of SEQ ID NO:: 28.

35. A human anti-influenza A virus nucleoprotein monoclonal antibody which specifically reacts with an influenza A virus nucleoprotein but does not react with an influenza B virus nucleoprotein, wherein the amino acid sequence of the heavy chain variable region comprises the amino acid sequence of any one of SEQ ID NOs: 8 to 13 and the amino acid sequence of the light chain variable region comprises the amino acid sequence of any one of SEQ ID NOs: 22 to 27.

36. A human anti-influenza B virus nucleoprotein monoclonal antibody which specifically reacts with an influenza B virus nucleoprotein but does not react with an influenza A virus nucleoprotein.

37. A human anti-influenza B virus nucleoprotein monoclonal antibody which specifically reacts with an influenza B virus nucleoprotein but does not react with an influenza A virus nucleoprotein, wherein the amino acid sequence of the heavy chain variable region comprises the amino acid sequence of SEQ ID NO: 14 and the amino acid sequence of the light chain variable region comprises the amino acid sequence of SEQ ID NO: 28.
